# EUROPÄISCHE PATENTSCHRIFT

(11) **EP 2 570 035 B1**
(45) Veröffentlichungstag und Bekanntmachung des Hinweises auf die Patenterteilung: **18.06.2014**
(21) Anmeldenummer: 11181504.9
(22) Anmeldetag: 15.09.2011
(51) Int. Cl.: A23L 1/226, A23L 1/22, C07C 59/48, C07C 59/64, C07D 311/00

(54) **Verwendung von Neoflavonoiden zur Geschmacksmodifizierung**
Use of neoflavanoids for modifying taste
Utilisation de néoflavonoïdes pour la modification du goût

(43) Veröffentlichungstag der Anmeldung: 20.03.2013
(73) Patentinhaber: Symrise AG, 37603 Holzminden (DE)
(72) Erfinder: Backes, Dr. Michael, 37603 Holzminden (DE); Vössing, Tobias, 37688 Beverungen (DE); Ley, Dr. Jakob Peter, 37603 Holzminden (DE); Paetz, Susanne, 37671 Höxter (DE)
(74) Vertreter: Eisenführ Speiser

(56) Entgegenhaltungen:
- EP-A2- 1 258 200
- DE-A1- 2 701 280
- US-A1- 2008 176 912
- US-A1- 2010 227 039
- ROELENS F ET AL: "Regioselective synthesis and estrogenicity of (+/-)-8-alkyl-5,7-dihydroxy-4-(4-hydroxyph enyl)-3,4-dihydrocoumarins", EUROPEAN JOURNAL OF MEDICINAL CHEMISTRY, EDITIONS SCIENTIFIQUE ELSEVIER, PARIS, FR, Bd. 40, Nr. 10, 1. Oktober 2005 (2005-10-01), Seiten 1042-1051, XP027857667, ISSN: 0223-5234 [gefunden am 2005-10-01]
- SUN XINGZHONG ET AL: "Neoflavonoids from Polygonum perfoliatum", PLANTA MEDICA, Bd. 65, Nr. 7, Oktober 1999 (1999-10), Seiten 671-673, XP002676796, ISSN: 0032-0943

## Beschreibung

Die vorliegende Erfindung betrifft primär die Verwendung einer oder mehrerer Neoflavonoide der nachfolgend definierten Formel (I) und/oder eines oder mehrerer physiologisch akzeptabler Salze eines oder mehrerer Neoflavonoide der nachfolgend definierten Formel (I) zur Geschmacksmodifizierung, insbesondere zum Verändern oder Maskieren eines bitteren, adstringierenden und/oder metallischen Geschmackseindruckes eines bitter, adstringierend und/oder metallisch schmeckenden Stoffes sowie ein entsprechendes Verfahren zur Geschmacksmodifizierung. Ferner betrifft die vorliegende Erfindung bestimmte Mischungen und bestimmte zum Verzehr geeignete Zubereitungen enthaltend ein oder mehrere Neoflavonoide der Formel (I) und/oder ein oder mehrere physiologisch akzeptable Salze eines oder mehrerer Neoflavonoide der Formel (I).

Weitere Aspekte ergeben sich aus der folgenden Beschreibung, den Ausführungsbeispielen und den Ansprüchen.

Nahrungs- oder Genussmittel enthalten häufig eine Vielzahl verschiedener Bitterstoffe, die zwar einerseits in bestimmten Lebensmitteln in Maßen erwünscht sind und zu deren charakteristischen Geschmack beitragen (z.B. Coffein in Tee oder Kaffee, Chinin in so genannten Bitter-Lemon-Getränken, Bitterstoffe aus Hopfen in Bier), andererseits den Wert aber auch stark mindern können (z.B. Flavonoidglycoside und Limonoide in ZitrusSäften, bitterer Nachgeschmack vieler hochintensiver Süßstoffe wie Aspartam, Cyclamat, Acesulfam K, Rebaudiosid A, Glyccyrhizinsäure oder Saccharin, hydrophobe Aminosäuren und/oder Peptide in Käse).

Bittergeschmack wird regelmäßig durch einzelne Stoffe (Beispiele vgl. unten) verursacht, die an spezielle Bitterrezeptoren auf Geschmackszellen (die in den so genannten Geschmacksknospen auf der Zunge zu finden sind) binden und über neurochemische Kaskaden ein Signal an das Gehirn senden, dass eine Abwehrreaktion und einen negativen Geschmackseindruck verursacht (vgl. Wolfgang Meyerhof, Reviews of Physiology, Biochemistry and Pharmacology 2005, 154, 37-72).

Adstringierender Geschmack wird in der Regel durch Fällung von Prolin-reichen Proteinen im Speichel durch Adstringenzien, z.B. Metallsalze, Polyphenole wie (Gallo-)Catechine, Proanthocyanidine, andere Flavonoide oder Tannine, verursacht. Der normalerweise als "Schmiermittel" dienende homogene Speichel enthält dann denaturierte Proteine, die die Gleitfähigkeit herabsetzen und dadurch ein raues oder trockenes, auch als adstringierend empfundenes Gefühl im Mund hinterlassen (Am. J. Clin. Nutr. 2005, 81, 330S-335S).

Zur Senkung des Gehalts an Bitterstoffen in Lebensmitteln, die entweder bereits im Ausgangsmaterial enthalten sind, wie z.B. in Zitrusfrüchten oder während des Herstellungsprozesses entstehen, wie Beispielsweise bei der Käseherstellung, ist daher oft eine nachträgliche Behandlung nötig. Dies kann zum einen extraktiv geschehen, wie bei der Entcoffeinierung von Tee bzw. Kaffee, oder enzymatisch, z.B. Behandlung von Orangensaft mit einer Glycosidase zur Zerstörung des bitteren Naringins oder Spaltung von Gallusestern der Catechine zu den freien Catechinen mit Esterasen oder Einsatz von speziellen Peptidasen bei der Reifung von Käse. Diese Behandlung ist belastend für das Produkt, erzeugt Abfallstoffe und bedingt z.B. auch Lösungsmittelreste und andere Rückstände (Enzyme) in den Produkten.

Daher ist es wünschenswert, Stoffe zu finden, die unangenehme Geschmackseindrücke, insbesondere bittere, adstringierende und/oder metallische Geschmackseindrücke wirkungsvoll verändern, vermindern oder gar unterdrücken können, ohne dabei die Qualität eines entsprechenden Lebensmittels bzw. einer entsprechenden zum Verzehr geeigneten Zubereitung durch zusätzliche Prozessschritte zu beeinflussen.

Auch bei vielen pharmazeutischen Wirkstoffen ist die Unterdrückung des bitteren Geschmacks besonders wichtig. Dadurch kann die Bereitschaft der Patienten, insbesondere bei bitterempfindlichen Patienten wie Kindern, die pharmazeutische Zubereitung oral zu sich zu nehmen, deutlich erhöht werden. Viele pharmazeutische Wirkstoffe, beispielsweise Aspirin, Salicin, Paracetamol, Ambroxol, Antibiotika wie Oxafloxazin oder Chinin, sowie eine ganze Reihe weiterer pharmazeutisch aktiver Verbindungen, weisen einen ausgeprägten bitteren, adstringierenden und/oder metallischen Geschmack und/oder Nachgeschmack auf.

Des Weiteren weisen auch verschiedene nicht-nutritive, hochintensive Süßstoffe oft geschmackliche Probleme auf. So sind sie zwar durch ihre geringe Einsatzkonzentration gut geeignet, Süße in Nahrungsmittel zu bringen, zeigen jedoch oft geschmackliche Probleme durch im Vergleich zu Zucker unähnliche Zeit-Intensitätsprofile (z.B. Sucralose, Stevioside, Cyclamat), einen bitteren und/oder adstringierenden Nachgeschmack (z.B. Acesulfam K, Saccharin, Steviosid, Rebaudiosid A, Rebaudiosid C) und/oder ausgeprägte zusätzliche Aromaeindrücke (z.B. Glycyrrhizinsäureammoniumsalz). Insbesondere bei süßen, kalorienfreien oder nahezu kalorienfreien Lebensmittel, beispielsweise Getränken, die mit Hilfe solcher Süßstoffe hergestellt wurden, senkt dieser unangenehme Neben- und/oder Nachgeschmack häufig die sensorische Akzeptanz und sollte daher maskiert werden.

Es sind bereits einige Ansätze zur, zumindest partiellen, Bitter-Reduktion beschrieben worden (z.B. in Chemosensory Perception 2008, 1(1): 58-77). Dazu können verschiedene Lösungsansätze verwendet werden: zum Einen die Entfernung des Bitterstoffes aus dem Lebensmittel, wie beispielsweise bei der Entbitterung von Zitrussäften, die Verwendung von Verkapselungssystemen oder das Überdecken von bitter schmeckenden Verbindungen mit Hilfe anderer Geschmacks- oder Aromastoffe, wie z.B. mit Süßstoffen (Recent Patents on Drug Delivery and Formulation, 2009, 3, 26-39). Die beschriebenen Lösungsansätze zeigen alle in der Anwendung regelmäßig und zum Teil erhebliche Limitationen, wie nicht-Natürlichkeit, teure Rohstoffe, unerwünschte Nebeneffekte (z.B. gleichzeitige Unterdrückung der Süße, gleichzeitig auftretender salziger Geschmack etc.) und/oder Löslichkeitsprobleme, so dass weiterhin Bedarf an natürlichen, einfach zu verwendenden bzw. einfach in oral konsumierbare Zubereitungen einarbeitbare maskierenden Substanzen, insbesondere an Bittermaskierungs-Lösungen, besteht.

Es war die primäre Aufgabe der vorliegenden Erfindung, Substanzen (einzelne Stoffe oder Stoffgemische) zu finden, die eine Veränderung oder Maskierung (d.h. Verminderung oder Unterdrückung) eines bitteren, adstringierenden und/oder metallischen Geschmackseindrucks unangenehm schmeckender Stoffe in zum Verzehr geeigneten Zubereitungen, insbesondere in Nahrungs- und Genussmitteln oder pharmazeutischen Zubereitungen, ermöglichen.

Vorzugsweise sollten die gesuchten Substanzen einen bitter-maskierenden Effekt gegen unterschiedliche Bitterstoffe aufweisen. Dabei sollten die gesuchten Substanzen vorzugsweise den sonstigen, erwünschten Geschmackseindruck einer zum Verzehr geeigneten Zubereitung nicht oder nicht wesentlich beeinflussen. Bevorzugt sollten die gesuchten Substanzen in einer breiten Produktpalette anwendbar und/oder leicht zugänglich sein.

Die primäre Aufgabe der vorliegenden Erfindung wird gelöst durch die Verwendung eines, zweier oder mehrerer verschiedener Verbindungen der Formel **(I)** 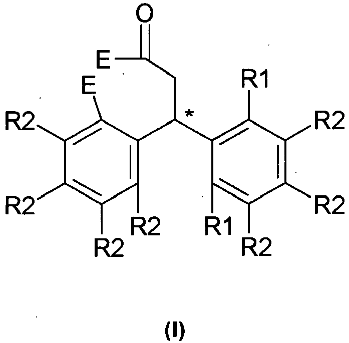 oder
eines, zweier oder mehrerer verschiedener physiologisch akzeptabler Salze eines, zweier oder mehrerer verschiedener Verbindungen der Formel **(I),**
oder
eines Gemisches eines, zweier oder mehrerer verschiedener Verbindungen der Formel **(I)** mit einem, zwei oder mehreren verschiedenen physiologisch akzeptablen Salzen eines, zweier oder mehrerer verschiedener Verbindungen der Formel **(I),**
wobei
E entweder jeweils OH oder beide E zusammen ein O bedeuten,
R1, unabhängig von dem jeweils anderen Rest R1, Wasserstoff oder OR^{a} bedeutet, wobei R^{a} Wasserstoff, C1-C5-Alkyl oder C2-C5-Alkenyl ist,
R2, unabhängig von den anderen Resten R2, Wasserstoff oder OR^{b} bedeutet, wobei R^{b} Wasserstoff, C1-C5-Alkyl oder C2-C5-Alkenyl ist,
wobei gegebenenfalls zwei unmittelbar benachbarte Reste R1 und/oder R2 zusammen eine Gruppe OCH₂O repräsentieren,
zum sensorischen Verändern oder Maskieren des unangenehmen Geschmackseindrucks, vorzugsweise des bitteren, adstringierenden und/oder metallischen Geschmackseindrucks, eines unangenehm schmeckenden Stoffes.

Überraschenderweise wurde gefunden, dass die erfindungsgemäß einzusetzenden Neoflavonoide der Formel **(I),** deren Gemische und/oder deren Salze (jeweils wie oben definiert) unangenehme, insbesondere bittere, adstringierende und/oder metallische, Geschmackseindrücke, einer Vielzahl von unangenehm schmeckenden Stoffen und oral konsumierbaren Zubereitungen, die einen oder mehrere unangenehm, insbesondere bitter, adstringierend und/oder metallisch, schmeckende Stoffe enthalten, maskieren, d.h. verringern oder sogar vollständig unterdrücken, können. Entsprechendes gilt auch für die nachfolgend beschriebenen erfindungsgemäßen Zubereitungen.

Unter Maskieren oder Maskierung wird im Rahmen des vorliegenden Textes eine Reduzierung, d.h. eine Verminderung, bzw. eine vollständige Unterdrückung verstanden.

Das Verändern oder Maskieren des unangenehmen Geschmackseindrucks bedeutet damit im Ergebnis regelmäßig eine Geschmacksverbesserung, insbesondere in Bezug auf bittere, adstringierende und/oder metallische Geschmackseindrücke.

Die Konfiguration an dem chiralen Kohlenstoffatom der Verbindungen der Formel **(I)** (d.h. an der mit einem "*" markierten Position im obigen Formelbild **(I))** kann dabei (R) oder (S) sein. Dies gilt auch für die nachfolgenden Ausführungen und nachfolgend dargestellten Strukturformeln der erfindungsgemäß einzusetzenden Verbindungen.

Die Verbindungen der Formel **(I)** können dabei in bevorzugten Ausgestaltungen als reine Enantiomere oder als Mischungen von Enantiomeren in jedem beliebigen Verhältnis zueinander miteinander kombiniert werden. In einer bevorzugten Ausgestaltung werden die Verbindungen der Formel **(I)** in Form von racemischen Gemischen, d.h. als Racemate eingesetzt.

Vorzugsweise bedeuten R1 und R2, unabhängig von den jeweils anderen Rest R1 und R2, Wasserstoff, Hydroxy oder einen Rest ausgewählt aus der Gruppe bestehend aus 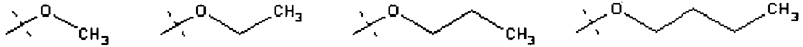 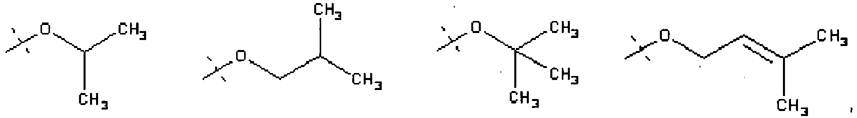 wobei die gestrichelte Linie die Bindung markiert, welche den Rest mit dem in der Formel **(I)** benachbarten C-Atom verknüpft.

Vorzugsweise gilt für die Verbindungen der Formel **(I):**
R1 bedeutet H oder OH,
und/oder
ein oder mehrere der Reste R1 oder R2 in Formel **(I)** bedeuten eine Hydroxygruppe.

Erfindungsgemäß bevorzugt ist die oben genannte Verwendung, wobei ein, zwei, mehrere oder sämtliche der eingesetzten Verbindungen jeweils ausgewählt sind aus der Gruppe bestehend aus den Verbindungen der Formel **(I)** und deren physiologisch akzeptablen Salzen, wobei
E jeweils OH oder beide E zusammen Sauerstoff bedeuten,
R1, unabhängig von dem jeweils anderen Rest R1, Wasserstoff oder Hydroxy bedeutet,
R2, unabhängig von den anderen Resten R2, Wasserstoff, Hydroxy, Methoxy, Ethoxy, n-Propoxy, iso-Propoxy, n-Butoxy, iso-Butoxy, tert-Butoxy oder OR^{b} bedeutet, wobei R^{b} C5-Alkenyl ist, dabei wiederum bevorzugt bedeutet R^{b} Prenyl,
wobei gegebenenfalls zwei unmittelbar benachbarte Reste R2 zusammen eine Gruppe OCH₂O repräsentieren,
wobei vorzugsweise ein oder mehrere der Reste R1 oder R2 eine Hydroxygruppe bedeuten.

Bevorzugt weist eine Verbindung der Formel **(I)** insgesamt ein, zwei, drei, vier oder fünf Hydroxygruppen auf.

Die erfindungsgemäß einzusetzenden Verbindungen der Formel (I) können je nach Bedeutung von E folgenden Strukturformeln **(I-A)** oder **(I-B)** entsprechen, wobei R1 und R2 jeweils die oben angegebene Bedeutung haben:

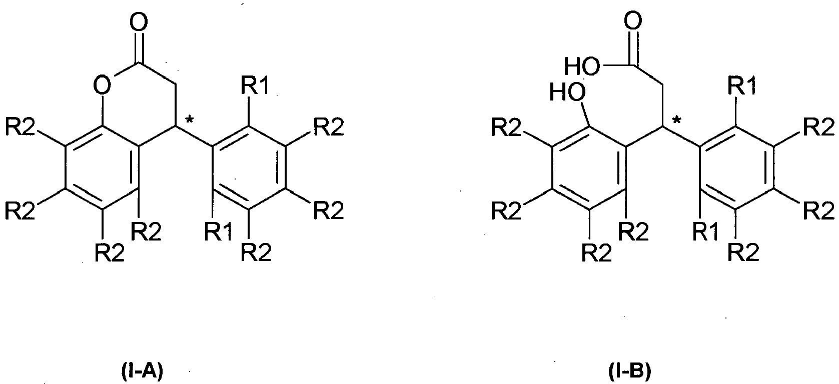

In Abhängigkeit des pH-Wertes kann der Lactonring der Verbindung der Formel **(I-A)** geöffnet werden und die Verbindung der Formel **(I-A)** im Gleichgewicht mit der entsprechenden "offenkettigen" Verbindung der Formel **(I-B)** vorliegen, wie nachfolgend schematisch gezeigt, wobei M⁺ ein (vorzugsweise physiologisch akzeptables) Gegenkation bedeutet (und wobei das Gegenkation bevorzugt die nachfolgend angegebene Bedeutung hat):

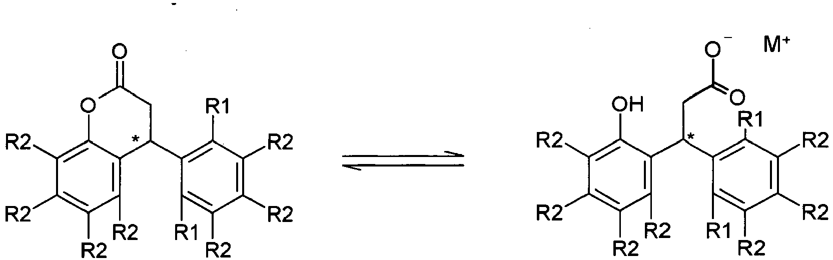

Für den Fall, dass mindestens einer der beiden Reste R1 in Formel **(I)** eine Hydroxygruppe bedeutet, kann zumeist in Abhängigkeit von der (Lebensmittel)Matrix und deren pH-Wert - insbesondere in Medien oder Matrices mit schwach saurem pH-Wert - ein Gleichgewicht zwischen den Substanzen der Formel **(I-A1)** und **(I-A2)** beobachtet werden.

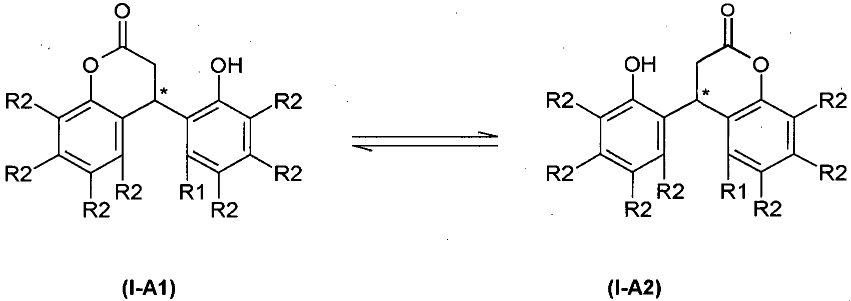

Ein weiterer Aspekt der vorliegenden Erfindung betrifft die Verwendung eines Substanzgemisches umfassend eine oder mehrere Verbindungen der Formel **(I-A1)** und eine oder mehrere Verbindungen der Formel **(I-A2),** und/oder deren physiologisch akzeptablen Salze.

Erfindungsgemäß bevorzugt einzusetzende Verbindungen der Formel **(I-A1)** sind die Substanzen **(1)** und **(2)**
**(1)** (4S)-5,7-Dihydroxy-4-(2-hydroxyphenyl)chroman-2-on
**(2)** (4R)-5,7-Dihydroxy-4-(2-hydroxyphenyl)chroman-2-on 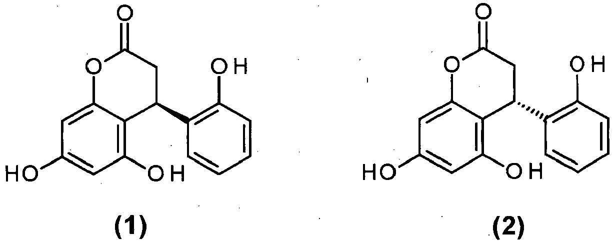 oder
   - ein Salz einer Verbindung der Formeln **(1)** und **(2)**
      oder
   - eine Mischung der Verbindungen der Formeln **(1)** und **(2),** zwei unterschiedliche Salze von Verbindungen der Formeln **(1)** und **(2)** oder eine Verbindung der Formel **(1)** und **(2)** und ein Salz von Verbindungen der Formel **(1)** und **(2),**
als Geschmacksverbesserer, bevorzugt zur Maskierung oder Verminderung unangenehmer Geschmackseindrücke, insbesondere bitterer, adstringierender und/oder metallischer Geschmackseindrücke.

Zur Bildung isomerer Verbindungen in Abhängigkeit von der Lebensmittelmatrix und des pH-Wertes gilt das oben Gesagte.

Erfindungsgemäß bevorzugt eingesetzt werden Verbindungen der Formel **(I),** die jeweils ausgewählt sind aus der Gruppe bestehend der Verbindungen der Formel **(II)** 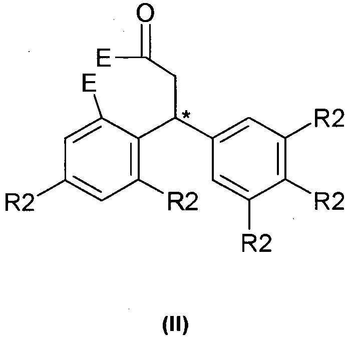 wobei
E jeweils OH oder beide E zusammen Sauerstoff bedeuten,
R2, unabhängig von den anderen Resten R2, Wasserstoff, Hydroxy, Methoxy, Ethoxy, n-Propoxy oder iso-Propoxy bedeutet, vorzugsweise H, OH, OCH₃ oder OCH₂CH₃,
wobei gegebenenfalls zwei unmittelbar benachbarte Reste R2 zusammen eine Gruppe OCH₂O repräsentieren,
wobei vorzugsweise ein oder mehrere der Reste R2 eine Hydroxygruppe bedeuten, und deren physiologisch akzeptablen Salzen.

Weiter bevorzugt ist die Verwendung von Verbindungen der Formel **(II-A),** 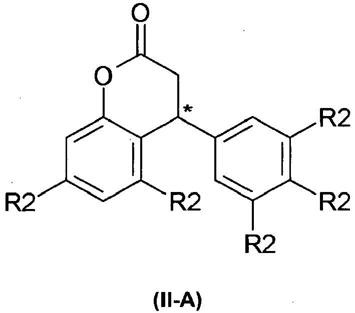 oder
- eines Salzes einer Verbindung der Formel **(II-A)**
   oder
- einer Mischung aus zwei oder mehr unterschiedlichen Verbindungen der Formel **(II-A),** zwei oder mehr unterschiedlichen Salzen von Verbindungen der Formel **(II-A)** oder einer oder mehreren unterschiedlichen Verbindungen der Formel **(II-A)** und einem oder mehreren unterschiedlichen Salzen von einer oder mehreren unterschiedlichen Verbindungen der Formel **(II-A),**
wobei
R2, unabhängig von den anderen Resten R2, Wasserstoff, Hydroxy, Methoxy, Ethoxy, n-Propoxy oder iso-Propoxy bedeutet, vorzugsweise H, OH, OCH₃ oder OCH₂CH₃,
wobei gegebenenfalls zwei unmittelbar benachbarte Reste R2 zusammen eine Gruppe OCH₂O repräsentieren,
wobei vorzugsweise mindestens einer der Reste R2 eine Hydroxygruppe bedeutet.

Die Konfiguration an dem chiralen Kohlenstoffatom (d.h. an der mit einem "*" markierten Position in den obigen Formelbildern **(II)** und **(II-A))** kann dabei jeweils (R) oder (S) sein.

Bevorzugt weist eine Verbindung der Formel **(II)** bzw. eine Verbindung der Formel **(II-A)** insgesamt eine, zwei, drei, vier oder fünf Hydroxygruppen auf, weiter bevorzugt insgesamt eine, zwei, drei oder vier Hydroxygruppen, insbesondere bevorzugt insgesamt zwei, drei oder vier Hydroxygruppen.

Erfindungsgemäß besonders bevorzugt ist dabei die Verwendung einer oder mehrerer Verbindungen der Formeln **(3) - (28)** und/oder deren physiologisch akzeptablen Salze
**(3)** ((4S)-5,7-Dihydroxy-4-(3-hydroxy-4-methoxy-phenyl)chroman-2-on
**(4)** ((4R)-5,7-Dihydroxy-4-(3-hydroxy-4-methoxy-phenyl)chroman-2-on
**(5)** ((4S)-5,7-Dihydroxy-4-(4-hydroxy-3-methoxy-phenyl)chroman-2-on
**(6)** ((4R)-5,7-Dihydroxy-4-(4-hydroxy-3-methoxy-phenyl)chroman-2-on
**(7)** ((4S)-5,7-Dihydroxy-4-(4-hydroxyphenyl)chroman-2-on
**(8)** ((4R)-5,7-Dihydroxy-4-(4-hydroxyphenyl)chroman-2-on
**(9)** ((4S)-5,7-Dihydroxy-4-(4-methoxyphenyl)chroman-2-on
**(10)** ((4R)-5,7-Dihydroxy-4-(4-methoxyphenyl)chroman-2-on
**(11)** ((4S)-4-(1,3-Benzodioxol-5-yl)-5,7-dihydroxy-chroman-2-on
**(12)** ((4R)-4-(1,3-Benzodioxol-5-yl)-5,7-dihydroxy-chroman-2-on
**(13)** ((4S)-4-(3,4-Dimethoxyphenyl)-5,7-dihydroxy-chroman-2-on
**(14)** ((4R)-4-(3,4-Dimethoxyphenyl)-5,7-dihydroxy-chroman-2-on
**(15)** ((4S)-4-(3,4-Dihydroxyphenyl)-5,7-dihydroxy-chroman-2-on
**(16)** ((4R)-4-(3,4-Dihydroxyphenyl)-5,7-dihydroxy-chroman-2-on
**(17)** ((4S)-7-Hydroxy-4-(4-hydroxyphenyl)chroman-2-on
**(18)** ((4R)- 7-Hydroxy-4-(4-hydroxyphenyl)chroman-2-on
**(19)** ((4S)-5-Hydroxy-4-(4-hydroxy-3-methoxy-phenyl)-7-methoxy-chroman-2-on
**(20)** ((4R)-5-Hydroxy-4-(4-hydroxy-3-methoxy-phenyl)-7-methoxy-chroman-2-on
**(21)** ((4S)-7-Hydroxy-4-(4-hydroxy-3-methoxy-phenyl)-5-methoxy-chroman-2-on
**(22)** ((4R)-7-Hydroxy-4-(4-hydroxy-3-methoxy-phenyl)-5-methoxy-chroman-2-on
**(23)** ((4*S*)-7-Hydroxy-4-(4-hydroxy-3-methoxy-phenyl)chroman-2-on
**(24)** ((4*R*)-7-Hydroxy-4-(4-hydroxy-3-methoxy-phenyl)chroman-2-on
**(25)** ((4S)-7-Hydroxy-4-(3-hydroxy-4-methoxy-phenyl)chroman-2-on
**(26)** ((4R)-7-Hydroxy-4-(3-hydroxy-4-methoxy-phenyl)chroman-2-on
**(27)** ((4S)-4-(3,4-Dihydroxyphenyl)-7-hydroxy-chroman-2-on
**(28)** ((4R)-4-(3,4-Dihydroxyphenyl)-7-hydroxy-chroman-2-on

Die Strukturformeln der erfindungsgemäß bevorzugt einzusetzenden Verbindungen **(3)** bis **(28)** sind zur Verdeutlichung nachfolgend angegeben:

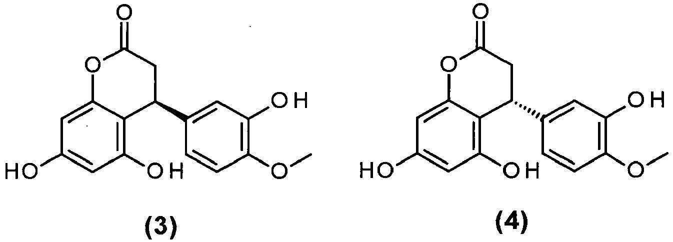

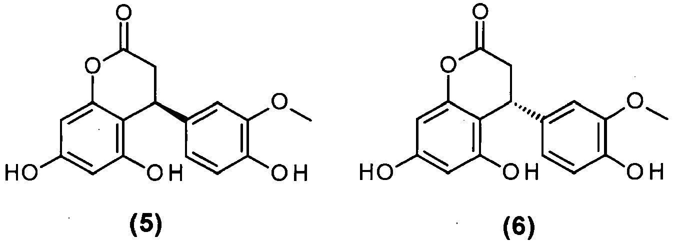

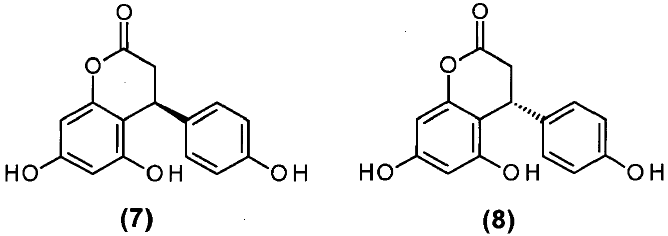

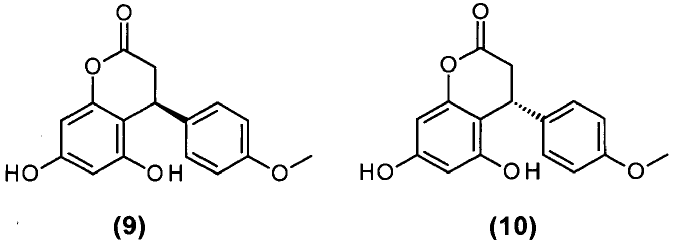

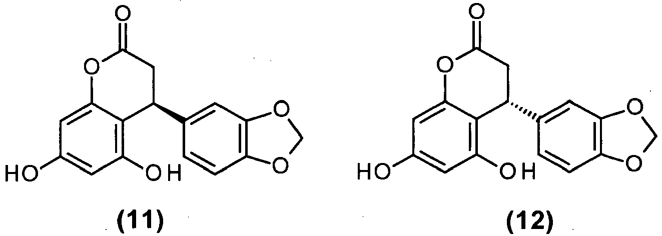

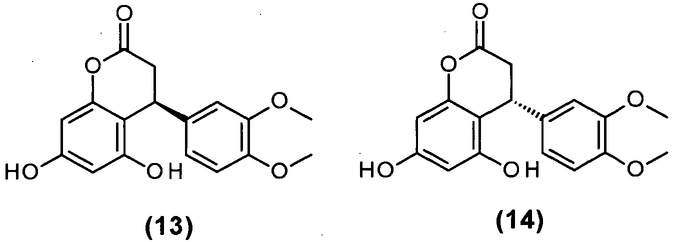

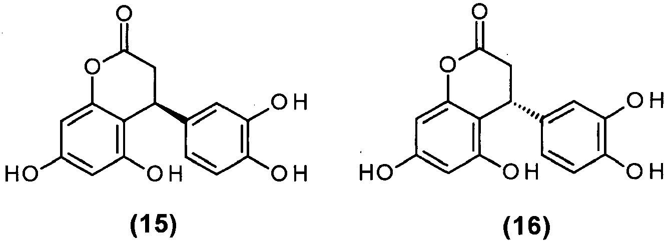

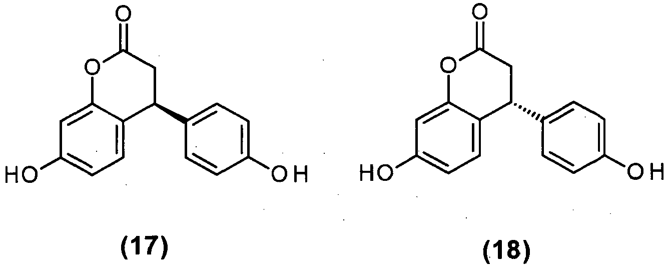

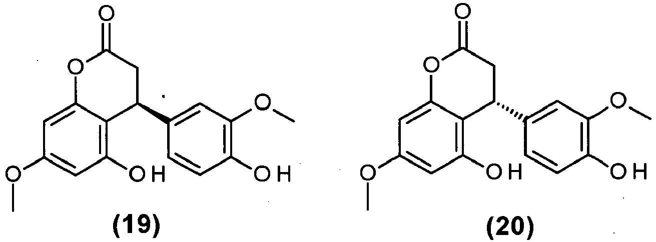

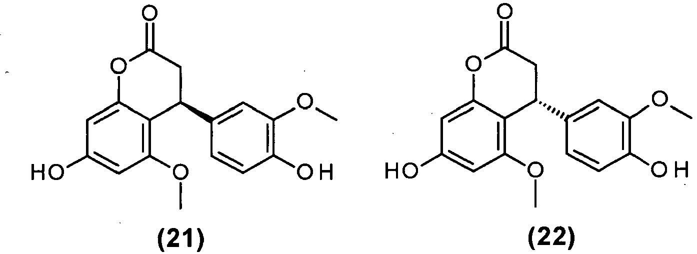

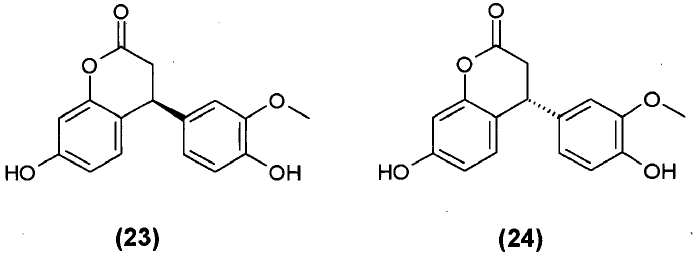

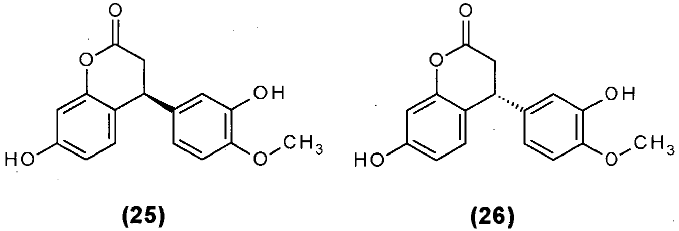

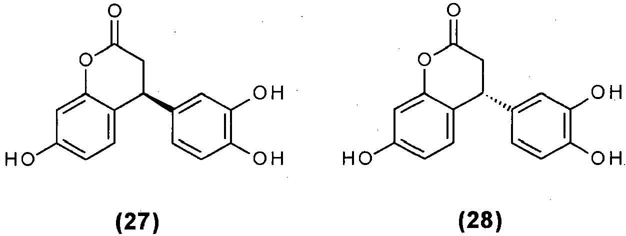

Für den Fall, dass im Einzelfall ein Widerspruch zwischen der vorstehend angegebenen chemischen Nomenklatur und der jeweils dargestellten Strukturformel der erfindungsgemäß bevorzugt einzusetzenden Verbindungen der Formeln **(1)** bis **(28)** bestehen sollte, gilt die Strukturformel.

Verschiedene Neoflavonoide der Formel **(I)** können auch in bestimmten Pflanzen oder Pflanzenteilen vorkommen.

Besonders bevorzugt sind hier die Neoflavonoide **(7)** und **(8)** sowie die ebenfalls unter die allgemeine Formel **(I)** fallenden Neoflavonoide **(29), (30), (31), (32), (33)** und **(34)** welche in *Polygonum Perfoliatum* gefunden wurden (siehe Planta Medica 1999, 65, 671-673; Chin. J. Appl. Environ. Biol. 2009, 15, 615-620) sowie deren physiologisch akzeptablen Salze.

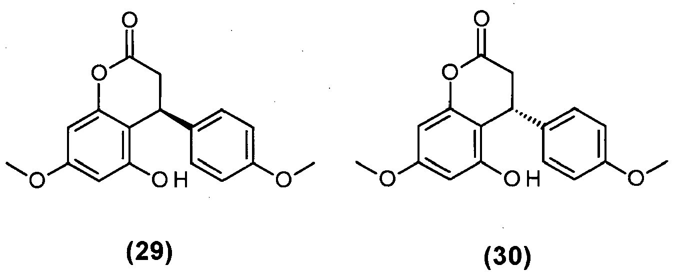

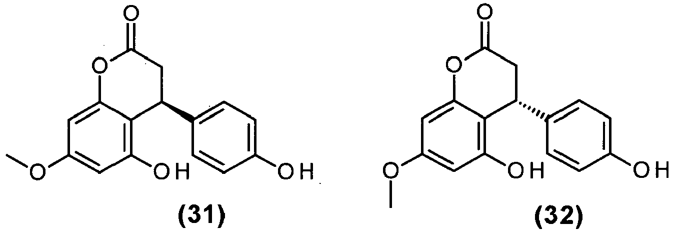

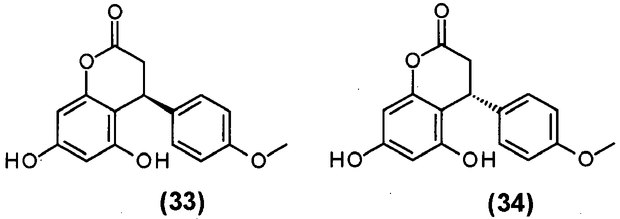

Dementsprechend können ein oder mehrere der erfindungsgemäß zu verwendenden Neoflavonoide der Formel **(I)** auch in Form von pflanzlichen Extrakten, insbesondere in Form von pflanzlichen Extrakten aus *Polygonum Perfoliatum,* erfindungsgemäß verwendet werden.

Ebenfalls erfindungsgemäß bevorzugt ist die Verwendung der Neoflavonoide **(35), (36), (37)** und **(38)** der nachfolgenden Strukturformeln bzw. deren physiologisch akzeptablen Salze.

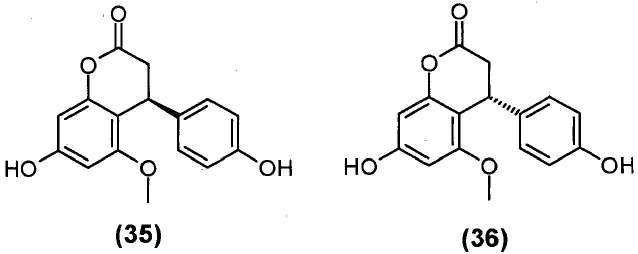

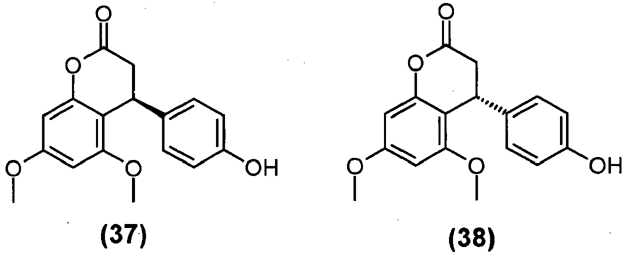

Dem Chemiker sind mehrere Wege bekannt die Verbindungen der Formel **(I)** herzustellen. Besonders bevorzugt ist die säurekatalysierte Umsetzung (klassisch z.B. unter Verwendung von Schwefelsäure oder Salzsäure) von Phenolderivaten der nachfolgenden Formel **(A)** mit Zimtsäurederivate der nachfolgenden Formel **(B)** oder der entsprechenden Ester der nachfolgenden Formel **(C)** zu den Verbindungen der Formel **(I)** (wie oben definiert), wie in dem nachfolgenden Schema skizziert: 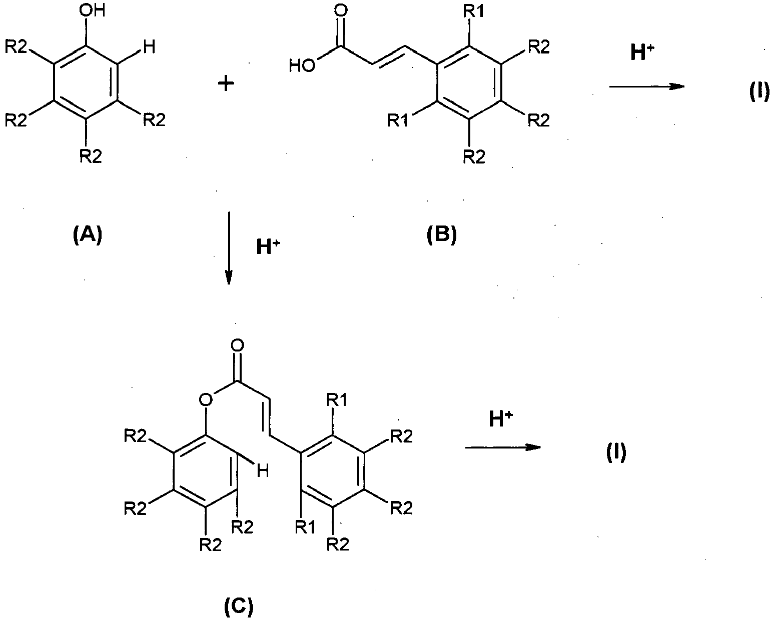 wobei jeweils die Reste R1 und R2 die oben bezüglich der Formel **(I)** angegebene Bedeutung haben.

Ausgewählte Literaturbeispiele jüngeren Datums beschreiben beispielsweise die Verwendung von Silica-geträgerter Schwefelsäure unter Mikrowellenbestrahlung (Guangzhou Huagong 2010, 38, 94-96), Trifluoressigsäure in Dichlormethan (J. Org. Chem. 2005, 70, 2881-2883), Montmorillonit K-10 in Dioxan (Synthesis 2001, 15, 2247-2254) oder p-Toluolsulfonsäure, entweder in Substanz oder mit Benzol bzw. Dichlormethan als Lösungsmittel (Bull. Korean Chem. Soc. 2011, 32, 65-70).

Des Weiteren können die erfindungsgemäß einzusetzenden Neoflavanoide auch analog zu der in Synth. Commun. 1987, 17(6) 723-727 beschriebenen Methode hergestellt werden, wie nachfolgend anhand der Reaktion von Meldrumsäure **(E)** mit Phloroglycinol **(D)** unter Zugabe eines Aldehyds (z.B. para-Hydroxybenzaldehyd **(F))** in Gegenwart von Pyridin zur Herstellung eines racemischen Gemisches der erfindungsgemäß einzusetzenden Verbindungen **(7)** und **(8)** schematisch dargestellt.

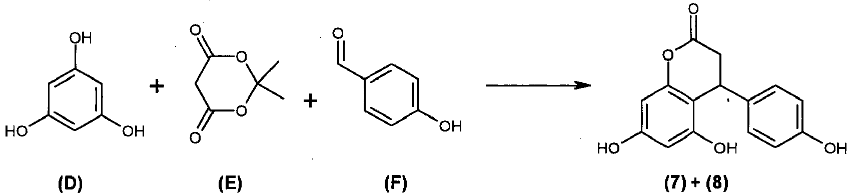

In der Literatur werden medizinische Eigenschaften verschiedener Substanzen der allgemeinen Strukturformel **(I)** beschrieben. Exemplarisch wird in CN 101906090 die Verbindung **(39)** als wirksam gegen die BGC823 Krebszelllinie getestet. Des Weiteren zeigte das Derivat **(40)** eine starke Cytotoxizität gegenüber Caco-2 Zellen (Chem. Pharm. Bull. 2010, 58, 242-246).

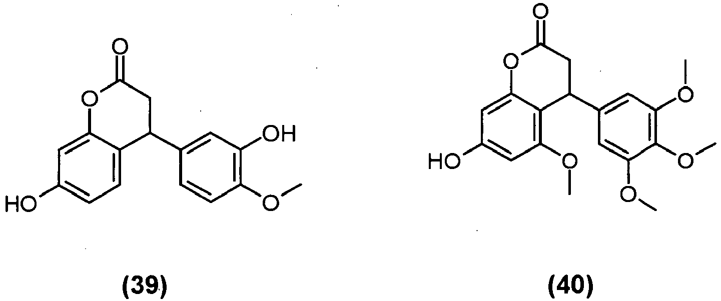

In keiner Veröffentlichung wird allerdings der Geschmack der Verbindungen der allgemeinen Formel **(I)** oder die geschmacksmodifizierenden Eigenschaften dieser Verbindungen beschrieben.

Die Erfindung betrifft somit in einer bevorzugten Ausgestaltung die Verwendung einer Verbindung der Formeln **(1) - (40),** vorzugsweise einer Verbindung der Formeln **(3) - (28),**
oder
eines Salzes einer Verbindung der Formeln **(1) - (40),** vorzugsweise einer Verbindung der Formeln **(3) - (28),**
oder
einer Mischung aus zwei oder mehr unterschiedlichen Verbindungen der Formeln **(1) - (40),** vorzugsweise einer Verbindung der Formeln **(3) - (28),**
oder
einer Mischung aus zwei oder mehr unterschiedlichen Salzen von Verbindungen der Formeln **(1) - (40),** vorzugsweise einer Verbindung der Formeln **(3) - (28),**
oder
einer Mischung einer oder mehreren unterschiedlichen Verbindungen der Formeln **(1) - (40),** vorzugsweise einer Verbindung der Formeln **(3) - (28),** und einem oder mehreren unterschiedlichen Salzen von einer oder mehreren unterschiedlichen Verbindungen der Formeln **(1) - (40),** vorzugsweise einer Verbindung der Formeln **(3) - (28),**
als Geschmacksverbesserer, bevorzugt zur Maskierung oder Verminderung unangenehmer Geschmackseindrücke, insbesondere bitterer, adstringierender und/oder metallischer Geschmackseindrücke.

Die erfindungsgemäßen Verbindungen der Formeln **(I), (I-A), (I-A1), (I-A2), (I-B), (II)** und **(II-A)** können bevorzugt als ein-, oder (insbesondere im Falle der Anwesenheit mehrerer Hydroxygruppen) mehrwertige Anionen vorliegen, wobei als Gegenkation die einfach positiv geladenen Kationen der ersten Haupt- und Nebengruppe, das Ammoniumion (NH₄⁺), ein Trialkylammoniumion, die zweiwertig geladenen Kationen der zweiten Nebengruppe, sowie die dreiwertigen Kationen der 3. Haupt- und Nebengruppe dienen. Bevorzugt ist ein, mehrere oder sämtliche Gegenkationen ausgewählt aus der Gruppe bestehend aus Na⁺, K⁺, NH₄⁺, Ca²⁺, Mg²⁺, Al³⁺ und Zn²⁺.

Überraschenderweise wurde gefunden, dass Verbindungen der allgemeinen Formel **(I),** deren Gemische und/oder Salze (wie oben definiert) insbesondere in Form der nachfolgend beschriebenen erfindungsgemäßen Mischungen und Aromakompositionen, einen bitteren, adstringierenden und/oder metallischen Eindruck einer Vielzahl von unangenehm schmeckenden Stoffen und oral konsumierbaren Zubereitungen, die einen oder mehrere bitter, adstringierend und/oder metallisch schmeckende Stoffe enthalten, verändern, verringern oder sogar vollständig unterdrücken können.

Unangenehm schmeckende Stoffe im Rahmen dieses Textes sind:
(a) Stoffe, die bitter, adstringierend, pappig, kalkig, staubig, trocken, mehlig, ranzig und/oder metallisch schmecken sowie
(b) Stoffe, die einen bitteren, adstringierenden, pappigen, kalkigen, staubigen, trockenen, mehligen, ranzigen und/oder metallischen Nachgeschmack aufweisen.

Die vorgenannten unangenehm schmeckenden Stoffe können weitere, nicht unangenehme Geschmacks- und/oder Geruchsqualitäten besitzen. Im Rahmen dieses Textes sind als nicht unangenehme Geschmacksqualitäten bevorzugt die Eindrücke würzig, umami, süß, salzig, sauer, scharf, kühlend, wärmend, brennend oder kribbelnd zu nennen.

Stoffe, die bitter, adstringierend, pappig, kalkig, staubig, trocken, mehlig, ranzig und/oder metallisch schmecken, sind beispielsweise: Xanthinalkaloide Xanthine (Coffein, Theobromin, Theophyllin), Alkaloide (Chinin, Brucin, Strychnin, Nicotin), phenolische Glycoside (z.B. Salicin, Arbutin), Flavanoidglycoside (z.B. Neohesperidin, Eriocitron, Neoeriocitrin, Narirutin, Hesperidin, Naringin), Chalcone oder Chalconglycoside, Dihydrochalconglycoside (Phloridzin, Trilobatin), hydrolisierbare Tannine (Gallus- oder Elagsäureester von Kohlenhydraten, z.B. Pentagalloylglucose), nichthydrolisierbare Tannine (ggfs. galloylierte Catechine oder Epicatechine und deren Oligomeren, z.B. Proanthyocyanidine oder Procyanidine, Thearubigenin), Flavone und deren Glycoside (z.B. Quercetin, Quercitrin, Rutin, Taxifolin, Myricetin, Myrictrin), andere Polyphenole (y-Oryzanol, Kaffeesäure oder deren Ester), terpenoide Bitterstoffe (z.B. Limonoide wie Limonin oder Nomilin aus Zitrusfrüchten, Lupolone und Humolone aus Hopfen, Iridoide, Secoiridoide), Absinthin aus Wermut, Amarogentin aus Enzian, metallische Salze (Kaliumchlorid, Natriumsulfat, Magnesiumsalze, Eisensalze, Aluminiumsalze, Zinksalze), pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin), Vitamine (beispielsweise Vitamin H, Vitamine aus der B-Reihe wie Vitamin B1, B2, B6, B12, Niacin, Panthotensäure), Denatoniumbenzoat oder andere Denatoniumsalze, Sucraloseoctaacetat, Harnstoff, ungesättigte Fettsäuren, insbesondere ungesättigte Fettsäuren in Emulsionen, Aminosäuren (z.B. Leucin, Isoleucin, Valin, Tryptophan, Prolin, Histidin, Tyrosin, Lysin oder Phenylalanin), Peptide (insbesondere Peptide mit einer Aminosäure aus der Gruppe Leucin, Isoleucin, Valin, Tryptophan, Prolin oder Phenylalanin am N- oder C-Terminus). Die vorgenannten Stoffe können entweder einzeln oder als Gemisch vorkommen, bevorzugt auch als natürliche Extrakte aus frischen, getrockneten, gerösteten, und/oder fermentierten Pflanzen oder Pflanzenteilen, so z.B. als Extrakte aus Blättern, Früchten, Ästen, Wurzeln, Fruchtschalen, Kernen, Samen z.B. aus Camellia sinensis, Camellia japonica, Coffea ssp., Cocoa theobroma, Vitis vinifera, Citrus ssp. und Hybriden, Poncirus ssp. und Hybriden, Perilla, Humulus lupulus, oder verwandten Arten stammen.

Erfindungsgemäß zu maskierende Bitterstoffe sind dabei insbesondere Xanthine (insbesondere Coffein, Theobromin, Theophyllin), phenolische Glycoside (insbesondere Salicin, Arbutin), Flavanoidglycoside (insbesondere Neohesperedin, Eriocitrin, Neoeriocitrin, Narirutin, Hesperidin, Naringin), Chalcone oder Chalconglycoside, Dihydrochalconglycoside (insbesondere Phloridzin, Trilobatin), hydrolisierbare Tannine (insbesondere Gallus- oder Ellagsäureester von Kohlenhydraten, z.B. Pentagalloylglucose), nichthydrolisierbare Tannine (insbesondere galloylierte Catechine oder Epicatechine und deren Oligomeren, z.B. Proanthyocyanidine oder Procyanidine, Thearubigenin), Flavone und deren Glycoside (insbesondere Quercetin, Quercitrin, Rutin, Taxifolin, Myricetin, Myrictrin), Kaffeesäure oder deren Ester, terpenoide Bitterstoffe (insbesondere Limonin, Nomilin, Lupolone und Humolone), metallische Salze (Kaliumchlorid, Natriumsulfat, Magnesiumsalze, Eisensalze, Aluminiumsalze, Zinksalze), pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin), Aminosäuren (z.B. Leucin, Isoleucin, Valin, Tryptophan, Prolin, Histidin, Tyrosin, Lysin oder Phenylalanin), Peptide (insbesondere Peptide mit einer Aminosäure aus der Gruppe Leucin, Isoleucin, Valin, Tryptophan, Prolin oder Phenylalanin am N- oder C-Terminus).

Weiter erfindungsgemäß bevorzugte zu maskierende Bitterstoffe sind ausgewählt aus der Gruppe bestehend aus Coffein, Theobromin, Chinin, Salicin, Arbutin, Neohesperedin, Eriocitrin, Neoeriocitrin, Narirutin, Hesperidin, Naringin, Phloridzin, Catechin, Epicatechin, Epigallocatechingallat (EGCG), Gallocatechin, Gallocatechin-3-gallat, Procyanidin B2, Procyanidin B5, Procyanidin C1, Thearubigenin, Rutin, Taxifolin, Myricetin, Myrictrin, Kaffeesäure oder deren Ester, Limonin und Nomilin, Aminosäuren (z.B. Leucin, Isoleucin, Valin, Tryptophan, Prolin, Histidin, Tyrosin, Lysin oder Phenylalanin), Peptide mit einer Aminosäure aus der Gruppe Leucin, Isoleucin, Valin, Tryptophan, Prolin oder Phenylalanin am N- oder C-Terminus, Kaliumchlorid, Paracetamol, Aspirin und β-Lactam-Antibiotika.

Stoffe, die einen bitteren, adstringierenden, pappigen, kalkigen, staubigen, trockenen, mehligen, ranzigen und/oder metallischen Neben- und/oder Nachgeschmack haben, können Aroma- oder Geschmackstoffe mit einem nicht unangenehmen Primärgeschmack (beispielsweise süß, salzig, würzig, sauer) und/oder -geruch sein, und z.B. zur Gruppe der Süßstoffe, Zuckeraustauschstoffe oder der Aromastoffe gehören. Beispielsweise seien genannt: Aspartam, Neotam, Superaspartam, Alitam, Saccharin, Sucralose, Tagatose, Monellin, Monatin, Stevioside, Rubusosid, Steviosid, Rebaudiosid A, Rebaudioside C, Thaumatin, Miraculin, Glycyrrhizin (Glycyrrhizinsäure), Glycyrrhetinsäure oder deren Derivate, Cyclamat oder die physiologisch akzeptablen Salze der vorgenannten Verbindungen.

Die erfindungsgemäß einzusetzenden Verbindungen der Formel **(I)** und die physiologisch akzeptablen Salze der Verbindungen der Formel **(I)** weisen in erfindungsgemäßen zum Verzehr geeignete Zubereitungen, insbesondere in den erfindungsgemäß (besonders) bevorzugt einzusetzenden Konzentrationen, keinen nennenswerten Eigengeschmack auf, insbesondere zeigen sie in den (bevorzugten bzw. besonders bevorzugten) Einsatzkonzentrationen keine unangenehmen oder störenden Geschmacksnoten.

Erfindungsgemäß bevorzugt sind Verbindungen **(1)** bis **(40),** weiter bevorzugt sind Verbindungen **(3)** bis **(28),** sowie deren oben definierten Gemische und Salze, wobei diese jeweils als racemische Gemische, Einzelstoffe oder in jedem beliebigen Verhältnis zueinander kombiniert werden können. Insbesondere bevorzugt sind die Verbindungen **(7)** und **(8),** welche erfindungsgemäß auch in Form eines Extraktes aus *Polygonum Perfoliatum* eingesetzt werden können.

Sofern die erfindungsgemäß einzusetzenden Verbindungen, insbesondere die Verbindungen **(7)** und **(8),** natürlich vorkommen, können diese auch in Form eines pflanzlichen Extraktes eingesetzt werden.

Entsprechend betrifft die vorliegende Erfindung ebenfalls die Verwendung eines aus pflanzlichem Material mittels Extraktion erhältlichen oder erhaltenen Produktes umfassend
ein, zwei oder mehrere verschiedene Verbindungen der Formel **(I)** wie oben definiert,
ein, zwei oder mehrere verschiedene physiologisch akzeptable Salze eines, zweier oder mehrerer verschiedener Verbindungen der Formel **(I)** wie oben definiert,
oder
ein Gemisch eines, zweier oder mehrerer verschiedener Verbindungen der Formel **(I)** wie oben definiert mit einem, zwei oder mehreren verschiedenen physiologisch akzeptablen Salzen eines, zweier oder mehrerer verschiedener Verbindungen der Formel **(I)** wie oben definiert,
zum sensorischen Verändern oder Maskieren des unangenehmen Geschmackseindrucks, vorzugsweise des bitteren, adstringierenden und/oder metallischen Geschmackseindrucks, eines unangenehm schmeckenden Stoffes.

Ein solcher erfindungsgemäß einzusetzender pflanzlicher Extrakt enthält vorzugsweise einen Gesamtanteil von 50 Gew.-% oder mehr, bevorzugt von 80 Gew.-% oder mehr, besonders bevorzugt von 90 Gew.-% oder mehr der Verbindungen der Formel **(I),** vorzugsweise der Verbindungen **(7)** und **(8),** jeweils bezogen auf die Trockenmasse des pflanzlichen Extraktes, wobei die Verbindungen der Formel **(I),** vorzugsweise die Verbindungen **(7)** und/oder **(8),** entweder als Einzelstoffe vorliegen oder in jedem beliebigen Verhältnis zueinander kombiniert werden können.

Die vorliegende Erfindung betrifft ferner bestimmte Zubereitungen, welche entweder unmittelbar oder nach Weiterverarbeitung zum Verzehr geeignet sind.

Erfindungsgemäße Zubereitungen können auch als Halbfertigware, beispielsweise als Riech-, Aroma- oder Geschmackstoffkomposition oder als Würzmischung vorliegen.

Die Erfindung betrifft eine zum Verzehr geeignete Zubereitung, ausgewählt aus der Gruppe bestehend aus
- der Ernährung, der Nahrungsergänzung, der Mundpflege oder dem Genuss dienenden Zubereitungen,
- kosmetischen Zubereitungen, vorzugsweise zur Applikation im Bereich des Kopfes,
- zur oralen Aufnahme bestimmten pharmazeutischen Zubereitungen,
enthaltend
einen, zwei oder mehrere unangenehm, insbesondere bitter, adstringierend und/oder metallisch, schmeckende Stoffe
sowie
ein, zwei oder mehrere verschiedene Verbindungen der Formel **(I)** wie oben definiert, oder
ein, zwei oder mehrere verschiedene physiologisch akzeptable Salze eines, zweier oder mehrerer verschiedener Verbindungen der Formel **(I)** wie oben definiert, oder
ein Gemisch eines, zweier oder mehrerer verschiedener Verbindungen der Formel **(I)** wie oben definiert mit einem, zwei oder mehreren verschiedenen physiologisch akzeptablen Salzen eines, zweier oder mehrerer verschiedener Verbindungen der Formel **(I)** wie oben definiert,
wobei die Menge des bzw. der unangenehm, insbesondere bitter, adstringierend und/oder metallisch, schmeckenden Stoffe ausreicht, um in einer Vergleichszubereitung, die weder eine Verbindung der Formel **(I)** wie oben definiert noch ein Salz einer Verbindung der Formel **(I)** wie oben definiert enthält, aber ansonsten identisch zusammengesetzt ist, als unangenehmer, insbesondere bitterer, adstringierender und/oder metallischer, Geschmack wahrgenommen zu werden,
und
die Gesamtmenge an Verbindungen der Formel **(I)** wie oben definiert, an Salzen der Verbindungen der Formel **(I)** wie oben definiert bzw. eines Gemisches wie oben definiert ausreicht, um im Vergleich mit der Vergleichszubereitung den unangenehmen, insbesondere bitteren, adstringierenden und/oder metallischen, Geschmackseindruck des bzw. der unangenehm schmeckenden Stoffe sensorisch zu verändern oder zu maskieren.

Die in Planta Medica 1999, 65, 671-673 und Chin. J. Appl. Environ. Biol. 2009, 15, 615-620 beschriebenen ethanolisch-wässrigen Extrakte aus der gemahlenen ganzen Pflanze *Polygonum Perfoliatum* sind, ebenso wie die dort aus den ethanolisch-wässrigen Extrakten erhaltenen Fraktionen und Substanzgemische, nicht Gegenstand der vorliegenden Erfindung.

Eine bevorzugte erfindungsgemäße Zubereitung ist dadurch gekennzeichnet, dass die Zubereitung kein Extrakt ist, der mittels Extraktion der gemahlenen ganzen Pflanze *Polygonum Perfoliatum* mit einem Extraktionsmittelgemisch aus Ethanol und Wasser im Massen- oder Volumenverhältnis 90 : 10 oder 95 : 5 erhältlich ist, und vorzugsweise kein Extrakt ist, der mittels Extraktion der ganzen Pflanze *Polygonum Perfoliatum* mit einem Extraktionsmittelgemisch aus Ethanol und Wasser erhältlich ist mit einem Volumenverhältnis größer als 1 : 1 erhältlich ist, und bevorzugt kein Extrakt ist, der mittels Extraktion der ganzen Pflanze *Polygonum Perfoliatum* mit einem Extraktionsmittelgemisch aus Ethanol und Wasser erhältlich ist.

Eine bevorzugte erfindungsgemäße Zubereitung enthält vorzugsweise kein Hexan und kein Methylenchlorid, und bevorzugt kein Hexan, kein Methylenchlorid, kein Aceton und kein Methanol.

Eine bevorzugte erfindungsgemäße Zubereitung ist keine Zubereitung, die 4-Dihydroxy-5,7-dihydroxy-4-(4-hydroxyphenyl)coumarin, α-Tocopherolchinon, 7'-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Saikosaponin M, Hydropiperosid, Quercetin-3-*O*-β-*D*-glucuronid-6"-butylester und Quercetin-3-*O*-β-*D*-glucuronid-6"-methylester enthält.

Hier hinsichtlich der diesen Verbindungen entsprechenden Strukturformeln sei auf die Literaturstelle Chin. J. Appl. Environ. Biol. 2009, 15, 615-620 hingewiesen, welche hinsichtlich der darin offenbarten entsprechenden Verbindungen auf dem Wege der Verweisung Bestandteil dieser Anmeldung wird.

Eine bevorzugte erfindungsgemäße Zubereitung ist keine Zubereitung, die 4-Dihydroxy-5,7-dihydroxy-4-(4-hydroxyphenyl)coumarin, α-Tocopherolchinon, 7'-Dihydroxymatairesinol, (24S)-Ethylchloesta-3β,5a,6a-triol, Quercetin, Cucurbitacin IIa, Cucurbitacin U, lotroridosid A, Pokeweedcerebrosid 5 und Bonarosid im Massenverhältnis von 4 : 9 : 8 : 37 : 30 : 24 : 14 : 5 : 12 : 41 enthält.

Sofern eine erfindungsgemäße Zubereitung 4-Dihydroxy-5,7-dihydroxy-4-(4-hydroxyphenyl)coumarin, enthält, gelten eine, mehrere oder sämtliche der folgenden Bedingungen:
- das Gewichtsverhältnis von 4-Dihydroxy-5,7-dihydroxy-4-(4-hydroxyphenyl)coumarin zu Quercetin ist ungleich 4 : 30,
- das Gewichtsverhältnis von 4-Dihydroxy-5,7-dihydroxy-4-(4-hydroxyphenyl)coumarin zu Cucurbitacin IIa ist ungleich 4 : 24,
- das Gewichtsverhältnis von 4-Dihydroxy-5,7-dihydroxy-4-(4-hydroxyphenyl)coumarin zu Cucurbitacin U ist ungleich 4 : 14,
- das Gewichtsverhältnis von 4-Dihydroxy-5,7-dihydroxy-4-(4-hydroxyphenyl)coumarin zu 7'-Dihydroxymatairesinol ist ungleich 4 : 8,
und wobei vorzugsweise zumindest einer der in der Zubereitung enthaltenen unangenehm, insbesondere bitter, schmeckenden Stoffe nicht Quercetin, nicht Cucurbitacin IIa, nicht Cucurbitacin U und nicht 7'-Dihydroxymatairesinol ist.

Sofern eine erfindungsgemäße Zubereitung 4-Dihydroxy-5,7-dihydroxy-4-(4-hydroxyphenyl)coumarin, enthält, beträgt das Massenverhältnis von 4-Dihydroxy-5,7-dihydroxy-4-(4-hydroxyphenyl)coumarin zu der Gesamtmasse von α-Tocopherolchinon, 7'-Dihydroxymatairesinol, (24S)-Ethylchloesta-3β,5a,6a-triol, Quercetin, Cucurbitacin IIa, Cucurbitacin U, lotroridosid A, Pokeweedcerebrosid 5 und Bonarosid nicht 4 : 180.

Eine bevorzugte erfindungsgemäße Zubereitung ist keine Zubereitung, die α-Tocopherolchinon, 7'-Dihydroxymatairesinol, (24S)-Ethylchloesta-3β,5a,6a-triol, Quercetin, Cucurbitacin IIa, Cucurbitacin U, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Saikosaponin M, Hydropiperosid, Quercetin-3-*O*-β-*D*-glucuronid-6"-butylester und Quercetin-3-*O*-β-*D*-glucuronid-6"-methylester enthält.

Eine bevorzugte erfindungsgemäße Zubereitung ist keine Zubereitung, die α-Tocopherolchinon, (24S)-Ethylchloesta-3β,5α,6α-triol, Cucurbitacin IIa, Cucurbitacin U, lotroridosid A, Pokeweedcerebrosid 5 und Bonarosid enthält.

Eine erfindungsgemäß bevorzugte Zubereitung enthält kein Cucurbitacin IIa, vorzugsweise kein Cucurbitacin IIa und kein Cucurbitacin U, und enthält bevorzugt keine Cucurbitacine.

Die oben beschriebenen Ausgestaltungen, insbesondere die oben als bevorzugt bzw. besonders bevorzugt gekennzeichneten Ausgestaltungen, bezüglich der erfindungsgemäßen Verwendung der Verbindungen der Formeln **(I), (I-A), (I-A1), (I-A2), (I-B), (II)** und **(II-A)** gelten entsprechend für eine erfindungsgemäße Zubereitung, insbesondere für eine bevorzugte bzw. besonders bevorzugte erfindungsgemäße Zubereitung.

Bevorzugt enthalten erfindungsgemäße Zubereitungen, insbesondere erfindungsgemäße zum, vorzugsweise direkten, Verzehr geeignete Zubereitungen, ein oder mehrere weitere Aromastoffe, Geschmackstoffe und/oder Geschmackskorrigenzien, um den Geschmack und/oder Geruch der Zubereitung abzurunden, zu verbessern bzw. zu verfeinern. Geeignete weitere Aromastoffe, Geschmackstoffe und/oder Geschmackskorrigenzien sind dabei vorzugsweise synthetische oder natürliche Aromastoffe, Geschmackstoffe und/oder Geschmackskorrigenzien, dabei vorzugsweise speichelfördernde, prickelnd, scharf und/oder warm schmeckende Substanzen, ätherische Öle oder Pflanzenextrakte, sowie gegebenenfalls zusätzlich zum Verzehr geeignete Hilfs- und Trägerstoffe. Als besonders vorteilhaft ist dabei, dass der bittere, adstringierende und/oder metallische Geschmackseindruck von weiteren Aromastoffen, Geschmackstoffen und/oder Geschmackskorrigenzien zusätzlich verändert und/oder vermindert werden kann und damit das gesamte Aroma- oder Geschmacksprofil einer erfindungsgemäße Zubereitung verbessert wird.

Erfindungsgemäße Zubereitungen enthalten vorzugsweise zumindest einen, zwei, drei, vier, fünf, sechs, sieben, acht, neun, zehn oder mehr weitere Aromastoffe.

Eine bevorzugt erfindungsgemäße Zubereitung ist dadurch gekennzeichnet, dass die Zubereitung
(i) einen, zwei, drei, vier, fünf, sechs, sieben, acht, neun, zehn oder mehr weitere Aromastoffe enthält mit einem Molgewicht von größer als 90 g/mol, vorzugsweise von größer als 100 g/mol, bevorzugt mit einem Molgewicht im Bereich von 110 g/mol bis 300 g/mol, weiter bevorzugt mit einem Molgewicht im Bereich von 120 g/mol bis 250 g/mol, besonders bevorzugt mit einem Molgewicht im Bereich von 125 g/mol bis 220 g/mol, insbesondere bevorzugt mit einem Molgewicht im Bereich von 130 g/mol bis 210 g/mol,
   und/oder
(ii) die Zubereitung eine, zwei, drei, vier, fünf, sechs, sieben, acht oder sämtliche der folgenden Substanzen nicht enthält:
   Tocopherolchinon, 7'-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5 und Bonarosid,
      und/oder
(iii) die Zubereitung keine der folgenden Substanzen enthält:
   (24S)-Ethylchloesta-3β,5α,6α-triol, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Hydropiperosid,
und/oder
(iv) die Zubereitung frei von frischen oder getrockneten Pflanzenteilen, insbesondere frei von Blättern und Blatteilen, von *Persicaria perfoliata* (= *Polygonum perfoliatum)* ist.

Eine besonders bevorzugt erfindungsgemäße Zubereitung ist dadurch gekennzeichnet, dass die Zubereitung
(i) zwei, drei, vier, fünf, sechs, sieben, acht, neun, zehn oder mehr weitere Aromastoffe enthält mit einem Molgewicht von größer als 120 g/mol, vorzugsweise mit einem Molgewicht von größer als 125 g/mol, bevorzugt mit einem Molgewicht im Bereich von 125 g/mol bis 220 g/mol, insbesondere bevorzugt mit einem Molgewicht im Bereich von 130 g/mol bis 210 g/mol.

Vorzugsweise gelten zumindest zwei der vorstehend definierten Bedingungen, bevorzugt Bedingung (i) und (iv) oder Bedingung (i) und (iii).

Vorzugsweise gelten zumindest drei der vorstehend definierten Bedingungen, bevorzugt Bedingung (i), (ii) und (iv) oder Bedingung (i), (ii) und (iii), oder sämtliche der Bedingungen (i) bis (iv).

Im Rahmen der vorliegenden Erfindung bevorzugt einzusetzende (einen oder mehrere) Aromastoffe werden vorzugsweise gewählt aus der Gruppe bestehend aus:
Acetophenon, Allylcapronat, alpha-lonon, beta-lonon, Anisaldehyd, Anisylacetat, Anisylformiat, Benzaldehyd, Benzothiazol, Benzylacetat, Benzylalkohol, Benzylbenzoat, beta-lonon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Der Ernährung oder dem Genuss dienende (d.h. zum Verzehr geeignete) Zubereitungen sind im Rahmen dieses Textes z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder vollständig hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Zubereitungen im Sinne der Erfindung können auch als Halbfertigware zur Herstellung weiterer der Ernährung oder dem Genuss dienenden Zubereitungen dienen. Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Überraschenderweise wurde gefunden, dass die erfindungsgemäß zu verwendenden Verbindungen, deren Gemische und/oder deren Salze (jeweils wie vorstehend definiert) - bereits in sehr geringen Konzentrationen - unangenehme Geschmackseindrücke, insbesondere den bitteren Geschmackseindruck, einer Vielzahl von Stoffen, maskieren, d.h. reduzieren oder sogar vollständig unterdrücken, können.

In einer erfindungsgemäß bevorzugten Zubereitung liegt die Gesamtmenge an Verbindungen der Formel **(I)** wie oben definiert und an physiologisch akzeptablen Salzen der Verbindungen der Formel **(I)** wie oben definiert im Bereich von 0,1 mg/kg (entsprechend 0,1 ppm) bis 1 Gew.-%, vorzugsweise im Bereich von 0,5 bis 1000 mg/kg (entsprechend 0,5 bis 1000 ppm), bevorzugt im Bereich von 1 bis 500 mg/kg, weiter bevorzugt im Bereich von 3 bis 300 mg/kg, besonders bevorzugt im Bereich von 5 bis 200 mg/kg, am meisten bevorzugt im Bereich von 10 bis 100 mg/kg, jeweils bezogen auf das Gesamtgewicht der Zubereitung.

Da sich die Bitterintensität verschiedener Bitterstoffe deutlich unterscheidet, wird die Bitterkeit einer Verbindung im Folgenden manchmal in relativen Bitterequivalenten (RBE) angegeben. Als Bezugssubstanz wird hier der bekannte Bitterstoff Coffein verwendet. Die Bestimmung des RBE-Wertes als Maß für die relative Bitterkeit einer Probe erfolgt mit Hilfe einer Skala von 1 bis 10. Eine relative Bitterkeit von 1, d.h. eine RBE-Wert von 1, entspricht dabei der Bitterkeit einer Menge von Coffein in der Dosierung von 100 mg/kg zu untersuchender Probe. Eine relative Bitterkeit von 5, d.h. eine RBE-Wert von 5, entspricht dabei der Bitterkeit einer Menge von Coffein in der Dosierung von 500 mg/kg zu untersuchender Probe. Die zu untersuchende Probe kann dabei in ihrer Zusammensetzung stark variieren. So kann es sich bei der zu untersuchenden Probe beispielsweise um eine der Ernährung, der Mundpflege, dem Genuss dienende Zubereitung, eine orale pharmazeutischen Zubereitung oder eine kosmetischen Zubereitung, beispielsweise um ein Lebensmittel, ein Getränk, ein Kaugummi, ein Mundwasser, ein Bonbon, einen Hustensaft oder eine Tablette handeln.

Die verwendete Skala zur Bestimmung der RBE-Werte entspricht ISO 4121 [Sensory Analysis - Guidelines for the use of quantitative response scales; A.3 Beispiel 2].

Die Auswahl der Panelisten zur Bestimmung der RBE-Werte erfolgt gemäß ISO 8586-1 [Sensory analysis - General guidance for the selection, training, and monitoring,of assessors - Part 1: Selected assessors].

Die Zahl der Panelisten entspricht dabei ISO 8586-I, 4.2.3 [Number of persons to be selected, zusammen mit ISO 6658 Sensory analysis - Methodology - General guidance - 5.3.5 Scoring (5 oder mehr ausgewählte Panelisten)].

Eine erfindungsgemäß bevorzugt Zubereitung ist dadurch gekennzeichnet, dass
der oder einer der unangenehm schmeckenden Stoffe ein Bitterstoff ist, der in einer Konzentration vorliegt, die mindestens 2 relativen Bitterequivalenten entspricht,
oder
mehrere oder sämtliche der unangenehm schmeckenden Stoffe Bitterstoffe sind, wobei die Gesamtkonzentration aller Bitterstoffe mindestens 2 relativen Bitterequivalenten entspricht.

Eine erfindungsgemäß bevorzugt Zubereitung ist dadurch gekennzeichnet, dass
die Gesamtmenge an Verbindungen der Formel **(I)** wie oben definiert und an physiologisch akzeptablen Salzen der Verbindungen der Formel **(I)** wie oben definiert ausreicht, um den unangenehmen, insbesondere bitteren, Geschmackseindruck des bzw. der unangenehm, insbesondere bitter, adstringierend und/oder metallisch, schmeckenden Stoffe sensorisch so zu verändern oder zu maskieren, dass er dem Geschmackseindruck einer Vergleichszubereitung entspricht, die (i) weder eine der oben definierten Verbindungen der Formel **(I)** noch ein physiologisch akzeptables Salz einer wie oben definierten Verbindung der Formel **(I)** sowie (ii) 90 Gew.-% oder weniger, vorzugsweise 80 Gew.-% oder weniger, bevorzugt 75 Gew.-% oder weniger, besonders bevorzugt 70 Gew.-% oder weniger des bzw. der unangenehm schmeckenden Stoffe enthält, aber ansonsten identisch zusammengesetzt ist,
und/oder
der oder einer der unangenehm schmeckenden Stoffe ein Bitterstoff ist, der in einer Konzentration von zumindest dem zweifachen seines Bitterschwellenwerts, vorzugsweise im Bereich des drei- bis tausendfachen seines Bitterschwellenwerts, vorliegt.

Insbesondere der bittere Geschmackseindruck von Methylxanthinen wie z.B. Coffein, Theobromin, Alkaloiden, wie z.B. Chinin, Flavonoiden wie z.B. Naringin, (Gallo)Catechinen und deren Gallaten, (Gallo-)-Epicatechinen und deren Gallaten, Phenolen wie z.B. Arbutin, Salicin, oder auch anorganischen Salzen wie Kaliumchlorid oder Magnesiumsulfat kann maskiert werden, wobei es besonders vorteilhaft ist, dass die erfindungsgemäß zu verwendenden Verbindungen, deren Gemische und/oder deren Salze (jeweils wie vorstehend definiert) in den bevorzugt verwendeten geringen Konzentrationen nahezu keinen Eigengeschmack besitzen.

Eine erfindungsgemäß bevorzugte Zubereitung enthält einen, zwei oder mehrere der folgenden Bitterstoffe:
- Catechine und Proanthocyanidine in einer Gesamtmenge von zumindest 0,01 Gew.-%, vorzugsweise von zumindest 0,05 Gew.-%, bevorzugt im Bereich von 0,075 Gew.-% bis 1 Gew.-%,
- Coffein und Theobromin in einer Gesamtmenge von zumindest 0,005 Gew.-%, vorzugsweise von zumindest 0,01 Gew.-%, bevorzugt von zumindest 0,02 Gew.-%, weiter bevorzugt im Bereich von 0,025 Gew.-% bis 1 Gew.-%,
- Naringin in einer Konzentration von zumindest 0,005 Gew.-%, vorzugsweise von zumindest 0,01 Gew.-%, bevorzugt im Bereich von 0,02 Gew.-% bis 0,5 Gew.-%,
- Süßstoffe in einer Gesamtmenge von zumindest 0,005 Gew.-%, vorzugsweise von zumindest 0,05 Gew.-%, bevorzugt im Bereich von 0,1 Gew.-% bis 2 Gew.-%,
jeweils bezogen auf das Gesamtgewicht der Zubereitung.

Der Mundpflege dienende Zubereitungen sind im Rahmen dieses Textes insbesondere Mund- und/oder Zahnpflegemittel wie Zahnpasten, Zahngele, Zahnpulver, Mundwässer, Kaugummis und andere Mundpflegemittel, enthaltend mindestens einen bitter schmeckenden Stoff mit einer RBE von 2 oder mehr.

Orale pharmazeutische Zubereitungen sind im Rahmen dieses Textes Zubereitungen, die z.B. in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden und mindestens einen bitter schmeckenden Stoff mit einer RBE von 2 oder mehr beinhalten.

Weitere übliche Wirk-, Grund-, Hilfs- und Zusatzstoffe für der Ernährung, der Mundpflege oder dem Genuss dienende oder orale pharmazeutische Zubereitungen können in Mengen von bis zu 99,9999999 Gew.-% enthalten sein, bezogen auf das Gesamtgewicht der Zubereitung. Ferner können die Zubereitungen Wasser in einer Menge bis zu 99,99 Gew.-% enthalten, vorzugsweise 5 bis 90 Gew.-%, bezogen auf das Gesamtgewicht der Zubereitung.

Die erfindungsgemäßen Zubereitungen, enthaltend eine oder mehrere Verbindungen der erfindungsgemäß zu verwendenden Formel **(I)** bzw. deren Salze oder Gemische, und werden gemäß einer bevorzugten Ausgestaltung hergestellt, indem die erfindungsgemäß zu verwendenden Verbindungen der Formel **(I),** Salze oder Gemische als Reinsubstanzen, als Lösung oder in Form eines Gemischs mit einem festen oder flüssigen Trägerstoff in eine der Ernährung, der Mundpflege oder dem Genuss dienende oder orale pharmazeutische Zubereitung eingearbeitet werden. Vorteilhafterweise können als Lösung vorliegende erfindungsgemäße Zubereitungen auch durch Sprühtrocknung in eine feste Zubereitung überführt werden.

Gemäß einer weiteren bevorzugten Ausführungsform werden zur Herstellung erfindungsgemäßer Zubereitungen die erfindungsgemäß zu verwendenden Verbindungen der Formel **(I)** bzw. deren Salze oder Gemische und gegebenenfalls andere Bestandteile der erfindungsgemäßen Zubereitung auch vorher in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet.

In einem weiteren bevorzugten Herstellungsverfahren für erfindungsgemäße Zubereitungen werden die erfindungsgemäß zu verwendenden Verbindungen der Formel **(I),** Salze oder Gemische vorher mit einem oder mehreren geeigneten Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße Zubereitung, bei der die Matrix so gewählt wird, dass die erfindungsgemäß zu verwendenden Verbindungen der Formel **(I),** Salze oder Gemische verzögert von der Matrix freigegeben werden, so dass man eine lang anhaltende Wirkung erhält.

Als weitere Bestandteile für erfindungsgemäße, der Ernährung oder dem Genuss dienende Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nukleinsäuren, Nucleotide, andere Geschmackskorrigenzien für unangenehme Geschmackseindrücke, Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), zusätzliche Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Erfindungsgemäße Zahnpflegemittel (als Beispiel für der Mundpflege dienende Zubereitungen), welche die erfindungsgemäß zu verwendenden Verbindungen der Formel **(I),** Salze oder Gemische enthalten, umfassen im allgemeinen ein abrasives System (Schleif oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke, Geschmackskorrigenzien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Als weitere Bestandteile für erfindungsgemäße (orale) pharmazeutische Zubereitungen können alle üblicherweise weiteren Wirk-, Grund-, Hilfs- und Zusatzstoffe für orale pharmazeutische Zubereitungen verwendet werden. Als Wirkstoffe können insbesondere auch unangenehm schmeckende oral formulierbare pharmazeutische Wirkstoffe verwendet werden. Die Wirk-, Grund-, Hilfs- und Zusatzstoffe können in an sich bekannter Weise in die oralen Applikationsformen überführt werden. Dies geschieht regelmäßig unter Verwendung inerter, nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) und Geruchskorrigentien sowie weitere und/oder nicht den bitteren Geschmack betreffende Geschmackskorrigentien.

Bevorzugt können die erfindungsgemäßen Zubereitungen auch ein oder mehrere (weitere) Riech-, Aroma- und/oder Geschmackstoffe enthalten, um den Geschmack und/oder Geruch der Zubereitung abzurunden und zu verfeinern. Geeignete Aromakompositionen enthalten z.B. synthetische oder natürliche Aromastoffe, Riechstoffe und/oder Geschmackstoffe sowie geeignete Hilfs- und Trägerstoffe. Als besonders vorteilhaft wird dabei angesehen, dass ein bitterer, adstringierender und/oder metallischer Geschmackseindruck, der von in den Zubereitungen ohne die erfindungsgemäß zu verwendenden Verbindungen der Formel **(I),** Salze oder Gemische (wie oben definiert) enthaltenen Aroma- oder Riechstoffen ausgehen würde, verändert und/oder maskiert werden kann und damit das gesamte Aroma- oder Geschmacksprofil verbessert wird.

Erfindungsgemäße Zubereitungen, die als Halbfertigwaren vorliegen, enthalten vorzugsweise zusätzlich mindestens einem weiteren (synthetischen oder natürlichen) Aromastoff, Riechstoff und/oder Geschmackstoff und können zur Veränderung oder Maskierung des unangenehmen Geschmackseindrucks von Fertigware-Zubereitungen dienen, die unter Verwendung der Halbfertigware-Zubereitung hergestellt werden.

Die Erfindung betrifft auch eine Mischung, vorzugsweise eine Halbfertigware zur Herstellung einer erfindungsgemäßen Zubereitung wie oben definiert, enthaltend
- ein, zwei oder mehrere verschiedene Verbindungen der Formel **(I)** wie oben definiert, oder
- ein, zwei oder mehrere verschiedene physiologisch akzeptable Salze eines, zweier oder mehrerer verschiedener Verbindungen der Formel **(I)** wie oben definiert, oder
- ein Gemisch eines, zweier oder mehrerer verschiedener Verbindungen der Formel **(I)** wie oben definiert mit einem, zwei oder mehreren verschiedenen physiologisch akzeptablen Salzen eines, zweier oder mehrerer verschiedener Verbindungen der Formel **(I)** wie oben definiert,
   und
   einen, zwei, drei, vier, fünf, sechs, sieben, acht, neun, zehn oder mehr weitere Aromastoffe enthält mit einem Molgewicht von größer als 90 g/mol, vorzugsweise von größer als 100 g/mol, bevorzugt mit einem Molgewicht im Bereich von 110 g/mol bis 300 g/mol, weiter bevorzugt mit einem Molgewicht im Bereich von 120 g/mol bis 250 g/mol, besonders bevorzugt mit einem Molgewicht im Bereich von 125 g/mol bis 220 g/mol, insbesondere bevorzugt mit einem Molgewicht im Bereich von 130 g/mol bis 210 g/mol,
   sowie vorzugsweise einen oder mehrere zum Verzehr geeignete Trägerstoffe, vorzugsweise ausgewählt aus der Gruppe bestehend aus Ethanol, Isopropanol, Glycerin, 1,2-Propylenglycol, Diacetin, Triacetin, Maltodextrin, Gummi Arabicum, Siliciumdioxid und deren Mischungen.

Die oben beschriebenen Ausgestaltungen, insbesondere die oben als bevorzugt bzw. besonders bevorzugt gekennzeichneten Ausgestaltungen, bezüglich der erfindungsgemäßen Verwendung der Verbindungen der Formeln **(I), (I-A), (I-A1), (I-A2), (I-B), (II)** und **(II-A)** gelten entsprechend für eine erfindungsgemäße Mischung, insbesondere für eine bevorzugte bzw. besonders bevorzugte erfindungsgemäße Mischung.

Die oben beschriebenen Ausgestaltungen, insbesondere die oben als bevorzugt bzw. besonders bevorzugt gekennzeichneten Ausgestaltungen, bezüglich einer erfindungsgemäßen Zubereitung gelten entsprechend für eine erfindungsgemäße Mischung, insbesondere für eine bevorzugte bzw. besonders bevorzugte erfindungsgemäße Mischung.

Eine bevorzugte erfindungsgemäße Mischung enthält einen oder mehrere zum Verzehr geeignete Trägerstoffe ausgewählt aus der Gruppe bestehend aus Glycerin, 1,2-Propylenglycol, Diacetin, Triacetin, Maltodextrin, Gummi Arabicum, Siliciumdioxid und deren Mischungen.

Eine besonders bevorzugt erfindungsgemäße Mischung ist dadurch gekennzeichnet, dass die Mischung
zwei, drei, vier, fünf, sechs, sieben, acht, neun, zehn oder mehr weitere Aromastoffe enthält mit einem Molgewicht von größer als 120 g/mol, vorzugsweise mit einem Molgewicht von größer als 125 g/mol, bevorzugt mit einem Molgewicht im Bereich von 125 g/mol bis 220 g/mol, insbesondere bevorzugt mit einem Molgewicht im Bereich von 130 g/mol bis 210 g/mol.

Eine bevorzugte erfindungsgemäße Mischung enthält vorzugsweise kein Hexan und kein Methylenchlorid, und bevorzugt kein Hexan, kein Methylenchlorid, kein Aceton und kein Methanol.

Eine bevorzugte erfindungsgemäße Mischung ist keine Mischung, die 4-Dihydroxy-5,7-dihydroxy-4-(4-hydroxyphenyl)coumarin, α-Tocopherolchinon, 7`-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Saikosaponin M, Hydropiperosid, Quercetin-3-0-β-D-glucuronid-6"-butylester und Quercetin-3-0-β-D-glucuronid-6"-methylester enthält,

Hier hinsichtlich der diesen Verbindungen entsprechenden Strukturformeln sei auch hier auf die Literaturstelle Chin. J. Appl. Environ. Biol. 2009, 15, 615-620 hingewiesen, welche hinsichtlich der darin offenbarten entsprechenden Verbindungen auf dem Wege der Verweisung Bestandteil dieser Anmeldung wird.

Eine bevorzugte erfindungsgemäße Mischung ist keine Mischung, die 4-Dihydroxy-5,7-dihydroxy-4-(4-hydroxyphenyl)coumarin, α-Tocopherolchinon, 7`-Dihydroxymatairesinol, (24S)-Ethylchloesta-3β,5a,6a-triol, Quercetin, Cucurbitacin IIa, Cucurbitacin U, lotroridosid A, Pokeweedcerebrosid 5 und Bonarosid im Massenverhältnis von 4 : 9 : 8 : 37 : 30 : 24 : 14 : 5 : 12 : 41 enthält.

Sofern eine erfindungsgemäße Mischung 4-Dihydroxy-5,7-dihydroxy-4-(4-hydroxyphenyl)coumarin, enthält, gelten eine, mehrere oder sämtliche der folgenden Bedingungen:
- das Gewichtsverhältnis von 4-Dihydroxy-5,7-dihydroxy-4-(4-hydroxyphenyl)coumarin zu Quercetin ist ungleich 4 : 30,
- das Gewichtsverhältnis von 4-Dihydroxy-5,7-dihydroxy-4-(4-hydroxyphenyl)coumarin zu Cucurbitacin IIa ist ungleich 4 : 24,
- das Gewichtsverhältnis von 4-Dihydroxy-5,7-dihydroxy-4-(4-hydroxyphenyl)coumarin zu Cucurbitacin U ist ungleich 4 : 14,
- das Gewichtsverhältnis von 4-Dihydroxy-5,7-dihydroxy-4-(4-hydroxyphenyl)coumarin zu 7'-Dihydroxymatairesinol ist ungleich 4 : 8,
und wobei vorzugsweise zumindest einer der in der Mischung enthaltenen unangenehm, insbesondere bitter, schmeckenden Stoffe nicht Quercetin, nicht Cucurbitacin IIa, nicht Cucurbitacin U und nicht 7'-Dihydroxymatairesinol ist.

Sofern eine erfindungsgemäße Mischung 4-Dihydroxy-5,7-dihydroxy-4-(4-hydroxyphenyl)coumarin, enthält, beträgt das Massenverhältnis von 4-Dihydroxy-5,7-dihydroxy-4-(4-hydroxyphenyl)coumarin zu der Gesamtmasse von α-Tocopherolchinon, 7'-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5 und Bonarosid nicht 4 : 180.

Eine bevorzugte erfindungsgemäße Mischung ist keine Mischung, die α-Tocopherolchinon, 7'-Dihydroxymatairesinol, (24S)-Ethylchloesta-3β,5a,6a-triol, Quercetin, Cucurbitacin Ila, Cucurbitacin U, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Saikosaponin M, Hydropiperosid, Quercetin-3-*O*-β-*D*-glucuronid-6"-butylester und Quercetin-3-*O*-β-*D*-glucuronid-6"-methylester enthält.

Eine bevorzugte erfindungsgemäße Mischung ist keine Mischung, die α-Tocopherolchinon, (24S)-Ethylchloesta-3β,5α,6α-triol, Cucurbitacin IIa, Cucurbitacin U, lotroridosid A, Pokeweedcerebrosid 5 und Bonarosid enthält.

Eine erfindungsgemäß bevorzugte Mischung enthält kein Cucurbitacin IIa, vorzugsweise kein Cucurbitacin IIa und kein Cucurbitacin U, und enthält bevorzugt keine Cucurbitacine.

Eine erfindungsgemäß bevorzugte Mischung ist dadurch gekennzeichnet, dass
die Mischung eine, zwei, drei, vier, fünf, sechs, sieben, acht oder sämtliche der folgenden Substanzen nicht enthält: Tocopherolchinon, 7'-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5 und Bonarosid,
und/oder
die Mischung keine der folgenden Substanzen enthält: (24S)-Ethylchloesta-3β,5a,6a-triol, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Hydropiperosid,

Erfindungsgemäße Mischungen, die in Form von Halbfertigwaren bzw. Aroma- und/oder Geschmackskompositionen vorliegen und damit nicht zum direkten Verzehr geeignet sind, und welche zur Herstellung einer zum direkten Verzehr geeigneten erfindungsgemäßen Zubereitung eingesetzt werden können, enthalten vorzugsweise eine Gesamtmenge an Verbindungen der Formel **(I)** wie oben definiert und an physiologisch akzeptablen Salzen der Verbindungen der Formel **(I)** wie oben definiert im Bereich von 0,001 Gew.-% bis 95 Gew.-%, bevorzugt im Bereich von 0,005 bis 80 Gew.-%, insbesondere bevorzugt im Bereich von 0,01 Gew.-% bis 50 Gew.-%, weiter bevorzugt im Bereich von 0,05 Gew.-% bis 40 Gew.-%, jeweils bezogen auf das Gesamtgewicht der Mischung.

Erfindungsgemäß zu verwendende Zubereitungen, die als Halbfertigwaren vorliegen, können zur Veränderung oder Maskierung des unangenehmen Geschmackseindrucks von Fertigware-Zubereitungen dienen, die unter Verwendung der Halbfertigware-Zubereitung hergestellt werden.

In einer besonders bevorzugten Ausführung der Erfindung werden die erfindungsgemäß zu verwendenden Verbindungen der Formel **(I),** Salze oder Gemische in den erfindungsgemäßen Zubereitungen in Kombination mit zumindest einer weiteren Substanz zum Verändern oder Maskieren des unangenehmen Geschmackseindrucks eines unangenehm schmeckenden Stoffes verwendet. Auf diese Weise kann eine besonders wirksame Maskierung erreicht werden. Insbesondere die Kombination der erfindungsgemäß einzusetzenden Verbindungen der Formel **(I),** Salze oder Gemische mit anderen Geschmackskorrigenzien für unangenehme, insbesondere bittere Geschmackseindrücke ist bevorzugt.

Weitere Substanzen zum Verändern oder Maskieren eines unangenehmen Geschmackseindrucks und/oder zum Verstärken eines angenehmen Geschmackseindrucks bzw. Geschmackskorrigenzien sind vorzugsweise ausgewählt aus der Gruppe bestehend aus Nucleotiden (z.B. Adenosin-5'-monophosphat, Cytidin-5'-monophosphat) oder deren physiologisch akzeptablen Salzen, Lactisolen, Natriumsalzen (z.B. Natriumchlorid, Natriumlactat, Natriumcitrat, Natriumacetat, Natriumgluconoat), Hydroxyflavanonen, dabei bevorzugt Eriodictyol, Sterubin (Eriodictyol-7-methylether), Homoeriodictyol, und deren Natrium-, Kalium-, Calcium-, Magnesium- oder Zinksalzen (insbesondere solche wie beschrieben in EP 1 258 200 A2, die bezüglich der darin offenbarten entsprechenden Verbindungen auf dem Wege der Verweisung Bestandteil dieser Anmeldung wird), Hydroxybenzoesäureamiden, dabei vorzugsweise 2,4-Dihydroxybenzoesäurevanillylamid, 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-*N-*(4-hydroxy-3-methoxybenzyl)amid, 2,4-Dihydroxybenzoesäure-*N*-(4-hydroxy-3-methoxybenzyl)amid-Mono-natriumsalz, 2,4-Dihydroxybenzoesäure-*N*-2-(4-hydroxy-3-methoxyphenyl)ethylamid, 2,4-Dihydroxybenzoesäure-*N*-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamiden (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, dabei vorzugsweise 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon und 1-(2-Hydroxy-4-methoxyphenyl)-2-(4-hydroxy-3-methoxyphenyl)ethanon) (insbesondere solche wie beschrieben in WO 2006/106023 beschrieben, die bezüglich der darin offenbarten entsprechenden Verbindungen auf dem Wege der Verweisung Bestandteil dieser Anmeldung wird); Hydroxyphenylalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); gamma-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird); Divanillinen (insbesondere solche wie beschrieben in WO 2004/078302, die bezüglich der darin offenbarten entsprechenden Verbindungen auf dem Wege der Verweisung Bestandteil dieser Anmeldung wird) und 4-Hydroxydihydrochalconen (vorzugsweise wie beschrieben in US 2008/0227867 A1, die bezüglich der darin offenbarten entsprechenden Verbindungen auf dem Wege der Verweisung Bestandteil dieser Anmeldung wird), dabei insbesondere Phloretin und Davidigenin, Aminosäuren oder Gemische von Molkeproteinen mit Lecithinen, Hesperetin wie in der WO 2007/014879 offenbart, die hinsichtlich dieser Verbindungen auf dem Wege der Verweisung Bestandteil dieser Anmeldung wird, 4-Hydroxydihydrochalkonen wie in der WO 2007/107596 offenbart, die hinsichtlich dieser Verbindungen auf dem Wege der Verweisung Bestandteil dieser Anmeldung wird, oder Propenylphenylglycosiden (Chavicolglycosiden) wie in EP 1 955 601 A1 beschrieben, die hinsichtlich dieser Verbindungen auf dem Wege der Verweisung Bestandteil dieser Anmeldung wird, oder Extrakten aus *Rubus suavissimus* wie in der europäischen Patentanmeldung mit der Anmeldenummer 11 165 566.8 (Symrise) beschrieben, die hinsichtlich dieser Extrakte auf dem Wege der Verweisung Bestandteil dieser Anmeldung wird, oder Extrakten aus *Hydrangea macrophylla* wie in EP 2 298 084 A1 beschrieben, die hinsichtlich dieser Extrakte auf dem Wege der Verweisung Bestandteil dieser Anmeldung wird, Pellitorin und abgeleiteten Aromakompositionen wie in EP 2 008 530 A1 beschrieben, die hinsichtlich dieser Aromakompositionen auf dem Wege der Verweisung Bestandteil dieser Anmeldung wird, Umami-Verbindungen wie in WO 2008/046895 A1 und EP 1 989 944 A1 beschrieben, die jeweils hinsichtlich dieser Verbindungen auf dem Wege der Verweisung Bestandteil dieser Anmeldung werden, Umami-Verbindungen wie beschrieben in EP 2 064 959 A1 bzw. EP 2 135 516 A1, die bezüglich der darin offenbarten entsprechenden Verbindungen auf dem Wege der Verweisung Bestandteil dieser Anmeldung werden sowie Vanillyllignanen wie beschrieben in der europäischen Patentanmeldung mit der Anmeldenummer 11 164 373.0 (Symrise), die auf dem Wege der Verweisung Bestandteil dieser Anmeldung werden.

Die Erfindung betrifft ferner ein Verfahren zum (a) Verändern oder Maskieren des unangenehmen, insbesondere bitteren, Geschmackseindrucks eines, zweier oder mehrerer unangenehm, insbesondere bitter, schmeckender Stoffe und/oder (b) Herstellen einer erfindungsgemäßen Zubereitung wie oben definiert, mit folgendem Schritt:

in Kontakt bringen oder Mischen des oder der unangenehm, insbesondere bitter, schmeckenden Stoffe mit
- ein, zwei oder mehrere verschiedene Verbindungen der Formel **(I)** wie oben definiert,
   oder
- ein, zwei oder mehrere verschiedene physiologisch akzeptable Salze eines, zweier oder mehrerer verschiedener Verbindungen der Formel **(I)** wie oben definiert,
oder
- ein Gemisch eines, zweier oder mehrerer verschiedener Verbindungen der Formel **(I)** wie oben definiert mit einem, zwei oder mehreren verschiedenen physiologisch akzeptablen Salzen eines, zweier oder mehrerer verschiedener Verbindungen der Formel **(I)** wie oben definiert.

### Beispiele

Die Beispiele dienen zur Verdeutlichung der Erfindung, ohne diese einzuschränken. Sofern nicht anders angegeben, beziehen sich alle Angaben auf das Gewicht.

### Allgemeine Arbeitsvorschrift (AAV 1)

Eine Suspension aus 20 mmol der entsprechenden Zimtsäurederivate, 30 mmol Phloroglucinol, 12 g Montmorillonit K-10 in 100 ml 1,4-Dioxan wird für 24 Stunden refluxiert. Anschließend wird der Katalysator abfiltriert und der Filterrückstand mit THF nachgewaschen. Die vereinigten Filtrate werden bis zur Trockene am Rotationsverdampfer eingeengt. Das eingeengte Filtrat wird in 250 ml Ethylacetat gelöst und jeweils mit 100 ml 5%-iger Natriumbicarbonat Lösung und 100 ml gesättigter Kochsalzlösung gewaschen. Die organische Phase wird über Natriumsulfat getrocknet und das Lösungsmittel am Rotationsverdampfer entfernt. Der so erhaltene Feststoff wird anschließend aus 200 bis 350 ml Wasser umkristallisiert.

### Beispiel 1: (4S)-5,7-Dihydroxy-4-(2-hydroxyphenyl)chroman-2-on (1) und (4R)-5,7-Dihydroxy-4-(2-hydroxyphenyl)chroman-2-on (2)

50 mmol Phloroglucin und 25 mmol o-Hydroxyzimtsäure wurden in 75 ml 1,4-Dioxan und 1,0 ml konzentrierte Schwefelsäure vorgelegt und für 12 Stunden unter Rückfluss erhitzt. Das Reaktionsgemisch wurde auf 300 ml Wasser gegossen und mit 250 ml Essigester extrahiert. Die vereinigten organischen Phasen wurden anschließend 5x mit 100 ml Wasser und 2x mit 100 ml 5%-iger Natriumbicarbonatlösung gewaschen, getrocknet und eingeengt. Der erhaltene Feststoff wurde aus Hexan/Aceton (2:1) umkristallisiert.

### Ausbeute: 4,7 mmol (19% der Theorie)

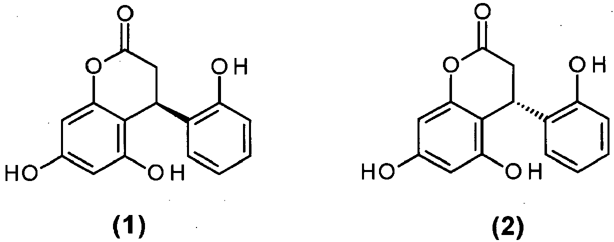

### Analysendaten:

**¹H-NMR (400 MHz, (CD₃)₂SO):** 2.81 (dd, *J* = 1.6/15.8 Hz, 1 H); 3.06 (dd, *J* = 7.4/15.8 Hz, 1H); 4.60 (d, *J* = 7.1 Hz, 1H); 6,03 (d, *J* = 2.3 Hz 1H); 6.16 (d, *J* = 2.3 Hz, 1H); 6.46 (dd, *J* = 1.7/7.6 Hz, 1 H); 6.61 (dt, *J* = 1.2/7.5 Hz, 1 H); 6.81 (dd, *J* = 1.1/8.0 Hz, 1H); 7.02 (dt, 1.7/7.6 Hz, 1 H); 9.52 (bs, 1 H); 9.66 (bs, 2H) ppm.

**¹³C-NMR (100 MHz, (CD₃)₂SO):** 28.9 (CH); 35.2 (CH₂); 94.4 (CH); 98.6 (CH); 102.2 (C); 115.0 (CH); 118.7 (CH); 126.8 (CH); 127.7 (CH); 127.8 (C); 153.5 (C); 154.6 (C); 155.2 (C); 157.7 (C); 167.9 (C=O) ppm.

**HRMS** [M-H; C₁₅H₁₁O₅]: ber.: 271,0612 gef.: 271.0655

### Beispiel 2: (4S)-5,7-Dihydroxy-4-(3-hydroxy-4-methoxy-phenyl)chroman-2-on (3) und (4R)-5,7-Dihydroxy-4-(3-hydroxy-4-methoxy-phenyl)chroman-2-on (4)

Nach **AAV1** wurden Phloroglycinol und Isoferulasäure miteinander umgesetzt.

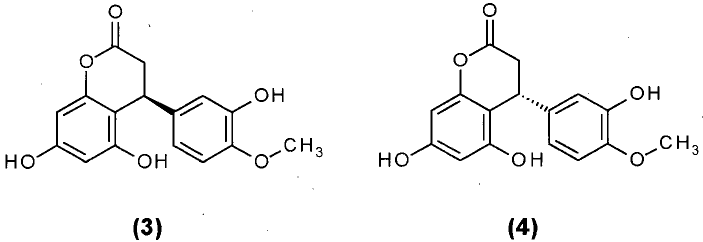

Ausbeute: 1,1 mmol (6 % der Theorie)

### Analysendaten:

**¹H-NMR (400 MHz, CD₃OD):** 2.90 (dd, *J* = 1.9/15.7 Hz, 1 H); 3.02 (dd, *J* = 6.9/15.7 Hz, 1H); 3.78 (s, 3H); 4.43 (dd, *J* = 1.9/6.9 Hz, 1H); 6,08 (dd, *J* = 0.4/2.3 Hz 1H); 6.16 (d, *J* = 2.3 Hz, 1H); 6.54 (ddd, *J* = 0.7/2.3/8.3 Hz, 1H); 6.58 (d, *J* = 2.2 Hz, 1H); 6.79 (d, *J* = 8.3Hz,1 H) ppm.

**¹³C-NMR (100 MHz, CD₃OD):** 35.2 (CH); 38.6 (CH₂); 56.4 (CH₃); 96.1 (CH); 99.9 (CH); 105.4 (C); 112.9 (CH); 115.1 (CH); 119.0 (CH); 136.4 (C); 147.6 (C); 147.9 (C); 154.6 (C); 156.9 (C); 159.3 (C); 170.6 (C=O) ppm.

**Massenspektrum (EI):** m/z (%) = 303 (20); 302 (M^{.+}, 100); 259 (34); 243 (30); 229 (19); 179 (16); 176 (39); 124 (52); 69 (24); 28 (17).

### Beispiel 3: (4S)-5,7-Dihydroxy-4-(4-hydroxy-3-methoxy-phenyl)chroman-2-on (5) und (4R)-5,7-Dihydroxy-4-(4-hydroxy-3-methoxy-phenyl)chroman-2-on (6)

Nach **AAV1** wurden Phloroglycinol und Ferulasäure miteinander umgesetzt.

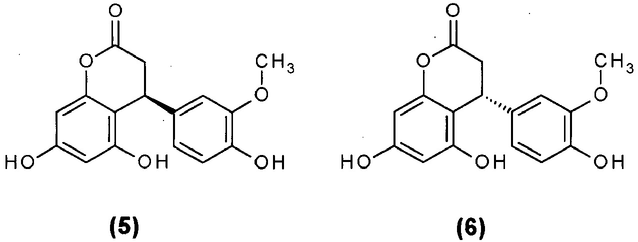

Ausbeute: 4,8 mmol (24 % der Theorie)

### Analysendaten:

**¹H-NMR (400 MHz, CD₃OD): 2.90** (dd, *J* = 2.0/15.7 Hz, 1H); 3.02 (dd, *J* = 6.8/15.7 Hz, 1H); 3.76 (s, 3H); 4.47 (dd, *J* = 2.0/6.8 Hz, 1H); 6,09 (dd, *J* = 0.4/2.3 Hz 1H); 6.17 (d, *J* = 2.3Hz,1 H); 6.61 (ddd, *J* = 0.7/2.1/8.2 Hz, 1 H); 6.67 (d, *J* = 8.2Hz,1 H); 6.73 (d, *J* = 2.1 Hz, 1 H) ppm.

**¹³C-NMR (100 MHz, CD₃OD):** 35.2 (CH); 38.6 (CH₂); 56.2 (CH₃); 96.1 (CH); 99.9 (CH); 105.5 (C); 111.7 (CH); 116.2 (CH); 120.1 (CH); 135.1 (C); 146.4 (C); 149.0 (C); 154.6 (C); 156.9 (C); 159.3 (C); 170.7 (C=O) ppm.

**Massenspektrum (EI):** m/z (%) = 303 (18); 302 (M^{·+}, 100); 259 (49); 244 (15); 243 (32); 229 (31); 179 (25); 176 (28); 124 (88); 77 (13).

### Beispiel 4: (4S)-5,7-Dihydroxy-4-(4-hydroxyphenyl)chroman-2-on (7) und (4R)-5,7-Dihydroxy-4-(4-hydroxyphenyl)chroman-2-on (8)

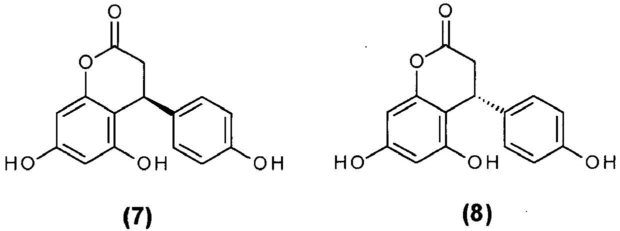

1.190 mol Phloroglycin und 0,912 mol *trans*-4-Hydroxyzimtsäure wurden in 850 ml 1,4-Dioxan vorgelegt und mit 0,187 mol konzentrierter Schwefelsäure versetzt. Das Reaktionsgemisch wurde für 16 Stunden unter Rückfluss erhitzt. Nach Abkühlen wurde der Reaktionsansatz auf 4 I destilliertes Wasser gegossen und über Nacht bei Raumtemperatur stehen gelassen. Das ausgefallene Rohprodukt wurde abfiltriert und mit Eiswasser gewaschen. Nach Umkristallisieren aus 1,4 I Wasser und 0,4 I Ethanol wurde das Produkt im Vakuumtrockenschrank bei 70°C vom Restlösungsmittel befreit.

Ausbeute: 0,273 mol (30 % der Theorie)

### Analysendaten:

**¹H-NMR (400 MHz, (CD₃)₂SO):** 2.76 (dd, *J* = 1.8/15.8 Hz, 1H); 3.12 (dd, *J* = 7.0/15.8 Hz, 1 H); 4.32 (dd, *J* = 1.2/6.7 Hz, 1 H); 6.01 (d, *J* = 2.2 Hz, 1 H); 6.16 (d, *J* = 2.3 Hz, 1 H); 6.65 (m, 2H); 6.85 (m, 2H); 9.26 (bs, 1 H); 9.53 (bs, 1 H); 9.69 (bs, 1H) ppm.

**¹³C-NMR (100 MHz, (CD₃)₂SO):** 32.8 (CH); 37.3 (CH₂); 94.5 (CH); 98.6 (CH); 103.5 (C); 115.1 (CH); 127.5 (CH); 132.4 (C); 152.8 (C); 155.2 (C); 155.9 (C); 157.6 (C); 167.9 (C=O) ppm.

**Massenspektrum (EI):** m/z (%) = 273 (13); 272 (M^{·+}, 77); 244 (12); 230 (16); 229 (100); 213 (39); 179 (39); 178 (25); 150 (19); 69 (27).

### Beispiel 5: (4S)-5,7-Dihydroxy-4-(4-methoxyphenyl)chroman-2-on (9) und (4R)- 5,7-Dihydroxy-4-(4-methoxyphenyl)chroman-2-on (10)

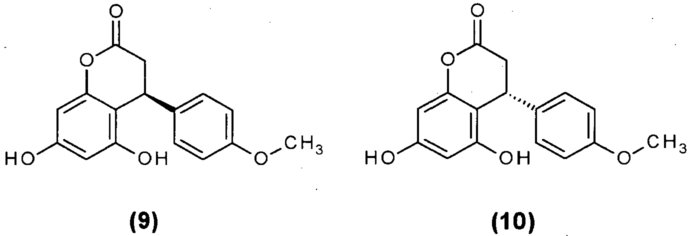

Nach **AAV1** wurden Phloroglycinol und *trans*-4-Methoxyzimtsäure miteinander umgesetzt.

Ausbeute: 2,6 mmol (13 % der Theorie)

### Analysendaten:

**¹H-NMR (400 MHz, CD₃OD):** 2.87 (dd, *J* = 1.9/15.7 Hz, 1H); 3.03 (dd, *J*= 6.9/15.7 Hz, 1H); 3.74 (s, 3H); 4.49 (dd, *J* = 1.9/6.9 Hz, 1H); 6,09 (dd, *J* = 0.4/2.3 Hz 1H); 6.16 (d, *J* = 2.3 Hz, 1 H); 6.80 (m, 2H); 6.01 (m, 2H) ppm.

**¹³C-NMR (100 MHz, CD₃OD):** 34.9 (CH); 38.6 (CH₂); 55.7 (CH₃); 96.1 (CH); 99.9 (CH); 105.4 (C); 115.0 (CH); 128.9 (CH); 135.5 (C); 154.6 (C); 156.9 (C); 159.4 (C); 160.0 (C); 170.5 (C=O) ppm.

**Massenspektrum (EI):** m/z (%) = 287 (12); 286 (M^{·+}, 68); 243 (54); 229 (11); 213 (37); 178 (22); 150 (16); 108 (100); 77 (10); 69 (20).

### Beispiel 6: (4S)-4-(1,3-Benzodioxol-5-yl)-5,7-dihydroxy-chroman-2-on (11) und (4R)-4-(1,3-Benzodioxol-5-yl)-5,7-dihydroxy-chroman-2-on (12)

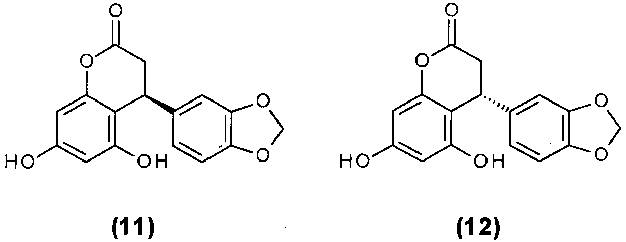

Nach **AAV1** wurden Phloroglycinol und *trans*-(3,4-Methylendioxy)zimtsäure miteinander umgesetzt.

Ausbeute: 0,8 mmol (4 % der Theorie)

### Analysendaten:

**¹H-NMR (400 MHz, (CD₃)₂SO):** 2.80 (dd, *J* = 1.9/16.0 Hz, 1 H); 3.15 (dd, *J* = 7.0/15.8 Hz, 1 H); 4.36 (d, *J* = 7.0 Hz, 1 H); 5,96 (s, 2H); 6.01 (d, *J* = 2.3 Hz, 1 H); 6.17 (d, *J* = 2.3 Hz, 1 H); 6.45 (ddd, *J* = 0.6/1.9/8.0 Hz, 1 H); 6.67 (d, *J* = 1.8 Hz, 1 H); 6.79 (d, *J* = 8.0 Hz, 1 H); 9.57 (bs, 1H); 9.76 (bs, 1 H) ppm.

**¹³C-NMR (100 MHz, (CD₃)₂SO):** 33.3 (CH); 37.1 (CH₂); 94.6 (CH); 98.6 (CH); 100.8 (CH₂); 103.1 (C); 107.2 (CH); 108.1 (CH); 119.2 (CH); 136.2 (C); 145.8 (C); 147.3 (C); 152.8 (C); 155.2 (C); 157.8 (C); 167.7 (C=O) ppm.

**Massenspektrum (EI):** m/z (%) = 301 (18); 300 (M^{·+}, 100); 258 (13); 257 (74); 174 (37); 122 (64); 115 (13); 69 (24); 63 (12); 28 (20).

### Beispiel 7: (4S)-4-(3,4-Dimethoxyphenyl)-5,7-dihydroxy-chroman-2-on (13) und (4R)-4-(3,4-Dimethoxyphenyl)-5,7-dihydroxy-chroman-2-on (14)

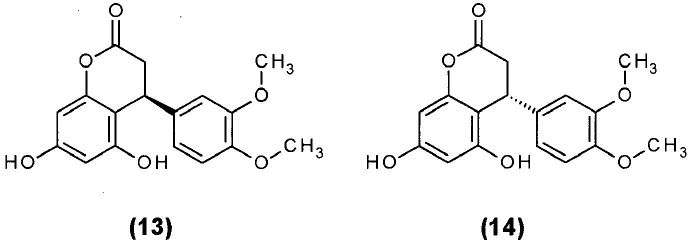

Nach **AAV1** wurden Phloroglycinol und *trans*-3,4-Dimethoxyzimtsäure miteinander umgesetzt.

Ausbeute: 3,6 mmol (18 % der Theorie)

### Analysendaten:

**¹H-NMR (400 MHz, CD₃OD):** 2.91 (dd, *J* = 2.0/15.7 Hz, 1H); 3.05 (dd, *J* = 6.9/15.7 Hz, 1 H); 3.75 (s, 3H); 3.77 (s, 3H); 4.50 (dd, *J* = 1.8/6.8 Hz, 1 H); 6,08 (dd, *J* = 0.4/2.2 Hz 1 H); 6.17 (d, *J* = 2.2 Hz, 1 H); 6.61 (ddd, *J* = 0.7/2.2/8.3 Hz, 1 H); 6.79 (d, *J* = 2.2 Hz, 1 H); 6.82 (d, *J* = 8.3 Hz, 1 H) ppm.

**¹³C-NMR (100 MHz, CD₃OD):** 35.3 (CH); 38.5 (CH₂); 56.4 (CH₃); 56.5 (CH₃); 96.2 (CH); 100.0 (CH); 105.4 (C); 112.2 (CH); 113.1 (CH); 120.0 (CH); 136.6 (C); 149.4 (C); 150.6 (C); 154.7 (C); 157.0 (C); 159.5 (C); 170.6 (C=O) ppm.

**Massenspektrum (EI):** m/z (%) = 317 (16); 316 (M^{·+}, 85); 273 (29); 259 (15); 243 (47); 190 (23); 139 (12); 138 (100); 77 (13); 69 (22).

### Beispiel 8: (4S)-4-(3,4-Dihydroxyphenyl)-5,7-dihydroxy-chroman-2-on (15) und (4R)-4-(3,4-Dihydroxyphenyl)-5,7-dihydroxy-chroman-2-on (16)

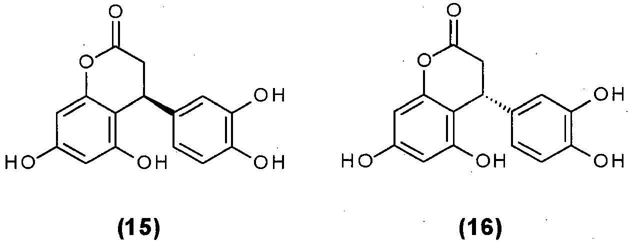

Eine Suspension aus 40,0 mmol Phloroglycin, 35,0 mmol trans-3,4-Dihydroxyzimtsäure und 16,0 g Montmorillonit in 150 ml 1,4-Dioxan wurde für 24 Stunden unter Rückfluss erhitzt. Anschließend wurde der Katalysator abfiltriert und der Filterrückstand mit THF gewaschen. Die vereinigten organischen Phasen wurden anschließend eingeengt und der Rückstand chromatographisch (Laufmittel: Essigester/Hexan 3:2) über Silicagel aufgereinigt. Anschließend wurde aus 100 ml entmineralisiertem Wasser umkristallisiert.

Ausbeute: 7,3 mmol (21 % der Theorie)

### Analysendaten:

**¹H-NMR (400 MHz, (CD₃)₂SO):** 2.72 (dd, *J* = 1.8/15.8 Hz; 1 H); 3.09 (dd, *J* = 7.0/15.8 Hz; 1 H); 4.25 (d, *J* = 6.8 Hz, 1 H); 6.00 (d, *J* = 2.2 Hz, 1 H); 6.15 (d, *J* = 8.3 Hz, 1 H); 6.34 (dd, *J* = 2.3/8.1 Hz; 1 H); 6.42 (d, *J* = 2.2 Hz, 1 H); 6.60 (d, *J* = 8.1 Hz, 1 H); 8.82 (bs, 2H); 9.63 (bs, 2H) ppm.

**¹³C-NMR (100 MHz, (CD₃)₂SO):** 33.0 (CH); 37.4 (CH₂); 94.5 (CH); 98.6 (CH); 103.6 (C); 114.0 (CH); 115.5 (CH); 117.3 (CH); 133.2 (C); 143.8 (C); 145.0 (C); 152.8 (C); 155.2 (C); 157.6 (C); 167.9 (C=O) ppm.

**HRMS** [M-H; C₁₅H₁₁O₆]: ber.: 287,0561 gef.: 287.0622

### Beispiel 9: (4S)-7-hydroxy-4-(4-hydroxyphenyl)chroman-2-one (17) und (4R)-7-hydroxy-4-(4-hydroxyphenyl)chroman-2-one (18)

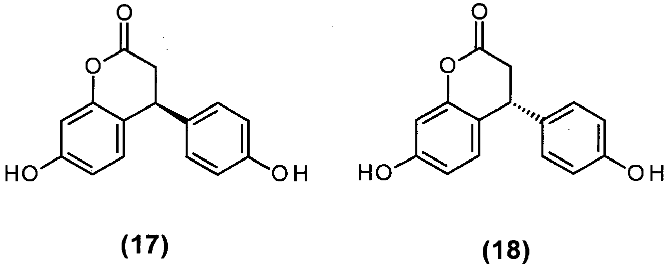

Eine Mischung aus 55 mmol *trans*-4-Hydroxyzimtsäure und 50 mmol Resorcin in 75 ml 1,4-Dioxan wurde mit 0,5 g para-Toluolsulfonsäure und 1,5 ml konzentrierter Schwefelsäure versetzt und für 12 Stunden unter Rückfluss erhitzt. Anschließend wurde ein Großteil des Lösungsmittels am Rotationsverdampfer entfernt und das Produktgemisch in 200 ml Ethylacetat aufgenommen. Die organische Phase wurde je zweimal mit 50 ml entmineralisiertem Wasser und 5%-iger Natriumbicarbonatlösung sowie einmal mit gesättigter Kochsalzlösung gewaschen. Nach Entfernen des Lösungsmittels wurde das Produkt säulenchromatographisch (Laufmittel: Essigester/Hexan 1:1) an Silicagel aufgereinigt, aus einem Lösungsmittelgemisch aus Diethylether/Pentan (4:15) umkristallisiert und das Produkt bei 60°C im Vakuumtrockenschrank getrocknet.

Ausbeute: 9,7 mmol (19 % der Theorie)

### Analysendaten:

**¹H-NMR (400 MHz, (CD₃)₂SO):** 2.94 (dd, *J* = 6.5/15.8 Hz, 1 H); 3.05 (dd, *J* = 6.0/15.8 Hz, 1 H); 4.24 (t, *J* = 6.1 Hz, 1 H); 6,51 (d, *J* = 2.3 Hz, 1 H); 6.54 (dd, *J* = 2.4/8.2 Hz, 1 H); 6.71 (m, 2H); 6.84 (m, 1 H); 6.92 (m, 2H) ppm.

**¹³C-NMR (100 MHz, (CD₃)₂SO):** 36.9 (CH₂); 37.9 (CH); 103.2 (CH); 111.5 (CH); 115.4 (CH); 116.7 (C); 128.1 (CH); 128.8 (CH); 131.8 (C); 151.8 (C); 156.2 (C); 157.4 (C); 167.8 (C=O) ppm.

**Massenspektrum (EI):** m/z (%) = 256 (M^{·+}, 76); 228 (26); 214 (20); 213 (100); 197 (42); 115 (15); 77 (21); 65 (17); 51 (15); 39 (21).

### Beispiel 10: (4S)-5-hydroxy-4-(4-hydroxy-3-methoxy-phenyl)-7-methoxy-chroman-2-one (19) und (4R)-5-hydroxy-4-(4-hydroxy-3-methoxy-phenyl)-7-methoxy-chroman-2-one (20) sowie (4S)-7-hydroxy-4-(4-hydroxy-3-methoxy-phenyl)-5-methoxy-chroman-2-on (21) und (4R)-7-hydroxy-4-(4-hydroxy-3-methoxy-phenyl)-5-methoxy-chroman-2-on (22)

Eine Mischung aus 17,5 mmol *trans*-4-Hydroxyzimtsäure und 17,5 mmol 5-Methylresorcin in 40 ml 1,4-Dioxan wurde mit 0,5 ml konzentrierter Schwefelsäure versetzt und für 12 Stunden unter Rückfluss erhitzt. Nach Abkühlen wurde das Reaktionsgemisch auf 250 ml entmineralisiertes Wasser gegossen und mit 150 ml Essigsäureethylester (EE) extrahiert. Die organische Phase wurde je zweimal mit 50 ml entmineralisiertem Wasser und 50 ml 5%-iger Natriumbicarbonatlösung gewaschen und über Natriumsulfat getrocknet. Nach Entfernen des Lösungsmittels wurde das Produkt säulenchromatographisch (Laufmittel: Essigester/Hexan 2:3) an Silicagel aufgereinigt. Das Produkt bestand gemäß NMR aus zwei Isomeren, die im Verhältnis 37:63 (19/20:21/22) vorlagen. Die weitere Auftrennung wurde mittels präparativer HPLC durchgeführt (Phenomenex Luna C18 (2), 5µm, 150x21,5mm; Acetonitril/Wasser 75:25; 25 ml/min; 105 bar)

Ausbeute: 9,8 mmol (56 % der Theorie)

### Beispiel 10a: (4S)-5-hydroxy-4-(4-hydroxy-3-methoxy-phenyl)-7-methoxy-chroman-2-one (19) und (4R)-5-hydroxy-4-(4-hydroxy-3-methoxy-phenyl)-7-methoxy-chroman-2-one (20)

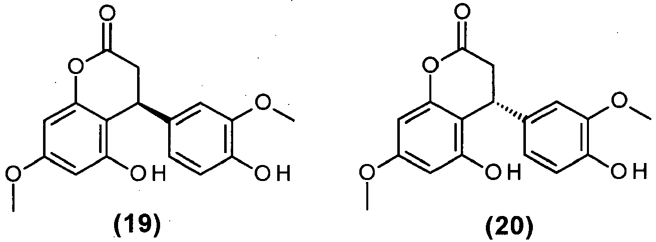

### Analysendaten:

**Retentionszeit:** 20,3 min - 21,8 min

**¹H-NMR (400 MHz, CDCl₃):** 2.97 (dd, *J* = 3.6/15.8 Hz; 1H); 3.03 (dd, *J* = 6.1/15.8 Hz; 1 H); 3.79 (s, 3H); 3.82 (s, 3H); 4.44 (dd, *J* = 3.6/6.1 Hz, 1 H); 4.96 (bs, 1 H); 5.53 (bs, 1 H); 6.21 (d, *J* = 2.3 Hz, 1 H); 6.35 (d, *J* = 2.3 Hz, 1 H); 6.62 (d, *J* = 2.0 Hz; 1 H); 6.64 (dd, *J* = 2.0/8.6 Hz, 1 H); 6.82 (d, *J* = 8.6 Hz, 1 H) ppm.

**¹³C-NMR (100 MHz, (CD₃)₂SO):** 33.2 (CH); 37.1 (CH₂); 55.1 (CH₃); 55.5 (CH₃); 93.0 (CH); 97.5 (CH); 105.2 (C); 111.3 (CH); 115.2 (CH); 118.2 (CH); 132.7 (C); 145.3 (C); 147.5 (C); 154.0 (C); 155.3 (C); 159.5 (C); 167.8 (C=O) ppm.

**HRMS** [M-H; C₁₇H₁₅O₆]: ber.: 315,0874 gef.: 315.0902

### Beispiel 10b: (4S)-7-hydroxy-4-(4-hydroxy-3-methoxy-phenyl)-5-methoxy-chroman-2-on (21) und (4R)-7-hydroxy-4-(4-hydroxy-3-methoxy-phenyl)-5-methoxy-chroman-2-on (22)

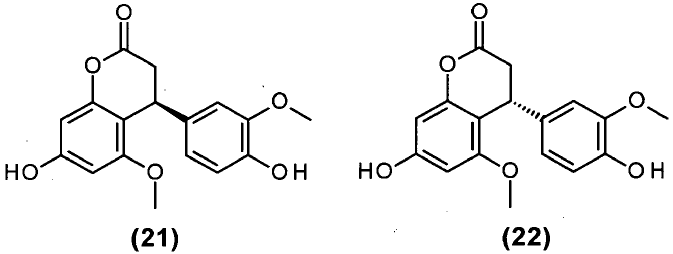

### Analysendaten:

**Retentionszeit:** 17,0 min - 19,8 min

**¹H-NMR (400 MHz, CDCl₃):** 2.95 - 2.99 (kB, 2H); 3.76 (s, 3H); 3.81 (s, 3H); 4.48 (dd, *J* = 3.9/4.7 Hz, 1H); 5.19 (bs, 1H); 5.49 (bs, 1 H); 6.25 (d, *J* = 2.3 Hz, 1 H); 6.27 (d, *J* = 2.3 Hz, 1 H); 6.59 (dd, *J* = 2.1/7.9 Hz; 1 H); 6.61 (d, *J* = 2.1 Hz, 1 H); 6.79 (d, *J* = 7.9 Hz, 1 H) ppm.

**¹³C-NMR (100 MHz, (CD₃)₂SO):** 33.1 (CH); 38.8 - 40.1 (CH₂; verdeckt durch Lösungsmittelsignal); 55.5 (CH₃); 55.6 (CH₃); 95.4 (CH); 95.6 (CH); 104.3 (C); 111.1 (CH); 115.2 (CH); 118.1 (CH); 132.7 (C); 145.3 (C); 147.5 (C); 152.5 (C); 157.0 (C); 158.2 (C); 167.7 (C=O) ppm.

**HRMS** [M-H; C₁₇H₁₅O₆]: ber.: 315,0874 gef.: 315.0920

### Beispiel 11: (4S)-7-hydroxy-4-(4-hydroxy-3-methoxy-phenyl)chroman-2-on (23) und (4R)-7-hydroxy-4-(4-hydroxy-3-methoxy-phenyl)chroman-2-on (24)

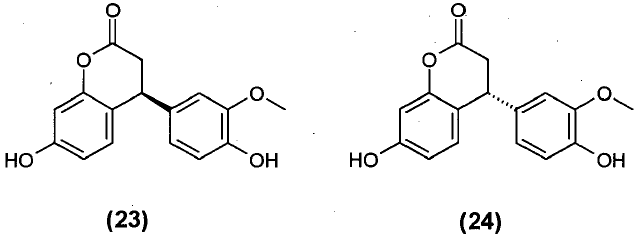

Eine Mischung aus 100 mmol Ferulasäure und 120 mmol Resorcin in 150 ml 1,4-Dioxan wurde mit 5 mmol p-Toluolsulfonsäure versetzt und für 14 Stunden unter Rückfluss erhitzt. Anschließend wurde das Reaktionsgemisch zur Trockene eingeengt und aus einer Mischung bestehend aus 450 ml Wasser und 150 ml Ethanol umkristallisiert. Das Kristallisat wurde in 250 ml Essigsäureethylester gelöst, die organische Phase zweimal mit je 150 ml 5%-iger Natriumbicarbonatlösung und einmal mit 100 ml gesättigter Kochsalzlösung gewaschen und über Natriumsulfat getrocknet. Nach Entfernen des Lösungsmittels wurde das Produkt bei 60 °C im Vakuumtrockenschrank getrocknet.

Ausbeute: 22,6 mmol (23 % der Theorie)

### Analysendaten:

**¹H-NMR (400 MHz, (CD₃)₂SO):** 3.00 (dd, *J* = 6.6/15.9 Hz, 1 H); 3.05 (dd, *J* = 6.1/15.8 Hz, 1H); 3.72 (s, 3H); 4.24 (t, *J* = 6.3 Hz, 1H); 6,45 (dd, *J* = 2.1/8.1 Hz, 1H); 6.51 (d, *J* = 2.3 Hz, 1 H); 6.54 (dd, *J* = 2.4/8.2 Hz, 1 H); 6.70 (d, *J* = 8.1 Hz, 1 H); 6.78 (d, *J* = 2.1 Hz, 1 H), 6.84 (d, *J* = 8.3 Hz, 1 H); 8.97 (bs, 1 H); 9.68 (bs, 1 H) ppm.

**¹³C-NMR (100 MHz, (CD₃)₂SO):** 36.8 (CH₂); 38.3 (CH); 55.5 (CH₃); 103.1 (CH); 111.5 (CH); 111.6 (CH); 115.4 (CH); 116.6 (C); 119.3 (CH); 128.8 (CH); 132.4 (C); 145.5 (C); 147.6 (C); 151.8 (C); 157.4 (C); 167.9 (C=O) ppm.

**Massenspektrum (EI):** m/z (%) = 287 (18); 286 (M˙⁺, 100); 253 (14); 243 (51); 228 (17); 227 (36); 213 (39); 176 (18); 115 (17); 77 (15).

### Beispiel 12: (4S)-7-Hydroxy-4-(3-hydroxy-4-methoxy-phenyl)chroman-2-on (25) und (4R)-7-Hydroxy-4-(3-hydroxy-4-methoxy-phenyl)chroman-2-on (26)

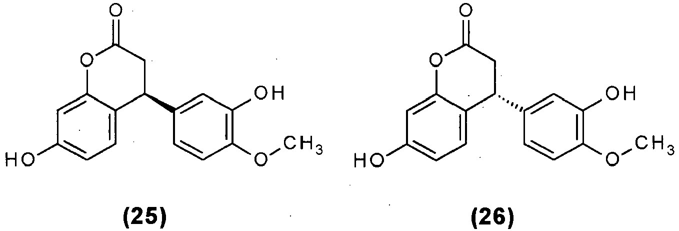

Eine Mischung aus 50 mmol Isoferulasäure und 75 mmol Resorcin in 75 ml 1,4-Dioxan wurde mit 0,5 g *para*-Toluolsulfonsäure und 1,5 ml konzentrierter Schwefelsäure versetzt und für 14 Stunden unter Rückfluss erhitzt. Da die Umsetzung laut LC-MS zu diesem Zeitpunkt nicht vollständig war, wurden weitere 1,5 ml konzentrierter Schwefelsäure zugegeben und das Gemisch für weitere 10 Stunden erhitzt. Anschließend wurde ein Großteil des Lösungsmittels am Rotationsverdampfer entfernt und das Produktgemisch in 200 ml Ethylacetat aufgenommen. Die organische Phase wurde je zweimal mit 50 ml entmineralisiertem Wasser und 5%-iger Natriumbicarbonatlösung sowie einmal mit gesättigter Kochsalzlösung gewaschen. Die organische Phase wurde danach über Natriumsulfat getrocknet und nach Entfernen des Lösungsmittels das Produkt säulenchromatographisch (Laufmittel: Essigester/Hexan 4:5) an Silicagel aufgereinigt, aus einem Lösungsmittelgemisch aus Diethylether/Pentan (1:6) umkristallisiert und das resultierende Produkt bei 60°C im Vakuumtrockenschrank getrocknet.

Ausbeute: 21,6 mmol (43 % der Theorie)

### Analysendaten:

**¹H-NMR (400 MHz, (CD₃)₂SO):** 2.90 (dd, *J* = 5.9/15.8 Hz, 1 H); 3.07 (dd, *J* = 6.1/15.8 Hz, 1H); 3.72 (s, 3H); 4.21 (t, *J* = 5.9 Hz, 1 H); 6,50 - 6.53 (kB, 3H); 6.54 (dd, *J* = 2.4/8.2 Hz, 1H); 6.84 (d, *J* = 8.4 Hz, 1 H); 6.87 (d, *J* = 8.2 Hz, 1 H); 9.02 (bs, 1 H); 9.68 (bs, 1 H) ppm. **¹³C-NMR (100 MHz, (CD₃)₂SO):** 36.9 (CH₂); 38.1 (CH); 55.5 (CH₃); 103.2 (CH); 111.6 (CH); 112.4 (CH); 114.3 (CH); 116.4 (C); 117.7 (CH); 129.0 (CH); 134.4 (C); 146.5 (C); 146.6 (C); 151.8 (C); 157.4 (C); 167.7 (C=O) ppm.

**Massenspektrum (EI):** m/z (%) = 287 (19); 286 (M˙⁺, 100); 253 (21); 243 (42); 229 (21); 227 (41); 213 (25); 176 (24); 115 (21); 77 (20).

### Beispiel 13: (4S)-4-(3,4-Dihydroxyphenyl)-7-hydroxy-chroman-2-on (27) und (4R)-4-(3,4-Dihydroxyphenyl)-7-hydroxy-chroman-2-on (28)

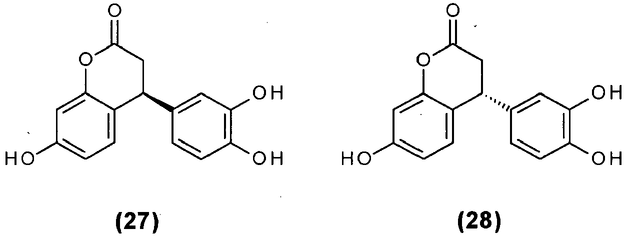

Eine Mischung aus 55 mmol Kaffeesäure und 50 mmol Resorcin in 75 ml 1,4-Dioxan wurde mit 0.5 g *para*-Toluolsulfonsäure und 1,5 ml konzentrierter Schwefelsäure versetzt und für 12 Stunden unter Rückfluss erhitzt. Anschließend wurde ein Großteil des Lösungsmittels am Rotationsverdampfer entfernt und das Produktgemisch in 200 ml Ethylacetat aufgenommen. Die organische Phase wurde je zweimal mit 50 ml entmineralisiertem Wasser und 5%-iger Natriumbicarbonatlösung sowie einmal mit gesättigter Kochsalzlösung gewaschen. Die organische Phase wurde über Natriumsulfat getrocknet und nach Entfernen des Lösungsmittels das Produkt säulenchromatographisch (Laufmittel: Essigester/Hexan 1:1) an Silicagel aufgereinigt, aus einem Lösungsmittelgemisch aus Diethylether/Pentan (1:6) umkristallisiert und das so erhaltene Produkt bei 60°C im Vakuumtrockenschrank getrocknet.

Ausbeute: 9,9 mmol (20 % der Theorie)

### Analysendaten:

**¹H-NMR (400 MHz, (CD₃)₂SO):** 2.87 (dd, *J* = 5.8/15.8 Hz, 1 H); 3.05 (dd, *J* = 6.1/15.8 Hz, 1H); 4.16 (t, *J* = 5.9 Hz, 1 H); 6,39 (ddd, *J* = 0.5/2.2/8.0 Hz, 1 H); 6.47 (dd, *J* = 0.3/2.2 Hz, 1H); 6.50 (d, *J* = 2.4 Hz, 1 H); 6.54 (dd, *J* = 2.4/8.3 Hz, 1 H); 6.66 (d, *J* = 8.0 Hz, 1 H), 6.87 (dd, *J* = 0.7/8.3 Hz, 1 H); 9.11 (bs, 3H) ppm.

**¹³C-NMR (100 MHz, (CD₃)₂SO):** 37.0 (CH₂); 38.1 (CH); 103.1 (CH); 111.5 (CH); 114.3 (CH); 115.6 (CH); 116.6 (C); 117.8 (CH); 129.0 (CH); 132.6 (C); 144.1 (C); 145.2 (C); 151.8 (C); 157.4 (C); 167.8 (C=O) ppm.

**Massenspektrum (EI):** m/z (%) = 272 (M^{•+}, 100); 254 (31); 229 (70); 213 (71); 162 (25); 115 (21); 77 (28); 63 (20); 51 (24); 39 (18).

### Anwendungsbeispiel 1: Bitter-Reduzierung einer Bitterstofflösung

Um die Reduzierung (d.h. die Maskierung oder Verminderung) des Bitter-Eindrucks in einer Probe zu quantifizieren, wurde die Bitterkeit einer Lösung enthaltend 500 ppm Coffein oder 200 ppm Theobromin oder 100 ppm Naringin oder 250 ppm Salicin von einer Expertengruppe jeweils mit einer Probe verglichen, die 500 ppm Coffein oder 200 ppm Theobromin oder 100 ppm Naringin oder 250 ppm Salicin und zusätzlich die jeweils angegebene Menge einer (hinsichtlich der Fähigkeit zur Bitter-Reduzierung) zu beurteilenden Substanz enthielt (Einstufung: 1 [nicht bitter] bis 10 [extrem bitter]). Für die Auswertung, d.h. die Berechnung der Reduktion (in %) des Bittereindrucks wurden jeweils die Durchschnittswerte der Einschätzungen der Expertengruppe verwendet.

| Test-Substanz | Bitterstoff | Bitter-Eindruck (1-10) | | % Reduktion des Bitter-Eindrucks (Signifikanz) |
|---|---|---|---|---|
| | | a) ohne Test-substanz | b) mit Test-substanz | |
| 5 ppm **(1) + (2)** (Verhältnis 1:1) | 100 ppm Naringin | 5,5 ± 3,0 | 3,7 + 2,6 | 31,7 % (p < 0,07) |
| 50 ppm **(5) + (6)** (Verhältnis 1:1) | 500 ppm Coffein | 3,9 ± 1,7 | 3,0 + 1,3 | 23,5 % (p < 0,05) |
| 25 ppm **(5) + (6)** (Verhältnis 1:1) | 300 ppm Theobromin | 4,4 ± 1,5 | 3,1 ± 1,6 | 30,3% (p < 0,03) |
| 25 ppm **(5) + (6)** (Verhältnis 1:1) | 100 ppm Naringin | 6,1 ±2,1 | 3,1 ± 1,4 | 49,0% (p < 0,0001) |
| 50 ppm **(5) + (6)** (Verhältnis 1:1) | 250 ppm Salicin | 6,6 ± 2,1 | 5,9 ± 2,5 | 11,1 % |
| 25 ppm **(7) + (8)** (Verhältnis 1:1) | 100 ppm Naringin | 5,6 ± 2,5 | 2,9 ± 1,9 | 48,6% (p < 0,002) |
| 10 ppm **(7) + (8)** (Verhältnis 1:1) | 300 ppm Theobromin | 4,2 ± 2,2 | 3,6 ± 1,5 | 21,7% |
| 25 ppm **(7) + (8)** (Verhältnis 1:1) | 500 ppm Coffein | 4,0 ± 2,0 | 3,4 + 1,4 | 15,5% |
| 25 ppm **(17) + (18)** (Verhältnis 1:1) | 100 ppm Naringin | 5,3 ± 2,2 | 3,0 ± 1,6 | 44,3% (p < 0,01) |
| 25 ppm **(23) + (24)** (Verhältnis 1:1) | 300 ppm Theobromin | 4,5 ± 1,9 | 3,5 ± 1,6 | 22,4 % (p < 0,1) |

### Anwendungsbeispiel 2: Bitter-/Adstringenz-Reduzierung einer Bitterlösung im Zeitverlauf

Um die Reduzierung (d.h. die Maskierung oder Verminderung) des Bitter, bzw. Adstringenz-Eindrucks in einer Probe über einen bestimmten Zeitverlauf zu quantifizieren, wurde die Bitterkeit, bzw. Adstringenz einer Lösung enthaltend 750 ppm Epigallocatechingallat (EGCG) sowie 125 ppm Ascorbinsäure von einer Expertengruppe jeweils mit einer Probe verglichen, die 750 ppm Epigallocatechingallat, 125 ppm Ascorbinsäure und zusätzlich die jeweils angegebene Menge einer (hinsichtlich der Fähigkeit zur Bitter-Reduzierung) zu beurteilenden Substanz enthielt, in diesem Fall eine racemische Mischung der Verbindungen **(7)** und **(8)** (Einstufung: 1 [nicht bitter] bis 10 [extrem bitter]; bzw. 1 [nicht adstringierend] bis 10 [extrem adstringierend]). Die Bewertung der Panelisten erfolgte zu festgelegten Zeitpunkten (10 Sekunden, 30 Sekunden, 50 Sekunden und 70 Sekunden nach Aufnahme der jeweiligen Probe). Die Proben wurden nacheinander nach Neutralisierung durch die Panelisten bewertet. Für die Auswertung, d.h. die Berechnung der Reduktion (in %) des Bitter-/Adstringenzeindrucks wurden zu jeden Meßzeitpunkt jeweils die Durchschnittswerte der Einschätzungen der Expertengruppe für die Epigallocatechingallatlösung und die zu vergleichende, Epigallocatechingallat und eine zu beurteilende Substanz enthaltende Probe verwendet.

| Test-Substanz | Bitterstoff | Zeit (sek.) | % Reduktion des Bitter-Eindrucks | % Reduktion des Adstringenz-Eindrucks |
|---|---|---|---|---|
| 50 ppm **(7) + (8)** (Verhältnis 1:1) | 750 ppm EGCG | 0 | -14 % | -1 % |
| | | 10 | -22 %* | -7 % |
| | | 30 | -25 %* | -7 % |
| | | 50 | -35 %* | -19 % |
| | | 70 | -29 % | -15 % |

| | | | | |
|---|---|---|---|---|
| * signifikant | | | | |

### Anwendungsbeispiel 3: Mischungen

| Inhaltsstoff | Mischung (Einsatz in Gew.-%) | | | | | | | |
|---|---|---|---|---|---|---|---|---|
| | A | B | C | D | E | F | G | H |
| **(7) + (8)** (Verhältnis 1:1) | 5 | 7 | 10 | 15 | 15 | 20 | 20 | 25 |
| Homoeriodictyol | 5 | | 2,5 | | | | | |
| Eriodictyol | | 2,5 | | | | | | |
| Phloretin | | | | 1 | | | | |
| Hesperetin | | | | | 0,5 | | | |
| 10 Gew.-% trans-Pellitorin (z.B. nach WO 2004/043906) in 1,2-Propylenglycol/ Diethylmalonat | | | | 0,25 | 0,25 | 0,5 | 0,25 | |
| Extrakt aus *Hydrangea macrophylla* gemäß EP 2 298 084 A1, enthaltend Phyllodulcin | 2,5 | | 2,5 | | | | | 2,5 |
| Extrakt aus *Rubus suavissimus* enthaltend Rubusosid gemäß der europäischen Patentanmeldung mit der Anmeldenummer 11 165 566.8 (Symrise) | | 2,5 | 2,5 | | | | | |
| 1,2-Propylenglycol | - | 20 | - | ad 100 | ad 100 | 20 | ad 100 | - |
| Glycerin | - | ad 100 | - | 20 | 20 | ad 100 | 20 | - |
| Maltodextrin | ad 100 | - | ad 100 | - | - | - | - | ad 100 |

Die Stoffe bzw. Lösungen werden in den oben angegebenen Mengenverhältnissen gemischt und dann mit 1,2-Propylenglycol und/oder Glycerin aufgenommen und durch leichtes Erwärmen vollständig gelöst bzw. mit den festen Trägerstoffen homogen gemischt.

### Anwendungsbeispiel 4: Sprühgetrocknete Halbfertigware

| Inhaltsstoff | Einsatz in Gew.-% | | |
|---|---|---|---|
| | A | B | C |
| Trinkwasser | 60,8 | 60,8 | 60,8 |
| Maltodextrin aus Weizen | 24,3 | 24,3 | 24,3 |
| Gummi Arabicum | 6,1 | 6,1 | 6,1 |
| **(7) + (8)** (Verhältnis 1:1) | 8,8 | --- | 4,4 |
| **(3) + (4)** (Verhältnis 1:1) | --- | 8,8 | 4,4 |

Das Trinkwasser wurde in einem Behälter vorgelegt und das Maltodextrin und das Gummi Arabicum darin gelöst. Anschließend wurde Verbindung 1 und/oder Verbindung 2 mit einem Turrax in die Trägerstofflösung emulgiert. Die Temperatur der Sprühlösung sollte dabei 30°C nicht überschreiten. Das Gemisch wurde dann sprühgetrocknet (Solltemperatur Eingang: 185 - 195°C, Solltemperatur Ausgang: 70 - 75°C). Die resultierende sprühgetrocknete Halbfertigware enthielt ca. 18 - 22 % der Verbindungen **(7)** + **(8)** und/oder **(3)** + **(4).**

### Anwendungsbeispiel 5: Tee-Zubereitung

| Inhaltsstoff | Einsatz in Gew.-% | | |
|---|---|---|---|
| | A | B | C |
| Schwarzer Tee, Ceylon, Blattware | 94 | --- | --- |
| Grüner Tee, China, Blattware | --- | 92 | --- |
| Mate Tee, Peru, Blattware | --- | --- | 95 |
| Mischung A aus Anwendungsbeispiel 3 | 6 | --- | --- |
| Mischung B aus Anwendungsbeispiel 3 | --- | 8 | --- |
| Mischung C aus Anwendungsbeispiel 3 | --- | --- | 5 |

Der Tee und die Halbfertigware werden gemischt und in Teebeutel aus Filterpapier abgepackt (je 2 g pro Filterbeutel). Zur Anwendung wird ein Teebeutel in 100 - 250 ml kochendes Wasser aufgegossen und 2 - 5 min ziehen gelassen.

### Anwendungsbeispiel 6: Schwarztee-Zubereitung

| Inhaltsstoff | Einsatz in Gew.-% |
|---|---|
| Schwarzer Tee, Ceylon, Blattware | 94 |
| Mischung A aus Anwendungsbeispiel 3 | 3 |
| Mischung B aus Anwendungsbeispiel 3 | 3 |

Der Tee und die Halbfertigwaren werden gemischt und in Teebeutel aus Filterpapier abgepackt (je 2 g pro Filterbeutel). Zur Anwendung wird ein Teebeutel in 100 - 250 ml kochendes Wasser aufgegossen und 2 - 5 min ziehen gelassen.

### Anwendungsbeispiel 7: Verwendung in einem Eistee-Getränk (Schwarztee)

Die Verbindungen **(7)** und **(8)**, bzw. **(3)** und **(4)** wurden jeweils 10 % in Ethanol vorgelöst. Schwarztee-Extrakt wurde in Wasser gelöst und zusammen mit Zucker, einer Aromazubereitung (Pfirsichgeschmack), sowie den ethanolischen Lösungen der Verbindungen **(7)** und **(8)** (Zubereitung A), bzw. **(3)** und **(4)** (Zubereitung B) in einem Becherglas verrührt.

| Inhaltsstoff | Einsatz in Gew.-% | |
|---|---|---|
| | A | B |
| Schwarztee-Extrakt | 1,4 | 1,4 |
| Wasser | 89,5 | 89,5 |
| Aromazubereitung (Typ Pfirsich) | 0,67 | 0,67 |
| Zucker | 7 | 7 |
| Citronensäure (kristallin) | 1,2 | 1,2 |
| Ascorbinsäure | 0,2 | 0,2 |
| **(7)** und **(8)** im Verhältnis 1:1 als 10%ige Lösung in Ethanol | 0,03 | --- |
| **(3)** und **(4)** im Verhältnis 1:1 als 10%ige Lösung in Ethanol | --- | 0,03 |

### Anwendungsbeispiel 8: Verwendung in einem Eistee-Getränk (Grüntee, zuckerreduziert

Die Verbindungen **(7)** und **(8)** bzw. **(3)** und **(4)** wurden jeweils 10 % in Ethanol vorgelöst. Grüntee-Extrakt wurde in Wasser gelöst und zusammen mit Zucker, sowie dem Süßstoff Saccharin bzw. Rebaudiosid A, einer Aromazubereitung (Zitronengeschmack), sowie den ethanolischen Lösungen der Verbindungen **(7)** und **(8)** bzw. **(3)** und **(4)** (Zubereitung B) in einem Becherglas verrührt.

| Inhaltsstoff | Einsatz in Gew.-% | |
|---|---|---|
| | A | B |
| Grüntee-Extrakt | 1,4 | 1,4 |
| Wasser | 92,95 | 93,03 |
| Aromazubereitung (Typ Zitrone) | 0,65 | 0,65 |
| Zucker | 3,45 | 3,45 |
| Süßstoff Saccharin | 0,1 | --- |
| Süßstoff Rebaudiosid A | --- | 0,02 |
| Citronensäure (kristallin) | 1,2 | 1,2 |
| Ascorbinsäure | 0,2 | 0,2 |
| **(7)** und **(8)** im Verhältnis 1:1, 10%ige Lösung in Ethanol | 0,05 | --- |
| **(3)** und **(4)** im Verhältnis 1:1, 10%ige Lösung in Ethanol | --- | 0,05 |

### Anwendungsbeispiel 9: Verwendung in einem Eistee-Getränk (Schwarztee, zuckerfrei)

Die Verbindungen **(7)** und **(8)** bzw. **(3)** und **(4)** wurden jeweils in Ethanol vorgelöst. Schwarztee-Extrakt wurde in Wasser gelöst und zusammen mit dem Süßstoff Saccharin, einer Aromazubereitung (Zitronengeschmack), sowie den ethanolischen Lösungen der Verbindungen **(7)** und **(8)** (Zubereitung A), bzw. **(3)** und **(4)** (Zubereitung B) in einem Becherglas verrührt.

| Inhaltsstoff | Einsatz in Gew.-% | |
|---|---|---|
| | A | B |
| Schwarztee-Extrakt | 1,4 | 1,4 |
| Wasser | 96,465 | 96,465 |
| Saccharin | 0,035 | 0,035 |
| Aromazubereitung (Typ Zitrone) | 0,65 | 0,65 |
| Citronensäure (kristallin) | 1,2 | 1,2 |
| Ascorbinsäure | 0,2 | 0,2 |
| **(7)** und **(8)** im Verhältnis 1:1, 10%ige Lösung in Ethanol | 0,05 | --- |
| **(3)** und **(4)** im Verhältnis 1:1, 10%ige Lösung in Ethanol | --- | 0,05 |

### Anwendungsbeispiel 10: Verwendung in einem löslichen Cappuccino-Getränk

Die angegebenen Rohstoffe werden vermischt. Jeweils 12,5 g des hergestellten Instant-Cappuccino-Pulvers werden in 150 ml heißem Wasser gelöst.

| Inhaltsstoff | Einsatz in Gew.-% | | |
|---|---|---|---|
| | A | B | C |
| Kaffee-Extrakt, sprühgetrocknet | 18,0 | 18,0 | 16,0 |
| Zucker | 26,3 | 26,3 | 30,8 |
| Fettpulver | 18,2 | 18,2 | 18,2 |
| Kaffeeweißer, schäumend | 30,0 | 30,0 | 28,0 |
| Hydrokolloide/ Emulgatoren | 1,8 | 1,8 | 1,8 |
| Lactose | 4,7 | 4,7 | 4,7 |
| Mischung A aus Anwendungsbeispiel 3 | 1,0 | --- | --- |
| Mischung C aus Anwendungsbeispiel 3 | --- | 1,0 | --- |
| Halbfertigware C aus Anwendungsbeispiel 4 | --- | --- | 0,5 |

### Anwendungsbeispiel 11: Verwendung in einem Soja-Getränk

Die Verbindungen **(7)** und **(8)** bzw. **(3)** und **(4)** wurden jeweils in Ethanol vorgelöst und zu einer Sojamilch aus einem lokalen Supermarkt hinzugefügt. Die Mischung wurde zusammen mit dem Milcharoma im Becherglas verrührt.

| Inhaltsstoff | Einsatz in Gew.-% | | | |
|---|---|---|---|---|
| | A | B | C | D |
| Sojamilch (aus lokalem Supermarkt, nicht aromatisiert, ungesüßt) | 96,775 | 99,75 | 98,33 | 97,65 |
| Vanillearoma | 0,1 | 0,1 | - | 0,05 |
| Milcharoma | - | - | 0,1 | 0,05 |
| Saccharose | 3,0 | - | 1,5 | 2,0 |
| Sucralose | - | 0,025 | 0,01 | - |
| Na-Saccharin | - | - | 0,01 | - |
| Emulgum | 0,1 | 0,1 | - | 0,1 |
| **(7)** und **(8)** im Verhältnis 1:1, 10%ige Lösung in Ethanol | 0,025 | - | 0,05 | - |
| **(3)** und **(4)** im Verhältnis 1:1, 10%ige Lösung in Ethanol | - | 0,025 | - | 0,05 |
| Hesperetin, 5 %ig in Ethanol | - | - | - | 0,1 |

### Anwendungsbeispiel 12: Verwendung in einem Soja-Getränk in Kombination mit γ-Aminobuttersäure

γ-Aminobuttersäure wurde in Wasser und die Verbindungen **(7)** und **(8)** in Ethanol vorgelöst und zu einer Sojamilch aus einem lokalen Supermarkt hinzugefügt. Die resultierende Mischung wurde zusammen mit dem Milcharoma im Becherglas verrührt.

| Inhaltsstoff | Einsatz in Gew.-% |
|---|---|
| Sojamilch (aus lokalem Supermarkt) | 99,75 |
| Milcharoma | 0,1 |
| **(7)** und **(8)** im Verhältnis 1:1, 10%ige Lösung in Ethanol | 0,05 |
| γ-Aminobuttersäure, 1%ig in Wasser | 0,1 |

### Anwendungsbeispiel 13: Verwendung in einem Grapefruitsaft

Verbindungen **(7)** und **(8)** wurden in Ethanol vorgelöst und zu einem Grapefruitsaft aus einem lokalen Supermarkt hinzugefügt. Die resultierende Mischung wurde in einem Becherglas durch Rühren homogenisiert.

| Inhaltsstoff | Einsatz in Gew.-% |
|---|---|
| Grapefruitsaft (aus lokalem Supermarkt) | 99,975 |
| **(7)** und **(8)** im Verhältnis 1:1, 10%ig in Ethanol | 0,025 |

### Anwendungsbeispiel 14: Verwendung in einer bitteren Schokolade

Eine bittere Schokolade wurde aus folgenden Rohstoffen hergestellt und anschließend in rechteckigen Formen ausgegossen:

| Inhaltsstoff | Einsatz in Gew.-% | Einsatz in Gew.-% |
|---|---|---|
| Kakaomasse | Ad 100 | Ad 100 |
| Kakaobutter | 11,70 | 11,70 |
| Zucker | 29,50 | 29,50 |
| Magermilch | 3,00 | 3,00 |
| Lecithin | 0,2 | 0,2 |
| Vanillin | 0,035 | 0,035 |
| **(7)** und **(8)** im Verhältnis 1:1, 10%ig in Ethanol | --- | 0,025 |
| **(3)** und **(4)** im Verhältnis 1:1, 10%ig in Ethanol | 0,05 | --- |

**Anwendungsbeispiel 15: Verwendung in einem Kaugummi**

| Teil | Inhaltsstoff | Einsatz in Gew.-% |
|---|---|---|
| A | Kaugummibase, Company "Jagum T" | 30,00 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam | 0,10 |
| | Acesulfam^{®} K | 0,10 |
| | Emulgum^{®} (Colloides Naturels, Inc.) | 0,30 |
| C | Sorbitol, 70%ig in Wasser | 14,00 |
| | Glycerin | 1,00 |
| D | Aroma, enthaltend 1 % der Verbindungen **(7)** und **(8)** im Verhältnis 1:1, bezogen auf das Gesamtgewicht des Aromas | 1,00 |

Teile A bis D werden gemischt und intensiv geknetet. Die Rohmasse kann z.B. in Form von dünnen Streifen zu verzehrfertigen Kaugummis verarbeitet werden.

### Anwendungsbeispiel 16: Verwendung in einer Zahnpasta

| Teil | Inhaltsstoff | Einsatz in Gew.-% |
|---|---|---|
| A | demineralisiertes Wasser | 21,50 |
| | Sorbitol (70%) | 45,00 |
| | Solbrol^{®} M, Natriumsalz (Bayer AG, p-Hydroxybenzoesäurealkylester) | 0,15 |
| | Trinatriumphosphat | 0,10 |
| | Saccharin, 450 fach | 0,20 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verdickendes Siliciumdioxid) | 8,00 |
| | Natriumcarboxymethylcellulose | 0,90 |
| | Titandioxid | 0,50 |
| C | demineralisiertes Wasser | 4,53 |
| | Natriumlaurylsulfat | 1,50 |
| D | Aroma, enthaltend 1 Gew.-% der Verbindungen **(7)** und **(8)** im Verhältnis 1:1, bezogen auf das Gesamtgewicht des Aromas | 1,50 |

Die Inhaltsstoffe der Teile A und B werden jeweils für sich vorgemischt und zusammen unter Vakuum bei 25 - 30°C 30 min gut verrührt. Teil C wird vorgemischt und zu A und B gegeben; D wird hinzugefügt und die Mischung unter Vakuum bei 25 - 30°C für 30 min gut verrührt. Nach Entspannung ist die Zahnpasta fertig und kann abgefüllt werden.

## Patentansprüche

1. Verwendung eines, zweier oder mehrerer verschiedener Verbindungen der Formel **(I)** 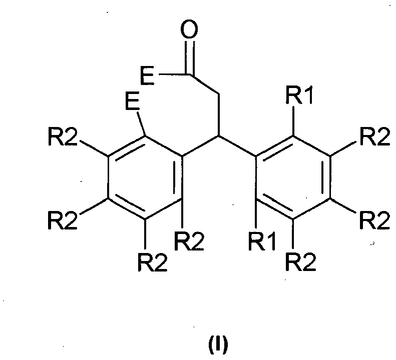 oder
eines, zweier oder mehrerer verschiedener physiologisch akzeptabler Salze eines, zweier oder mehrerer verschiedener Verbindungen der Formel **(I),**
oder
eines Gemisches eines, zweier oder mehrerer verschiedener Verbindungen der Formel **(I)** mit einem, zwei oder mehreren verschiedenen physiologisch akzeptablen Salzen eines, zweier oder mehrerer verschiedener Verbindungen der Formel **(I),**
wobei
E jeweils OH oder beide E zusammen Sauerstoff bedeuten,
R1, unabhängig von dem jeweils anderen Rest R1, Wasserstoff oder OR^{a} bedeutet, wobei R^{a} Wasserstoff, C1-C5-Alkyl oder C2-C5-Alkenyl ist,
R2, unabhängig von den anderen Resten R2, Wasserstoff oder OR^{b} bedeutet, wobei R^{b} Wasserstoff, C1-C5-Alkyl oder C2-C5-Alkenyl ist,
wobei gegebenenfalls zwei unmittelbar benachbarte Reste R1 und/oder R2 zusammen eine Gruppe OCH₂O repräsentieren,
zum sensorischen Verändern oder Maskieren des unangenehmen Geschmackseindrucks, vorzugsweise des bitteren, adstringierenden und/oder metallischen Geschmackseindrucks, eines unangenehm schmeckenden Stoffes.

2. Verwendung nach Anspruch 1, wobei ein, zwei, mehrere oder sämtliche der eingesetzten Verbindungen jeweils ausgewählt sind aus der Gruppe bestehend aus den Verbindungen der Formel **(I)** und deren physiologisch akzeptablen Salzen, wobei
E jeweils OH oder beide E zusammen Sauerstoff bedeuten,
R1, unabhängig von dem jeweils anderen Rest R1, Wasserstoff oder Hydroxy bedeutet,
R2, unabhängig von den anderen Resten R2, Wasserstoff, Hydroxy, Methoxy, Ethoxy, n-Propoxy, iso-Propoxy, n-Butoxy, iso-Butoxy, tert-Butoxy oder OR^{b} bedeutet, wobei R^{b} C5-Alkenyl ist,
wobei gegebenenfalls zwei unmittelbar benachbarte Reste R2 zusammen eine Gruppe OCH₂O repräsentieren,
wobei vorzugsweise ein oder mehrere der Reste R1 oder R2 eine Hydroxygruppe bedeuten.

3. Verwendung nach Anspruch 1 oder 2, wobei ein, zwei, mehrere oder sämtliche der eingesetzten Verbindungen der Formel **(I)** jeweils ausgewählt sind aus der Gruppe bestehend der Verbindungen der Formel **(II)** 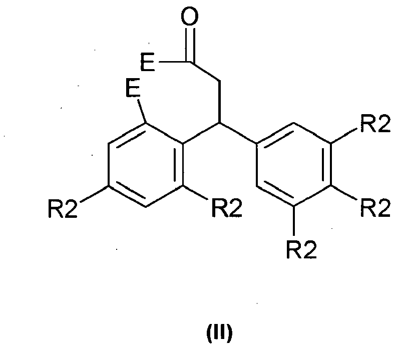 wobei
E jeweils OH oder beide E zusammen Sauerstoff bedeuten,
R2, unabhängig von den anderen Resten R2, Wasserstoff, Hydroxy, Methoxy, Ethoxy, n-Propoxy oder iso-Propoxy bedeutet, vorzugsweise H, OH, OCH₃ oder OCH₂CH₃,
wobei gegebenenfalls zwei unmittelbar benachbarte Reste R2 zusammen eine Gruppe OCH₂O repräsentieren,
wobei vorzugsweise ein oder mehrere der Reste R2 eine Hydroxygruppe bedeuten,
und deren physiologisch akzeptablen Salzen.

4. Verwendung nach einem der vorhergehenden Ansprüche, wobei ein, zwei, mehrere oder sämtliche der eingesetzten Verbindungen der Formel **(I)** jeweils ausgewählt sind aus der Gruppe bestehend aus 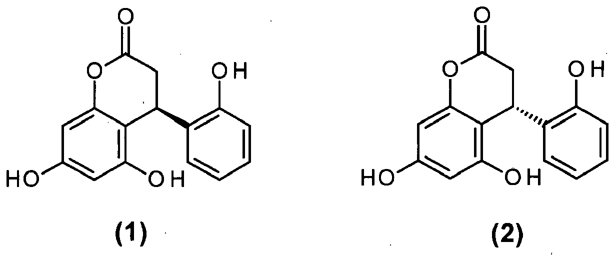 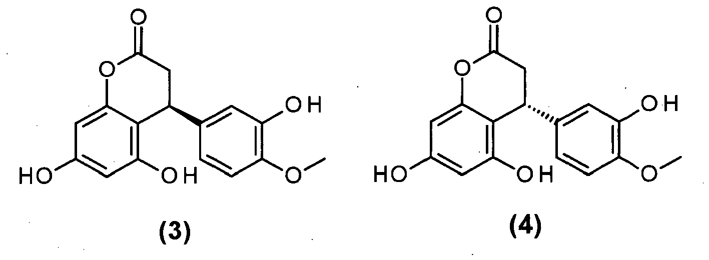 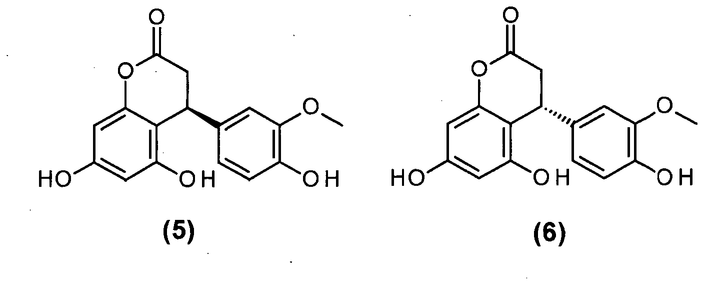 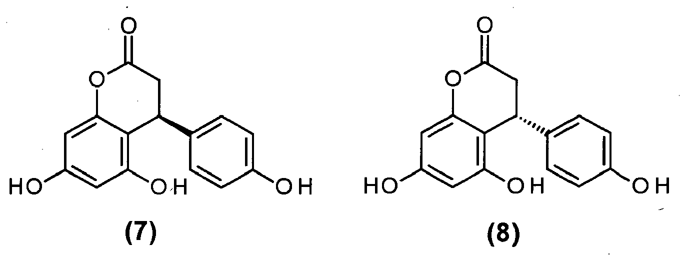 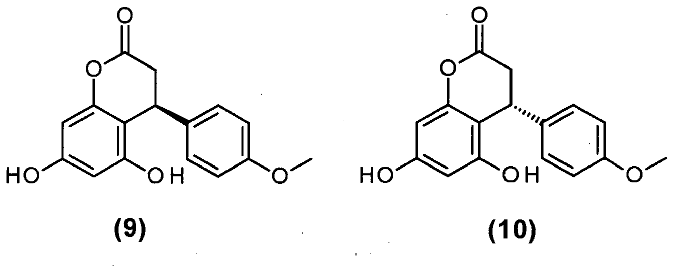 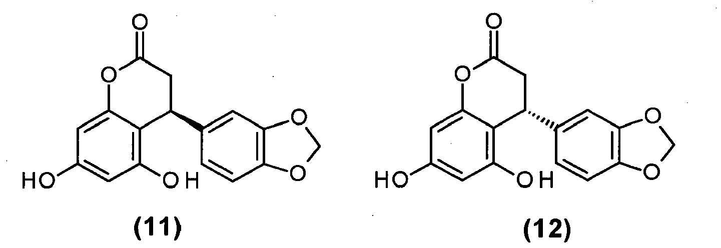 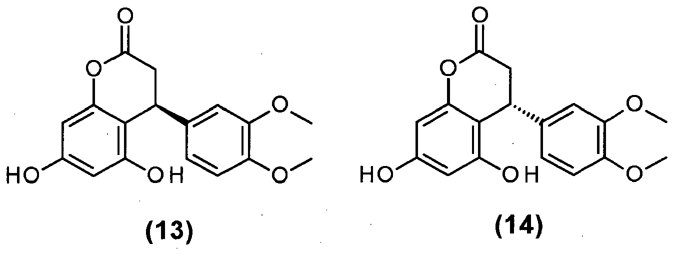 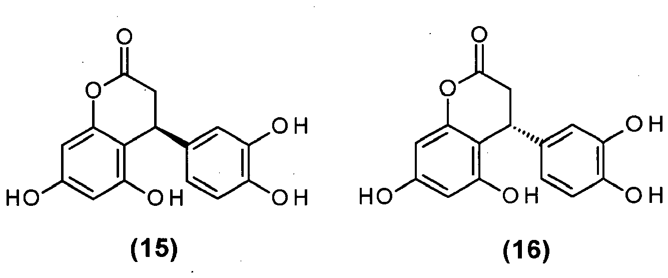 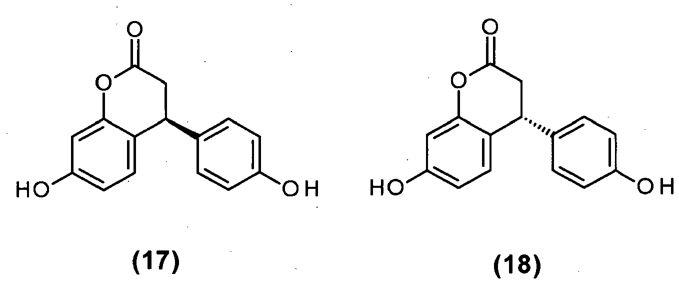 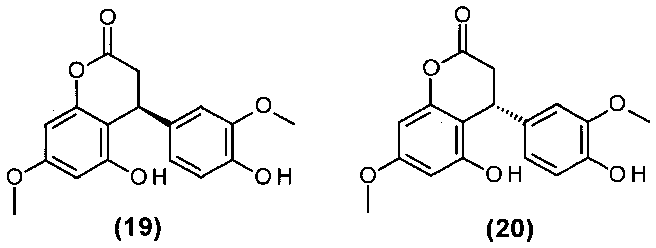 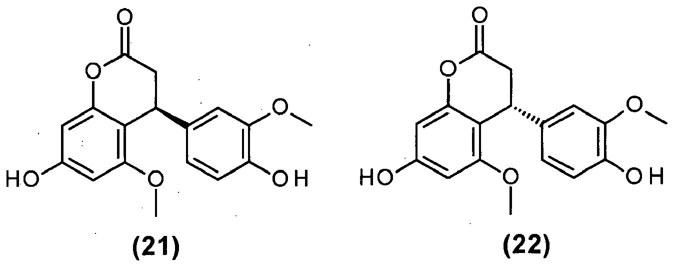 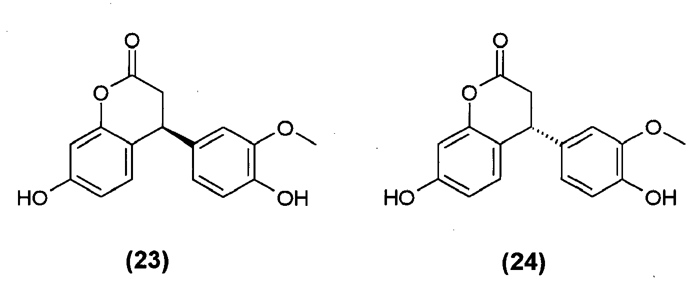 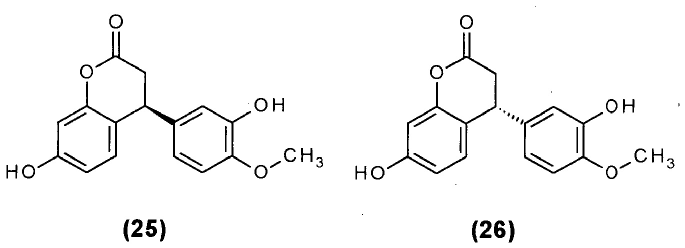 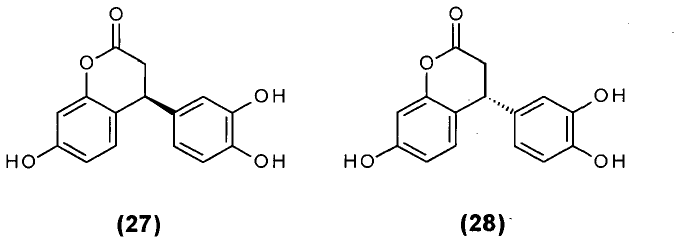 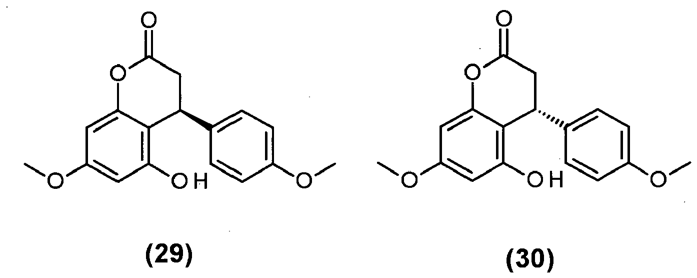 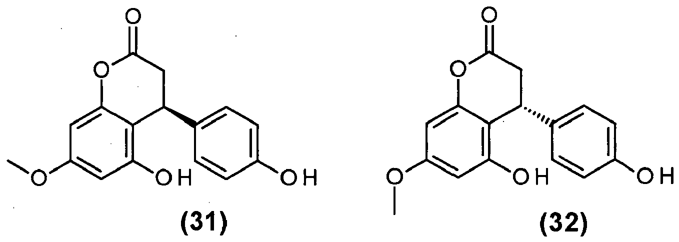 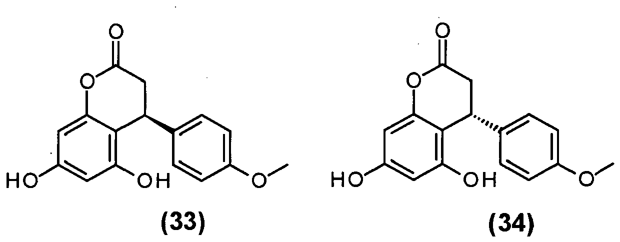 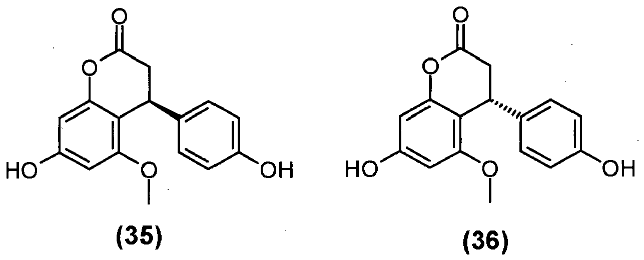 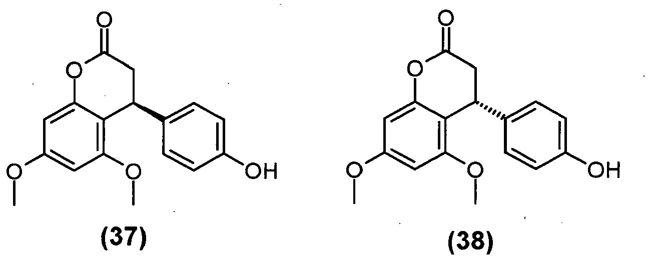 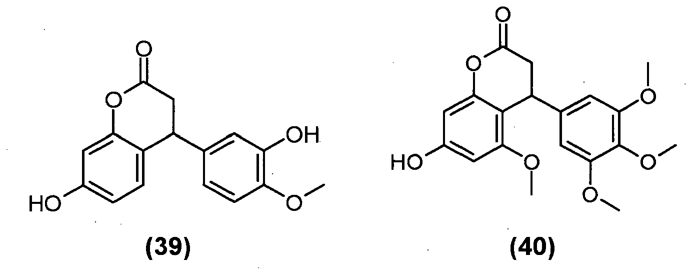 und deren physiologisch akzeptablen Salzen.

5. Verwendung eines, zweier oder mehrerer verschiedener Salze eines, zweier oder mehrerer verschiedener Verbindungen der Formel **(I)** wie in einen der vorhergehenden Ansprüche definiert
oder
eines Gemischs eines, zweier oder mehrerer verschiedener Verbindungen der Formel **(I)** wie in einen der vorhergehenden Ansprüche definiert mit einem, zwei oder mehreren verschiedenen physiologisch akzeptablen Salzen eines, zweier oder mehrerer verschiedener Verbindungen der Formel **(I)** wie in einen der vorhergehenden Ansprüche definiert,
wobei das bzw. die Gegenkationen des bzw. eines, mehrerer oder sämtlicher der physiologisch akzeptablen Salze vorzugsweise ausgewählt ist bzw. sind aus der Gruppe bestehend aus Na⁺, K⁺, NH₄⁺, Ca²⁺, Mg²⁺, Al³⁺ und Zn²⁺.

6. Zum Verzehr geeignete Zubereitung, ausgewählt aus der Gruppe bestehend aus
- der Ernährung, der Nahrungsergänzung, der Mundpflege oder dem Genuss dienenden Zubereitungen,
- kosmetischen Zubereitungen, vorzugsweise zur Applikation im Bereich des Kopfes,
- zur oralen Aufnahme bestimmten pharmazeutischen Zubereitungen, enthaltend
einen, zwei oder mehrere unangenehm, insbesondere bitter, adstringierend und/oder metallisch, schmeckende Stoffe
sowie
ein, zwei oder mehrere verschiedene Verbindungen der Formel **(I)** wie in einem der Ansprüche 1 bis 4 definiert, oder
ein, zwei oder mehrere verschiedene physiologisch akzeptable Salze eines, zweier oder mehrerer verschiedener Verbindungen der Formel **(I)** wie in einem der Ansprüche 1 bis 5 definiert, oder
ein Gemisch eines, zweier oder mehrerer verschiedener Verbindungen der Formel **(I)** wie in einem der Ansprüche 1 bis 4 definiert mit einem, zwei oder mehreren verschiedenen physiologisch akzeptablen Salzen eines, zweier oder mehrerer verschiedener Verbindungen der Formel **(I)** wie in einem der Ansprüche 1 bis 5 definiert,
wobei die Menge des bzw. der unangenehm, insbesondere bitter, adstringierend und/oder metallisch, schmeckenden Stoffe ausreicht, um in einer Vergleichszubereitung, die weder eine Verbindung der Formel **(I)** wie ein einem der Ansprüche 1 bis 4 definiert noch ein Salz einer Verbindung der Formel **(I)** wie in einem der Ansprüche 1 bis 5 definiert enthält, aber ansonsten identisch zusammengesetzt ist, als unangenehmer, insbesondere bitterer, adstringierender und/oder metallischer, Geschmack wahrgenommen zu werden,
und
die Gesamtmenge an Verbindungen der Formel **(I)** wie in einem der Ansprüche 1 bis 4 definiert, an Salzen der Verbindungen der Formel **(I)** wie in einem der Ansprüche 1 bis 5 definiert bzw. eines Gemisches wie in einem der Ansprüche 1 bis 5 definiert ausreicht, um im Vergleich mit der Vergleichszubereitung den unangenehmen, insbesondere bitteren, adstringierenden und/oder metallischen, Geschmackseindruck des bzw. der unangenehm schmeckenden Stoffe sensorisch zu verändern oder zu maskieren.

7. Zubereitung nach Anspruch 6, **dadurch gekennzeichnet, dass** die Zubereitung kein Extrakt ist, der mittels Extraktion der gemahlenen ganzen Pflanze *Polygonum Perfoliatum* mit einem Extraktionsmittelgemisch aus Ethanol und Wasser im Massen- oder Volumenverhältnis 90 : 10 oder 95 : 5 erhältlich ist, und bevorzugt kein Extrakt ist, der mittels Extraktion der ganzen Pflanze *Polygonum Perfoliatum* mit einem Extraktionsmittelgemisch aus Ethanol und Wasser erhältlich ist.

8. Zubereitung nach Anspruch 6 oder 7, **dadurch gekennzeichnet, dass** die Zubereitung
(i) einen, zwei, drei, vier, fünf, sechs, sieben, acht, neun, zehn oder mehr weitere Aromastoffe enthält mit einem Molgewicht von größer als 90 g/mol, vorzugsweise von größer als 100 g/mol, bevorzugt mit einem Molgewicht im Bereich von 110 g/mol bis 300 g/mol, weiter bevorzugt mit einem Molgewicht im Bereich von 120 g/mol bis 250 g/mol, besonders bevorzugt mit einem Molgewicht im Bereich von 125 g/mol bis 220 g/mol, insbesondere bevorzugt mit einem Molgewicht im Bereich von 130 g/mol bis 210 g/mol, und/oder
(ii) die Zubereitung eine, zwei, drei, vier, fünf, sechs, sieben, acht oder sämtliche der folgenden Substanzen nicht enthält:
Tocopherolchinon, 7'-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5 und Bonarosid,
und/oder
(iii) die Zubereitung keine der folgenden Substanzen enthält:
(24S)-Ethylchloesta-3β,5α,6α-triol, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Hydropiperosid,
und/oder
(iv) die Zubereitung frei von frischen oder getrockneten Pflanzenteilen, insbesondere frei von Blättern und Blatteilen, von *Persicaria perfoliata* (= *Polygonum perfoliatum)* ist.

9. Zubereitung nach Anspruch einem der Ansprüche 6 bis 8,
wobei die Gesamtmenge an Verbindungen der Formel **(I)** wie in einem der Ansprüche 1 bis 4 definiert und an physiologisch akzeptablen Salzen der Verbindungen der Formel **(I)** wie in einem der Ansprüche 1 bis 5 definiert im Bereich von 0,1 mg/kg bis 1 Gew.-% liegt, vorzugsweise im Bereich von 0,5 bis 1000 mg/kg, bevorzugt im Bereich von 1 bis 500 mg/kg, weiter bevorzugt im Bereich von 3 bis 300 mg/kg, besonders bevorzugt im Bereich von 5 bis 200 mg/kg, am meisten bevorzugt im Bereich von 10 bis 100 mg/kg, jeweils bezogen auf das Gesamtgewicht der Zubereitung.

10. Zubereitung nach einem der Ansprüche 6 bis 9, **dadurch gekennzeichnet, dass**
der oder einer der unangenehm schmeckenden Stoffe ein Bitterstoff ist, der in einer Konzentration vorliegt, die mindestens 2 relativen Bitterequivalenten entspricht,
oder
mehrere oder sämtliche der unangenehm schmeckenden Stoffe Bitterstoffe sind, wobei die Gesamtkonzentration aller Bitterstoffe mindestens 2 relativen Bitterequivalenten entspricht.

11. Zubereitung nach einem der Ansprüche 6 bis 10, **dadurch gekennzeichnet, dass**
die Gesamtmenge an Verbindungen der Formel **(I)** wie in einem der Ansprüche 1 bis 4 definiert und an physiologisch akzeptablen Salzen der Verbindungen der Formel **(I)** wie in einem der Ansprüche 1 bis 5 definiert ausreicht, um den unangenehmen, insbesondere bitteren, Geschmackseindruck des bzw. der unangenehm, insbesondere bitter, adstringierend und/oder metallisch, schmeckenden Stoffe sensorisch so zu verändern oder zu maskieren, dass er dem Geschmackseindruck einer Vergleichszubereitung entspricht, die (i) weder eine der oben definierten Verbindungen der Formel **(I)** noch ein physiologisch akzeptables Salz einer wie oben definierten Verbindung der Formel **(I)** sowie (ii) 90 Gew.-% oder weniger, vorzugsweise 80 Gew.-% oder weniger, bevorzugt 75 Gew.-% oder weniger, besonders bevorzugt 70 Gew.-% oder weniger des bzw. der unangenehm schmeckenden Stoffe enthält, aber ansonsten identisch zusammengesetzt ist,
und/oder
der oder einer der unangenehm schmeckenden Stoffe ein Bitterstoff ist, der in einer Konzentration von zumindest dem zweifachen seines Bitterschwellenwerts, vorzugsweise im Bereich des drei- bis tausendfachen seines Bitterschwellenwerts, vorliegt.

12. Zubereitung nach einem der Ansprüche 6 bis 11, **dadurch gekennzeichnet, dass** die Zubereitung einen, zwei oder mehrere der folgenden Bitterstoffe enthält:
- Catechine und Proanthocyanidine in einer Gesamtmenge von zumindest 0,01 Gew.-%, vorzugsweise von zumindest 0,05 Gew.-%, bevorzugt im Bereich von 0,075 Gew.-% bis 1 Gew.-%,
- Coffein und Theobromin in einer Gesamtmenge von zumindest 0,005 Gew.-%, vorzugsweise von zumindest 0,01 Gew.-%, bevorzugt von zumindest 0,02 Gew.-%, weiter bevorzugt im Bereich von 0,025 Gew.-% bis 1 Gew.-%,
- Naringin in einer Konzentration von zumindest 0,005 Gew.-%, vorzugsweise von zumindest 0,01 Gew.-%, bevorzugt im Bereich von 0,02 Gew.-% bis 0,5 Gew.-%,
- Süßstoffe in einer Gesamtmenge von zumindest 0,005 Gew.-%, vorzugsweise von zumindest 0,05 Gew.-%, bevorzugt im Bereich von 0,1 Gew.-% bis 2 Gew.-%,
jeweils bezogen auf das Gesamtgewicht der Zubereitung.

13. Mischung, vorzugsweise eine Halbfertigware zur Herstellung einer Zubereitung wie in einem der Ansprüche 6 bis 12 definiert, enthaltend
- ein, zwei oder mehrere verschiedene Verbindungen der Formel **(I)** wie in einem der Ansprüche 1 bis 4 definiert, oder
- ein, zwei oder mehrere verschiedene physiologisch akzeptable Salze eines, zweier oder mehrerer verschiedener Verbindungen der Formel **(I)** wie in einem der Ansprüche 1 bis 5 definiert, oder
- ein Gemisch eines, zweier oder mehrerer verschiedener Verbindungen der Formel **(I)** wie in einem der Ansprüche 1 bis 4 definiert mit einem, zwei oder mehreren verschiedenen physiologisch akzeptablen Salzen eines, zweier oder mehrerer verschiedener Verbindungen der Formel **(I)** wie in einem der Ansprüche 1 bis 5 definiert,
und
einen, zwei, drei, vier, fünf, sechs, sieben, acht, neun, zehn oder mehr weitere Aromastoffe enthält mit einem Molgewicht von größer als 90 g/mol, vorzugsweise von größer als 100 g/mol, bevorzugt mit einem Molgewicht im Bereich von 110 g/mol bis 300 g/mol, weiter bevorzugt mit einem Molgewicht im Bereich von 120 g/mol bis 250 g/mol, besonders bevorzugt mit einem Molgewicht im Bereich von 125 g/mol bis 220 g/mol, insbesondere bevorzugt mit einem Molgewicht im Bereich von 130 g/mol bis 210 g/mol,
sowie vorzugsweise einen oder mehrere zum Verzehr geeignete Trägerstoffe, vorzugsweise ausgewählt aus der Gruppe bestehend aus Ethanol, Isopropanol, Glycerin, 1,2-Propylenglycol, Diacetin, Triacetin, Maltodextrin, Gummi Arabicum, Siliciumdioxid und deren Mischungen.

14. Mischung nach Anspruch 13,
wobei die Gesamtmenge an Verbindungen der Formel **(I)** wie in einem der Ansprüche 1 bis 4 definiert und an physiologisch akzeptablen Salzen der Verbindungen der Formel **(I)** wie in einem der Ansprüche 1 bis 5 definiert im Bereich von 0,001 Gew.-% bis 95 Gew.-% liegt, bevorzugt im Bereich von 0,005 bis 80 Gew.-%, insbesondere bevorzugt im Bereich von 0,01 Gew.-% bis 50 Gew.-%, weiter bevorzugt im Bereich von 0,05 Gew.-% bis 40 Gew.-%, jeweils bezogen auf das Gesamtgewicht der Mischung.

15. Verfahren zum (a) Verändern oder Maskieren des unangenehmen, insbesondere bitteren, Geschmackseindrucks eines, zweier oder mehrerer unangenehm, insbesondere bitter, schmeckender Stoffe und/oder (b) Herstellen einer Zubereitung nach einem der Ansprüche 6 bis 12, mit folgendem Schritt:
in Kontakt bringen oder Mischen des oder der unangenehm, insbesondere bitter, schmeckenden Stoffe mit
- ein, zwei oder mehrere verschiedene Verbindungen der Formel **(I)** wie in einem der Ansprüche 1 bis 4 definiert,
oder
- ein, zwei oder mehrere verschiedene physiologisch akzeptable Salze eines, zweier oder mehrerer verschiedener Verbindungen der Formel **(I)** wie in einem der Ansprüche 1 bis 5 definiert,
oder
- ein Gemisch eines, zweier oder mehrerer verschiedener Verbindungen der Formel **(I)** wie in einem der Ansprüche 1 bis 4 definiert mit einem, zwei oder mehreren verschiedenen physiologisch akzeptablen Salzen eines, zweier oder mehrerer verschiedener Verbindungen der Formel **(I)** wie in einem der Ansprüche 1 bis 5 definiert.

## Claims

1. Use of one, two or more different compounds of formula **(I)** 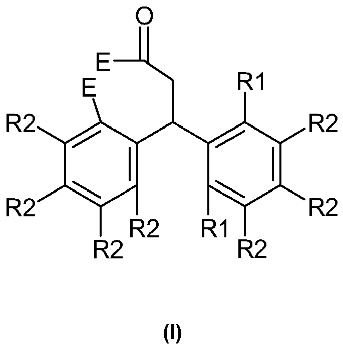 or
of one, two or more different physiologically acceptable salts of one, two or more different compounds of formula **(I),**
or
of a mixture of one, two or more different compounds of formula **(I)** with one, two or more different physiologically acceptable salts of one, two or more different compounds of formula **(I),**
wherein
E either denotes OH in each case or both E together denote an oxygen, R1, independent of the respective other residue R1 denotes hydrogen or OR^{a},
wherein R^{a} is hydrogen, C1-C5-alkyl or C2-C5-alkenyl,
R2, independent of the other residues R2 denotes hydrogen or OR^{b},
wherein R^{b} is hydrogen, C1-C5-alkyl or C2-C5-alkenyl,
wherein optionally two directly adjacent residues R1 and/or R2 together represent a group OCH₂O,
for sensorically modifying or masking the unpleasant taste impression, preferably the bitter, astringent and/or metallic taste impression, of an unpleasant tasting substance.

2. Use according to claim 1, wherein one, two, several or all of the compounds used are in each case selected from the group consisting of the compounds of formula **(I)** and the physiologically acceptable salts thereof, wherein
E in each case denotes OH or both E together denote oxygen,
R1, independent of the respective other residue R1 denotes hydrogen or hydroxy,
R2, independent of the other residues R2 denotes hydrogen, hydroxy, methoxy, ethoxy, n-propoxy, iso-propoxy, n-butoxy, iso-butoxy, tert-butoxy or OR^{b}, wherein R^{b} is C5-alkenyl,
wherein optionally two directly adjacent residues R2 together represent a group OCH₂O,
wherein preferably one or more of the residues R1 or R2 denote a hydroxy group.

3. Use according to claim 1 or 2, wherein one, two, several or all of the compounds of formula **(I)** used are in each case selected from the group consisting of the compounds of formula **(II)** 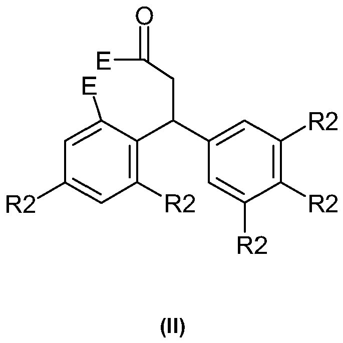 wherein
E in each case denotes OH or both E together denote oxygen,
R2, independent of the other residues R2 denotes hydrogen, hydroxy, methoxy, ethoxy, n-propoxy or iso-propoxy, preferably H, OH, OCH₃ or OCH₂CH₃,
wherein optionally two directly adjacent residues R2 together represent a group OCH₂O,
wherein preferably one or more of the residues R2 denote a hydroxy group,
and the physiologically acceptable salts thereof.

4. Use according to one of the preceding claims, wherein one, two, several or all of the compounds of formula **(I)** used are in each case selected from the group consisting of 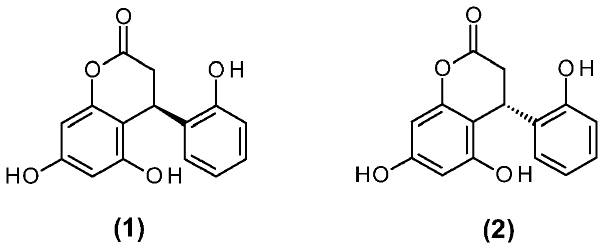 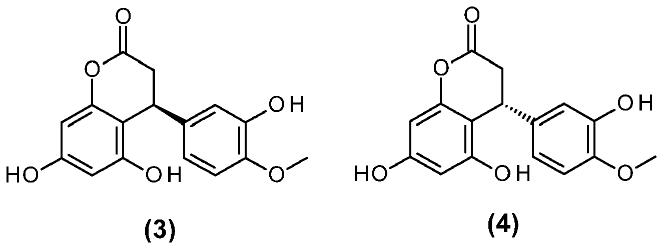 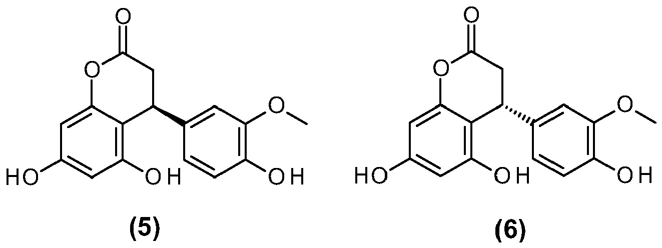 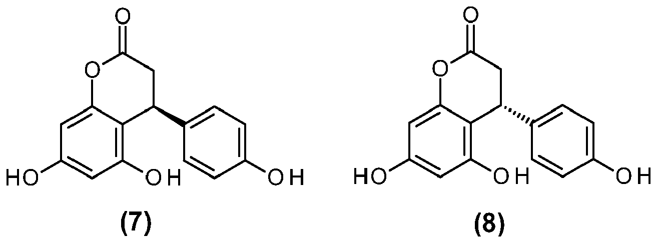 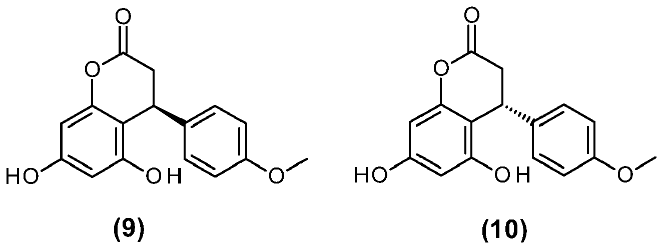 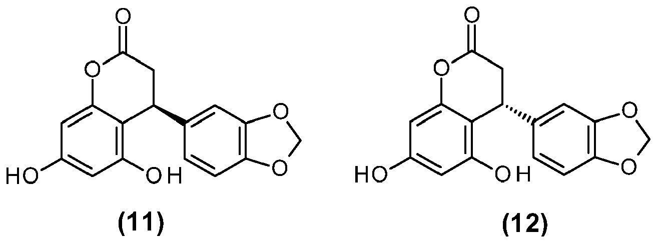 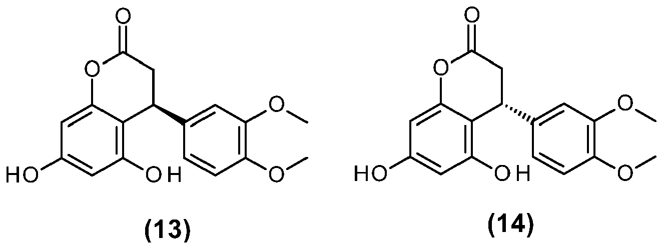 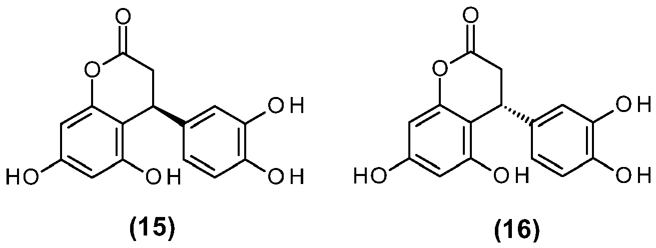 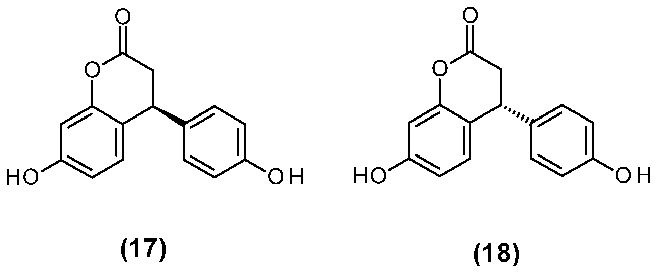 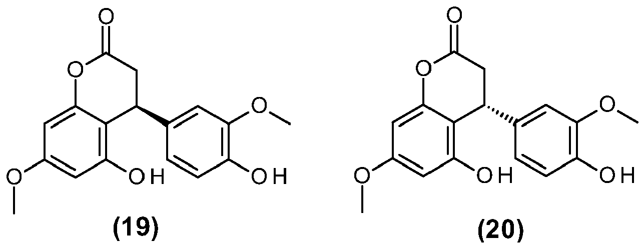 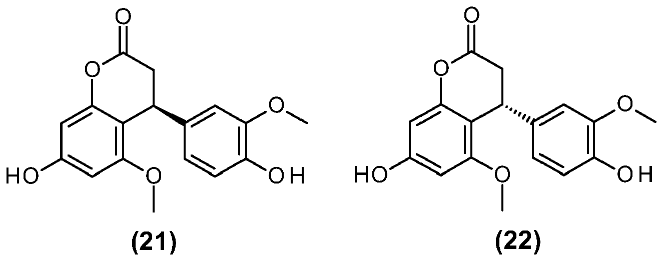 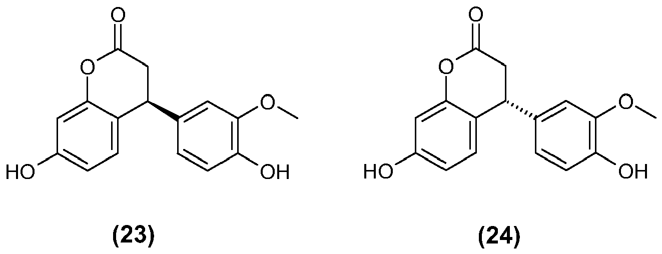 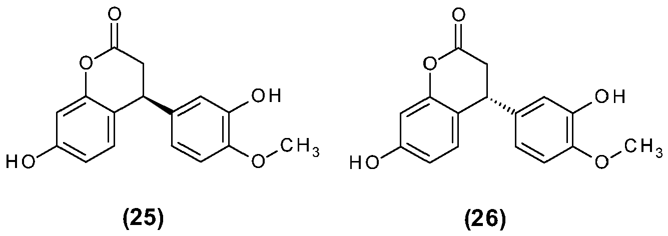 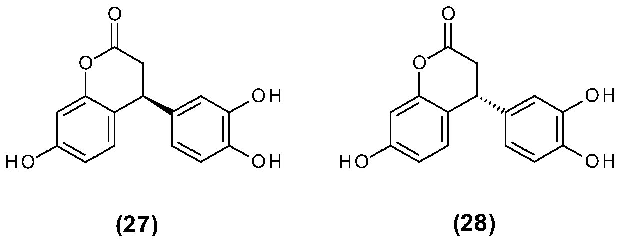 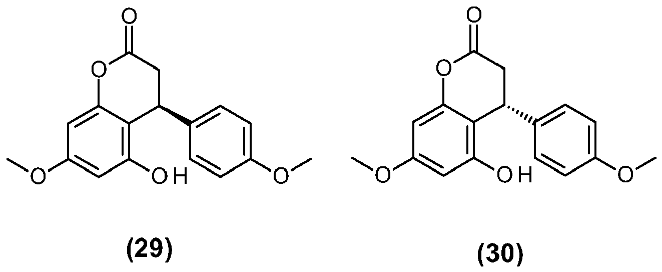 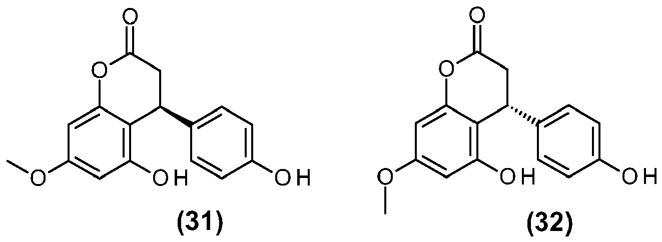 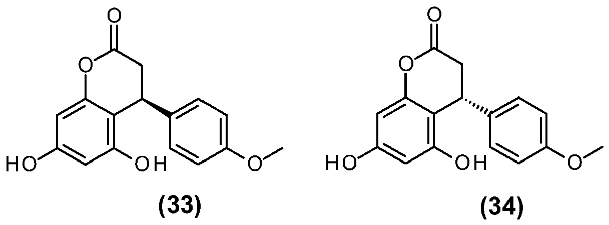 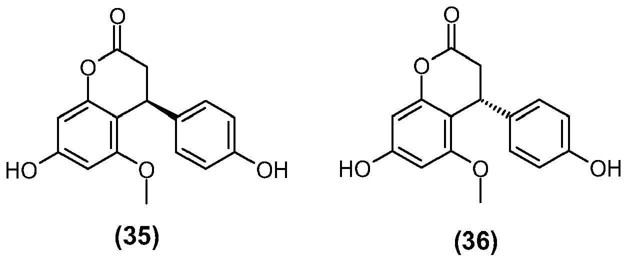 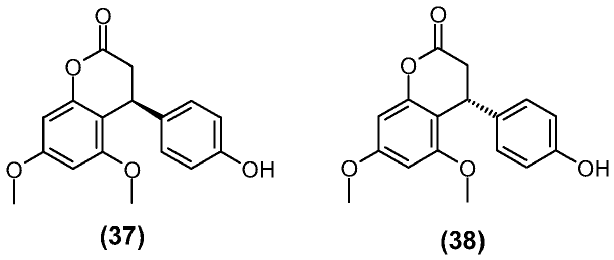 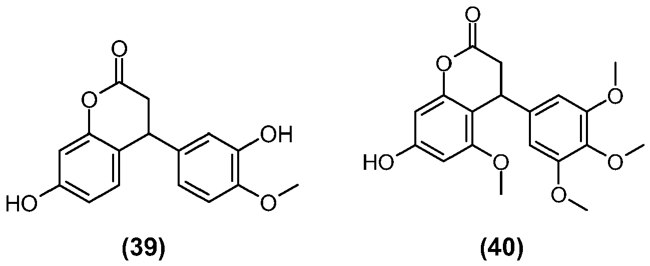 and the physiologically acceptable salts thereof.

5. Use of one, two or more different salts of one, two or more different compounds of formula **(I)** as defined in one of the preceding claims
or
of a mixture of one, two or more different compounds of formula **(I)** as defined in one of the preceding claims with one, two or more different physiologically acceptable salts of one, two or more different compounds of formula **(I)** as defined in one of the preceding claims,
wherein the counter-cation(s) of the or one, several or all of the physiologically acceptable salts is/are preferably selected from the group consisting of Na⁺, K⁺, NH₄⁺, Ca²⁺, Mg²⁺, Al³⁺ and Zn²⁺.

6. Preparation suitable for consumption, selected from the group consisting of
- food, food supplement, oral care or semi-luxury preparations,
- cosmetic preparations, in particular for application in the area of the head,
- pharmaceutical preparations for oral consumption,
comprising
one, two or more unpleasant, in particular bitter, astringent and/or metallic, tasting substances
as well as
one, two or more different compounds of formula **(I)** as defined in one of claims 1 to 4, or
one, two or more different physiologically acceptable salts of one, two or more different compounds of formula **(I)** as defined in one of claims 1 to 5, or
a mixture of one, two or more different compounds of formula **(I)** as defined in one of claims 1 to 4 with one, two or more physiologically acceptable salts of one, two or more different compounds of formula **(I)** as defined in one of claims 1 to 5,
wherein the amount of the unpleasant, in particular bitter, astringent and/or metallic, tasting substance(s) is sufficient to be perceived as an unpleasant, in particular bitter, astringent and/or metallic taste in a comparable preparation, which neither comprises a compound of formula **(I)** as defined in one of claims 1 to 4 nor a salt of a compound of formula **(I)** as defined in one of claims 1 to 5, but is otherwise identically compounded,
and
the total amount of compounds of formula **(I)** as defined in one of claims 1 to 4, of salts of compounds of formula **(I)** as defined in one of claims 1 to 5 or of a mixture as defined in one of claims 1 to 5, respectively, to sensorically modify or mask the unpleasant, in particular bitter, astringent and/or metallic taste impression of the unpleasant tasting substance(s).

7. Preparation according to claim 6, **characterized in that** the preparation is not an extract obtainable by extraction of the ground plant *Polygonum Perfoliatum* using an extractant mixture of ethanol and water with a mass or volume ratio of 90 : 10 or 95 : 5, and preferably is not an extract obtainable by extraction of the whole plant *Polygonum Perfoliatum* using an extractant mixture of ethanol and water.

8. Preparation according to claim 6 or 7, **characterized in that** the preparation
(i) comprises one, two, three, four, fife, six, seven, eight, nine, ten or more further flavor substances with a molecular weight of greater than 90 g/mol, preferably greater than 100 g/mol, preferred with a molecular weight in the range from 110 g/mol to 300 g/mol, further preferred with a molecular weight in the range from 120 g/mol to 250 g/mol, particularly preferred with a molecular weight in the range from 125 g/mol to 220 g/mol, especially preferred with a molecular weight in the range from 130 g/mol to 210 g/mol,
and/or
(ii) the preparation does not comprise one, two, three, four, fife, six, seven, eight or all of the following substances:
tocopherolchinon, 7'-dihydroxymatairesinol, (24S)-ethylchloesta-3β,5α,6α-triol, quercetin, cucurbitacin IIa, cucurbitacin U, iotroridoside A, pokeweed cerebroside 5 and bonaroside,
and/or
(iii) the preparation does not comprise any of the following substances:
(24S)-ethylchloesta-3β,5α,6α-triol, iotroridoside A, pokeweed cerebroside 5, bonaroside, helonioside A, helonioside B, lapathoside D, vanicoside B, vanicoside C, vanicoside F, asteryunnanoside, hydropiperoside,
and/or
(iv) the preparation is free of fresh or dried plant parts, in particular free of leaves and leave parts, of *Persicaria perfoliata* (= *Polygonum perfoliatum).*

9. Preparation according to one of claims 6 to 8,
wherein the total amount of compounds of formula **(I)** as defined in one of claims 1 to 4 and of physiologically acceptable salts of the compounds of formula **(I)** as defined in one of claims 1 to 5 is in the range from 0,1 mg/kg to 1 wt.%, preferably in the range from 0,5 to 1000 mg/kg, preferred in the range from 1 to 500 mg/kg, further preferred in the range from 3 to 300 mg/kg, particularly preferred in the range from 5 to 200 mg/kg, most preferred in the range from 10 to 100 mg/kg, in each case with respect to the total weight of the preparation.

10. Preparation according to one of claims 6 to 9, **characterized in that**
the or one of the unpleasant tasting substance(s) is a bitter-tasting compound, which is present in a concentration corresponding to at least 2 relative bitter equivalents,
or
several or all of the unpleasant tasting substance(s) are bitter-tasting compounds, wherein the total concentration of all bitter-tasting compounds corresponds to at least 2 relative bitter equivalents.

11. Preparation according to one of claims 6 to 10, **characterized in that**
the total amount of compounds of formula **(I)** as defined in one of claims 1 to 4 and of physiologically acceptable salts of the compounds of formula **(I)** as defined in one of claims 1 to 5 is sufficient to sensorically modify or mask the unpleasant, in particular bitter, taste impression of the unpleasant, in particular bitter, astringent and/or metallic tasting substance(s) such that it corresponds to the taste impression of a comparable preparation, which (i) neither contains one of the above defined compounds of formula **(I)** nor a physiologically acceptable salt of a compound of formula **(I)** as defined above as well as (ii) contains 90 wt.-% or less, preferably 80 wt.-% or less, preferred 75 wt.-% or less, particularly preferred 70 wt.-% or less of the unpleasant tasting substance(s) but is otherwise is compounded identically,
and/or
the or one of the unpleasant tasting substance(s) is a bitter-tasting compound, which is present in a concentration of at least twice its bitter threshold, preferably in the range of three times to thousand times its bitter threshold.

12. Preparation according to one of claims 6 to 11, **characterized in that** the preparation comprises one, two or more of the following bitter-tasting compounds:
- catechins and proanthocyanidins in a total amount of at least 0,01 wt.-%, preferably of at least 0,05 wt.-%, preferred in the range from 0,075 wt.% to 1 wt.-%,
- caffeine and theobromin in a total amount of at least 0,005 wt.%, preferably of at least 0,01 wt.-%, preferred of at least 0,02 wt.-%, further preferred in the range from 0,025 wt.-% to 1 wt.-%,
- naringin in a concentration of at least 0,005 wt.-%, preferably of at least 0,01 wt.-%, preferred in the range from 0,02 wt.-% to 0,5 wt.-%,
- sweeteners in a total amount of at least 0,005 wt.-%, preferably of at least 0,05 wt.-%, preferred in the range from 0,1 wt.-% to 2 wt.-%,
in each case with respect of the total weight of the preparation.

13. Mixture, preferably semi-finished product for the production of a preparation as defined in one of claims 6 to 12, comprising
- one, two or more different compounds of formula **(I)** as defined in one of claims 1 to 4, or
- one, two or more different physiologically acceptable salts of one, two or more different compounds of formula **(I)** as defined in one of claims 1 to 5, or
- a mixture of one, two or more different compounds of formula **(I)** as defined in one of claims 1 to 4 with one, two or more different physiologically acceptable salts of one, two or more different compounds of formula **(I)** as defined in one of claims 1 to 5,
and
comprising one, two, three, four, five, six, seven, eight, nine, ten or more further flavor substances with a molecular weight of greater than 90 g/mol, preferably greater than 100 g/mol, preferably with a molecular weight in the range from 110 g/mol to 300 g/mol, further preferred with a molecular weight in the range from 120 g/mol to 250 g/mol, particularly preferred with a molecular weight in the range from 125 g/mol to 220 g/mol, especially preferred with a molecular weight in the range from 130 g/mol to 210 g/mol,
as well as preferably one or more carrier substances suitable for consumption, preferably selected from the group consisting of ethanol, isopropanol, glycerol, 1,2-propyleneglycol, diacetin, triacetin, maltodextrin, gum arabicum, silicon dioxide and mixtures thereof.

14. Mixture according to claim 13,
wherein the total amount of compounds of formula **(I)** as defined in one of claims 1 to 4 and of physiologically acceptable salts of the compounds of formula **(I)** as defined in one of claims 1 to 5 is in the range from 0,001 wt.-% to 95 wt.-%, preferred in the range from 0,005 to 80 wt.-%, particularly preferred in the range from 0,01 wt.-% to 50 wt.-%, further preferred in the range from 0,05 wt.-% to 40 wt.-%, in each case with respect to the total weight of the preparation.

15. Method for (a) modifying or masking of the unpleasant, in particular bitter, taste impression of one, two or more unpleasant, in particular bitter, tasting substances and/or (b) producing a preparation according to one of claims 6 to 12 with the following step:
contacting or mixing the unpleasant, in particular bitter, tasting substance(s) with
- one, two or more different compounds of formula **(I)** as defined in one of claims 1 to 4,
or
- one, two or more different physiologically acceptable salts of one, two or more different compounds of formula **(I)** as defined in one of claims 1 to 5,
or
- a mixture of one, two or more different compounds of formula **(I)** as defined in one of claims 1 to 4 with one, two or more different physiologically acceptable salts of one, two or more different compounds of formula **(I)** as defined in one of claims 1 to 5.

## Revendications

1. Utilisation d'un, de deux ou de plusieurs composés différents de la formule (I) 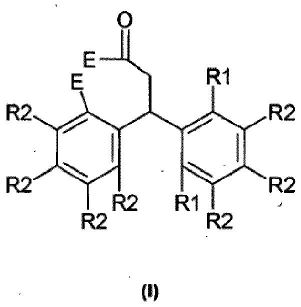 ou
d'un, de deux ou de plusieurs sels physiologiquement acceptables différents d'un, de deux ou de plusieurs composés différents de la formule (I),
ou
d'un mélange d'un, de deux ou de plusieurs composés différents de la formule (I) avec un, deux ou plusieurs sels physiologiquement acceptables différents d'un, de deux ou de plusieurs composés différents de la formule (I),
dans laquelle
soit E représente chacun OH soit les deux E représentent ensemble de l'oxygène,
R1, indépendamment de l'autre radical R1 respectivement, représente de l'hydrogène ou OR^{a}, où R^{a} est de l'hydrogène, de l'alkyle en C1-C5 ou de l'alkényle en C2-C5,
R2, indépendamment des autres radicaux R2, représente de l'hydrogène ou OR^{b}, où R^{b} est de l'hydrogène, de l'alkyle en C1-C5 ou de l'alkényle en C2-C5,
le cas échéant, deux radicaux R1 et/ou R2 immédiatement voisins représentant ensemble un groupe OCH₂O,
pour modifier de façon sensorielle ou masquer l'impression gustative désagréable, de préférence l'impression gustative amère, astringente et/ou métallique d'une substance ayant un goût désagréable.

2. Utilisation selon la revendication 1, dans laquelle un, deux, plusieurs ou l'ensemble des composés mis en oeuvre sont choisis chacun dans le groupe constitué par les composés de la formule (I) et les sels physiologiquement acceptables de ceux-ci, où
E représente chacun OH ou les deux E représentent ensemble de l'oxygène,
R1, indépendamment de l'autre radical R1 respectivement, représente de l'hydrogène ou hydroxy,
R2, indépendamment des autres radicaux R2, représente de l'hydrogène, hydroxy, méthoxy, éthoxy, n-propoxy, iso-propoxy, n-butoxy, iso-butoxy, tert-butoxy ou OR^{b}, où R^{b} est de l'alkényle en C5,
le cas échéant, deux radicaux R2 immédiatement voisins représentant ensemble un groupe OCH₂O,
de préférence, un ou plusieurs des radicaux R1 ou R2 représentant un groupe hydroxy.

3. Utilisation selon la revendication 1 ou 2, dans laquelle un, deux, plusieurs ou l'ensemble des composés de la formule (I) mis en oeuvre sont choisis chacun dans le groupe des composés de la formule (II) 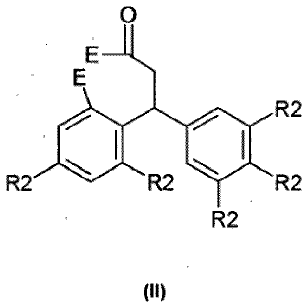 dans laquelle
E représente chacun OH ou les deux E représentent ensemble de l'oxygène,
R2, indépendamment des autres radicaux R2, représente de l'hydrogène, hydroxy, méthoxy, éthoxy, n-propoxy ou iso-propoxy, de préférence H, OH, OCH₃ ou OCH₂CH₃,
le cas échéant, deux radicaux R2 immédiatement voisins représentant ensemble un groupe OCH₂O,
de préférence un ou plusieurs des radicaux R2 représentant un groupe hydroxy,
et les sels physiologiquement acceptables de ceux-ci.

4. Utilisation selon l'une quelconque des revendications précédentes, dans laquelle un, deux, plusieurs ou l'ensemble des composés de la formule (I) mis en oeuvre sont choisis chacun dans le groupe constitué par 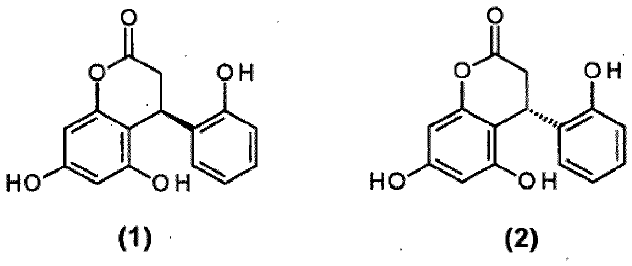 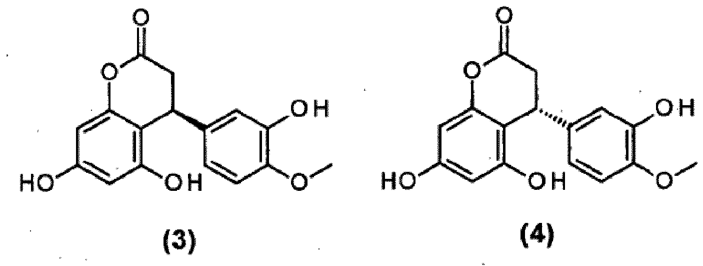 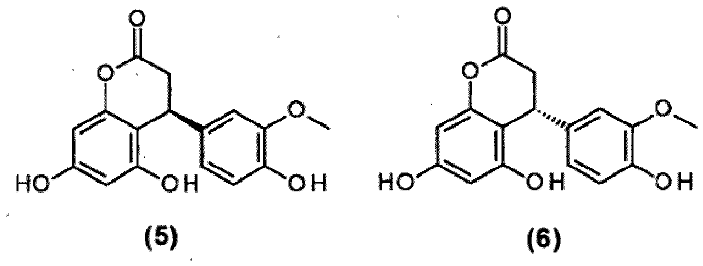 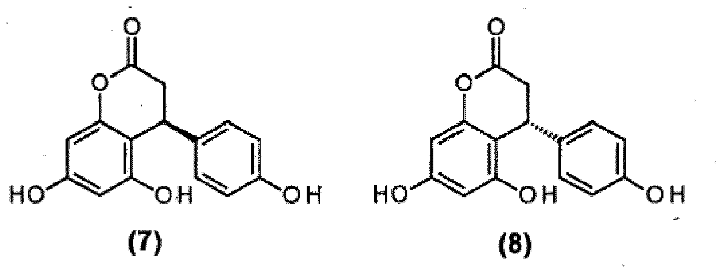 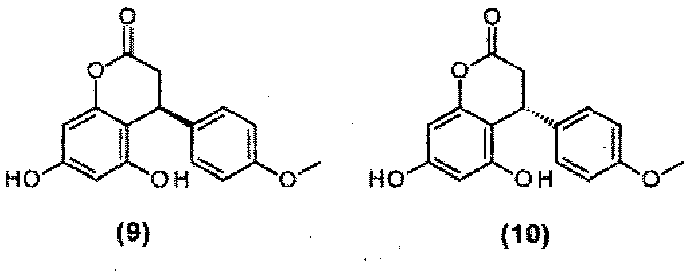 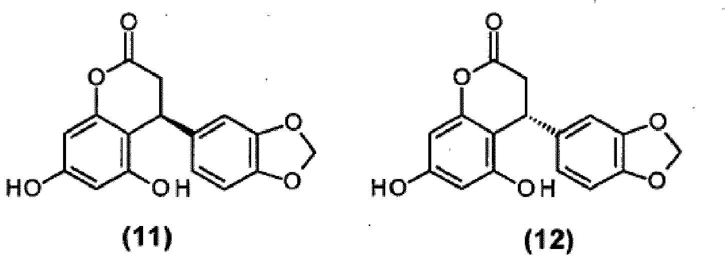 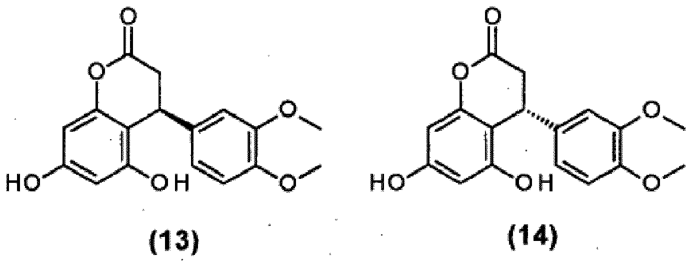 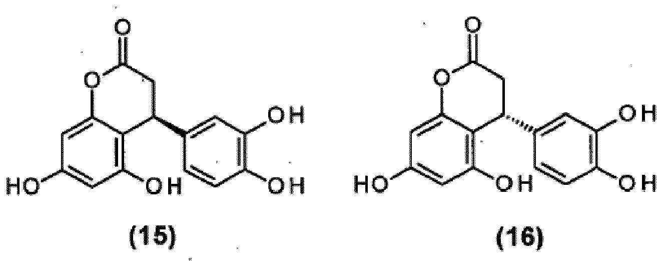 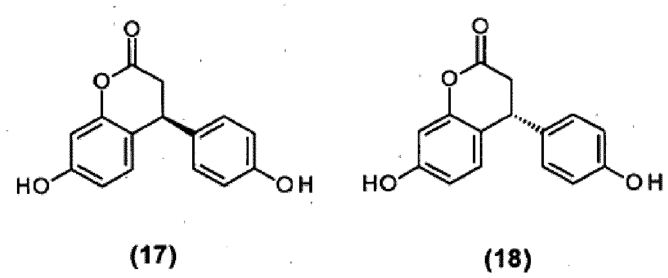 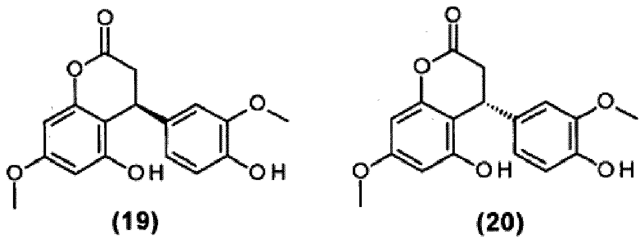 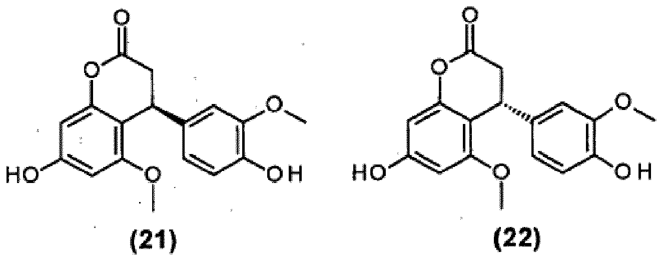 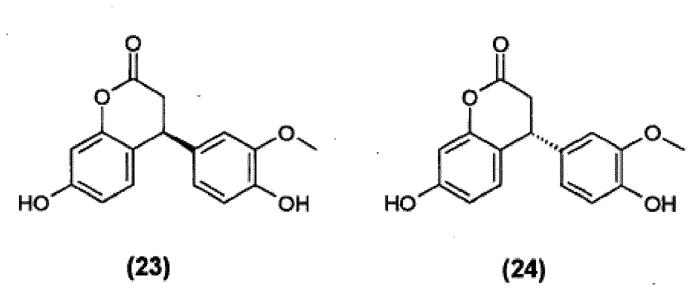 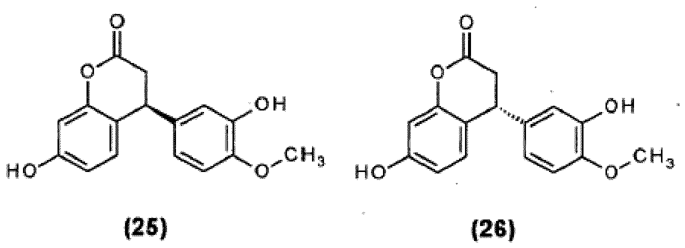 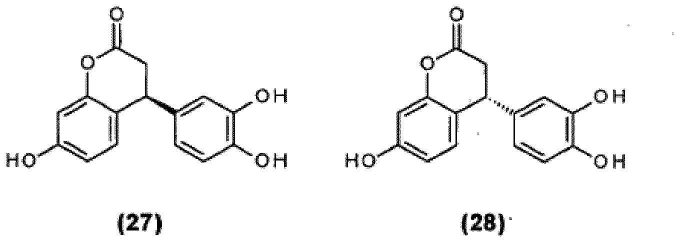 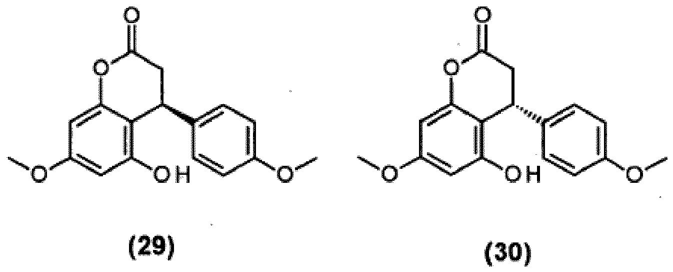 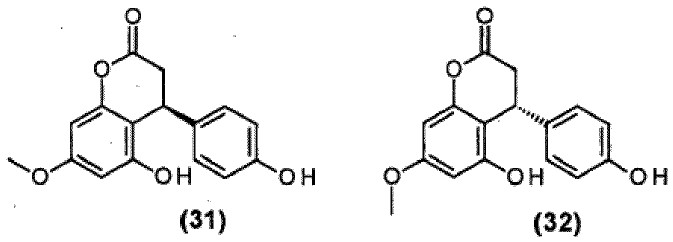 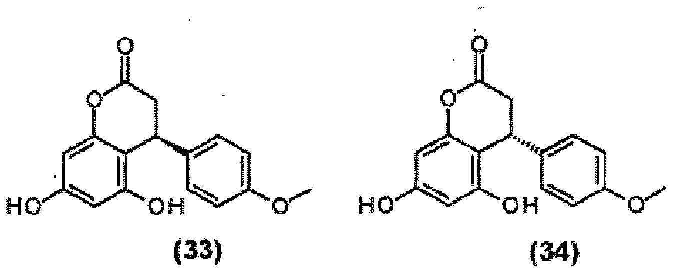 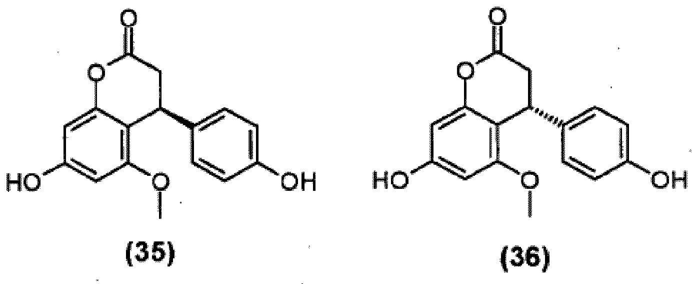 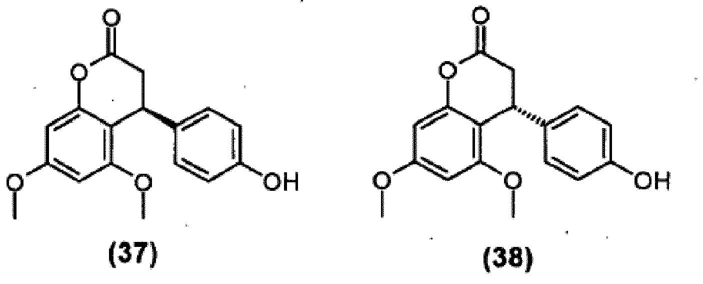 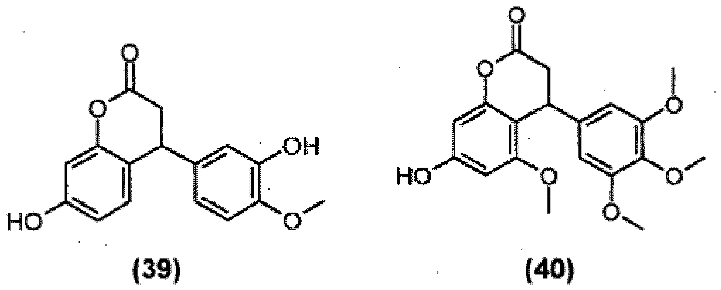 et les sels physiologiquement acceptables de ceux-ci.

5. Utilisation d'un, de deux ou de plusieurs sels différents d'un, de deux ou
de plusieurs composés différents de la formule (I) tels que définis dans l'une quelconque des revendications précédentes
ou
d'un mélange d'un, de deux ou de plusieurs composés différents de la formule (I) tels que définis dans l'une quelconque des revendications précédentes, avec un, deux ou plusieurs sels physiologiquement acceptables différents d'un, de deux ou de plusieurs composés différents de la formule (I) tels que définis dans l'une quelconque des revendications précédentes,
le ou bien les contre-cations du ou bien d'un, de plusieurs ou de l'ensemble des sels physiologiquement acceptables étant choisi(s) de préférence dans le groupe constitué par Na⁺, K⁺, NH₄⁺, Ca²⁺, Mg²⁺, Al³⁺ et Zn²⁺.

6. Préparation appropriée à la consommation, choisie dans le groupe constitué par
- les préparations servant à la nourriture, de complément alimentaire, à l'hygiène buccale ou à la consommation,
- les préparations cosmétiques, de préférence destinées à être appliquées au niveau de la tête,
- les préparations pharmaceutiques destinées à être prises oralement, contenant
une, deux ou plusieurs substances ayant un goût désagréable, en particulier amer, astringent et/ou métallique,
ainsi qu'
un, deux ou plusieurs composés différents de la formule (I) tels que définis dans l'une quelconque des revendications 1 à 4, ou
un, deux ou plusieurs sels physiologiquement acceptables différents d'un, de deux ou de plusieurs composés différents de la formule (I) tels que définis dans l'une quelconque des revendications 1 à 5, ou
un mélange d'un, de deux ou de plusieurs composés différents de la formule (I) tels que définis dans l'une quelconque des revendications 1 à 4, avec un, deux ou plusieurs sels physiologiquement acceptables différents d'un, de deux ou de plusieurs composés différents de la formule (I) tels que définis dans l'une quelconque des revendications 1 à 5,
la quantité de la ou des substances ayant un goût désagréable, en particulier amer, astringent et/ou métallique étant suffisante afin d'être perçue(s) en tant que goût désagréable, en particulier amer, astringent et/ou métallique dans une préparation de comparaison qui ne contient ni un composé de la formule (I) tel que défini dans l'une quelconque des revendications 1 à 4 ni un sel d'un composé de la formule (I) tel que défini dans l'une quelconque des revendications 1 à 5, mais qui, pour le reste, présente une composition identique,
et
la quantité totale de composés de la formule (I) tels définis dans l'une quelconque des revendications 1 à 4, de sels des composés de la formule (I) tels que définis dans l'une quelconque des revendications 1 à 5 ou bien d'un mélange tel que défini dans l'une quelconque des revendications 1 à 5, étant suffisante afin de modifier de façon sensorielle ou de masquer, en comparaison de ladite préparation de comparaison, l'impression gustative désagréable, en particulier amère, astringente et/ou métallique de la ou bien des substances ayant un goût désagréable.

7. Préparation selon la revendication 6, **caractérisée par le fait que** la préparation n'est pas un extrait qui peut être obtenu au moyen d'une extraction de l'ensemble de la plante *Polygonum Perfoliatum* broyée, avec un mélange d'extractant composé d'éthanol et d'eau, dans le rapport de masse ou de volume de 90 : 10 ou de 95 : 5, et, de préférence, n'est pas un extrait qui peut être obtenu au moyen d'une extraction de l'ensemble de la plante *Polygonum Perfoliatum,* avec un mélange d'extractant composé d'éthanol et d'eau.

8. Préparation selon la revendication 6 ou 7, **caractérisée par le fait que** la préparation
(i) contient un, deux, trois, quatre, cinq, six, sept, huit, neuf, dix autres aromatisants ou plus, avec un poids moléculaire supérieur à 90 g/mol, de préférence supérieur à 100 g/mol, de préférence avec un poids moléculaire compris entre 110 g/mol et 300 g/mol, de préférence encore avec un poids moléculaire compris entre 120 g/mol et 250 g/mol, de manière particulièrement préférée avec un poids moléculaire compris entre 125 g/mol et 220 g/mol, en particulier de préférence avec un poids moléculaire compris entre 130 g/mol et 210 g/mol,
et/ou
(ii) ladite préparation ne contient pas une, deux, trois, quatre, cinq, six, sept, huit ou l'ensemble des substances suivantes :
la tocophérolquinone, le 7'-dihydroxymatairésinol, le (24S)-éthylcholesta-3β,5α,6α-triol, la quercétine, la cucurbitacine IIa, la cucurbitacine U, l'iotroridoside A, le cérébroside de raisin d'Amérique 5 et le bonaroside,
et/ou
(iii) la préparation ne contient aucune des substances suivantes :
(24S)-éthylcholesta-3β,5α,6α-triol, l'iotroridoside A, le cérébroside de raisin d'Amérique 5, le bonaroside, l'hélonioside A, l'hélonioside B, le lapathoside D, le vanicoside B, le vanicoside C, le vanicoside F, l'asteryunnanoside, l'hydropiperoside,
et/ou
(iv) la préparation est exempte de parties fraîches ou séchées de plante, en particulier exempte de feuilles et de parties de feuille, de *Persicaria perfoliata (= Polygonum perfoliatum).*

9. Préparation selon l'une quelconque des revendications 6 à 8,
dans laquelle la quantité totale de composés de la formule (I) tels que définis dans l'une quelconque des revendications 1 à 4 et de sels physiologiquement acceptables des composés de la formule (I) tels que définis dans l'une quelconque des revendications 1 à 5 est comprise entre 0,1 mg/kg et 1 % en poids, de préférence entre 0,5 et 1000 mg/kg, de préférence entre 1 et 500 mg/kg, de préférence encore entre 3 et 300 mg/kg, de manière particulièrement préférée entre 5 et 200 mg/kg, de manière la plus préférée entre 10 et 100 mg/kg, respectivement par rapport au poids total de la préparation.

10. Préparation selon l'une quelconque des revendications 6 à 9, **caractérisée par le fait que**
la ou l'une des substances ayant un goût désagréable est une substance amère qui est présente en une concentration qui correspond à au moins 2 équivalents amers relatifs
ou
plusieurs ou l'ensemble des substances ayant un goût désagréable sont des substances amères, la concentration totale de l'ensemble des substances amères correspondant à au moins 2 équivalents amers relatifs.

11. Préparation selon l'une quelconque des revendications 6 à 10, **caractérisée par le fait que**
la quantité totale de composés de la formule (I) tels que définis dans l'une quelconque des revendications 1 à 4 et de sels physiologiquement acceptables des composés de la formule (I) tels que définis dans l'une quelconque des revendications 1 à 5 est suffisante afin de modifier de façon sensorielle ou de masquer l'impression gustative désagréable, en particulier amère, de la ou bien des substances ayant un goût désagréable, en particulier amer, astringent et/ou métallique, de telle sorte qu'elle corresponde à l'impression gustative d'une préparation de comparaison qui (i) ne contient ni l'un des composés de la formule (I) définis ci-dessus ni un sel physiologiquement acceptable d'un composé de la formule (I) tel que défini ci-dessus et qui (ii) contient 90 % en poids ou moins, de préférence 80 % en poids ou moins, de préférence 75 % en poids ou moins, de manière particulièrement préférée 70 % en poids ou moins de la ou bien des substances ayant un goût désagréable, mais qui, pour le reste, présente une composition identique,
et/ou
la ou l'une des substances ayant un goût désagréable est une substance amère qui est présente en une concentration correspondant à au moins deux fois sa valeur seuil amère, de préférence comprise entre trois et mille fois sa valeur seuil amère.

12. Préparation selon l'une quelconque des revendications 6 à 11, **caractérisée par le fait que** la préparation contient une, deux ou plusieurs des substances amères suivantes :
- les catéchines et les proanthocyanidines en une quantité totale d'au moins 0,01 % en poids, de préférence d'au moins 0,05 % en poids, de préférence comprise entre 0,075 % en poids et 1 % en poids,
- la caféine et la théobromine en une quantité totale d'au moins 0,005 % en poids, de préférence d'au moins 0,01 % en poids, de préférence d'au moins 0,02 % en poids, de préférence encore comprise entre 0,025 % en poids et 1 % en poids,
- la naringine en une concentration d'au moins 0,005 % en poids, de préférence d'au moins 0,01 % en poids, de préférence comprise entre 0,02 % en poids et 0,5 % en poids,
- les édulcorants en une quantité totale d'au moins 0,005 % en poids, de préférence d'au moins 0,05 % en poids, de préférence comprise entre 0,1 % en poids et 2 % en poids,
respectivement par rapport au poids total de la préparation.

13. Mélange, de préférence un produit semi-fini pour produire une préparation telle que définie dans l'une quelconque des revendications 6 à 12, contenant
- un, deux ou plusieurs composés différents de la formule (I) tels que définis dans l'une quelconque des revendications 1 à 4, ou
- un, deux ou plusieurs sels physiologiquement acceptables différents d'un, de deux ou de plusieurs composés différents de la formule (I) tels que définis dans l'une quelconque des revendications 1 à 5, ou
- un mélange d'un, de deux ou de plusieurs composés différents de la formule (I) tels que définis dans l'une quelconque des revendications 1 à 4, avec un, deux ou plusieurs sels physiologiquement acceptables différents d'un, de deux ou de plusieurs composés différents de la formule (I) tels que définis dans l'une quelconque des revendications 1 à 5,
et
un, deux, trois, quatre, cinq, six, sept, huit, neuf, dix autres aromatisants ou plus, avec un poids moléculaire supérieur à 90 g/mol, de préférence supérieur à 100 g/mol, de préférence avec un poids moléculaire compris entre 110 g/mol et 300 g/mol, de préférence encore avec un poids moléculaire compris entre 120 g/mol et 250 g/mol, de manière particulièrement préférée avec un poids moléculaire compris entre 125 g/mol et 220 g/mol, en particulier de préférence avec un poids moléculaire compris entre 130 g/mol et 210 g/mol
ainsi que, de préférence, un ou plusieurs excipients appropriés à la consommation, de préférence choisis dans le groupe constitué par l'éthanol, l'isopropanol, le glycérol, le 1,2-propylène glycol, la diacétine, la triacétine, la maltodextrine, la gomme arabique, le dioxyde de silicium et les mélanges de ceux-ci.

14. Mélange selon la revendication 13,
dans lequel la quantité totale de composés de la formule (I) tels que définis dans l'une quelconque des revendications 1 à 5 et de sels physiologiquement acceptables des composés de la formule (I) tels que définis dans l'une quelconque des revendications 1 à 5 se situe dans la plage allant de 0,001 % en poids à 95 % en poids, de préférence dans la page allant de 0,005 à 80 % en poids, de manière particulièrement préférée dans la page allant de 0,01 % en poids à 50 % en poids, encore de préférence dans la page allant de 0,05 % en poids à 40 % en poids, respectivement par rapport au poids total du mélange.

15. Procédé destiné à (a) modifier ou masquer l'impression gustative désagréable, en particulier amère, d'une, de deux ou de plusieurs substances ayant un goût désagréable, en particulier amer, et/ou à (b) produire une préparation selon l'une quelconque des revendications 6 à 12, comprenant l'étape suivante :
mettre en contact ou mélanger la ou les substances ayant un goût désagréable, en particulier amer, avec
- un, deux ou plusieurs composés différents de la formule (I) tels que définis dans l'une quelconque des revendications 1 à 4,
ou
- un, deux ou plusieurs sels physiologiquement acceptables différents d'un, de deux ou de plusieurs composés différents de la formule (I) tels que définis dans l'une quelconque des revendications 1 à 5,
ou
- un mélange d'un, de deux ou de plusieurs composés différents de la formule (I) tels que définis dans l'une quelconque des revendications 1 à 4, avec un, deux ou plusieurs sels physiologiquement acceptables différents d'un, de deux ou de plusieurs composés différents de la formule (I) tels que définis dans l'une quelconque des revendications 1 à 5.
